(19) Europäisches Patentamt
European Patent Office
Office européen des brevets

(11) **EP 1 704 507 B1**

(12) **EUROPEAN PATENT SPECIFICATION**

(45) Date of publication and mention of the grant of the patent:
**16.09.2009 Bulletin 2009/38**

(21) Application number: **05706844.7**

(22) Date of filing: **07.01.2005**

(51) Int Cl.:
***G06F 19/00*** *(2006.01)*

(86) International application number:
**PCT/EP2005/000090**

(87) International publication number:
**WO 2005/069187 (28.07.2005 Gazette 2005/30)**

(54) **METHODS AND SYSTEM FOR THE IDENTIFICATION AND CHARCTERIZATION OF PEPTIDES AND THEIR FUNCTIONAL RELATIONSHIPS BY USE OF MEASURES OF CORRELATION**

METHODEN UND SYSTEM ZUR IDENTIFIKATION UND CHARAKTERISIERUNG VON PEPTIDEN UND IHRER FUNKTIONELLEN BEZIEHUNG MITTELS BERECHNUNG VON KORRELATION

MÉTHODE ET SYSTÈME POUR L'IDENTIFICATION ET CARACTÈRISATION DE PEPTIDES ET LEUR RÉLATION FONCTIONELLE PAR LA MESURE DE CORRÉLATION

(84) Designated Contracting States:
**AT BE BG CH CY CZ DE DK EE ES FI FR GB GR HU IE IS IT LI LT LU MC NL PL PT RO SE SI SK TR**

(30) Priority: **07.01.2004 EP 04000170**

(43) Date of publication of application:
**27.09.2006 Bulletin 2006/39**

(73) Proprietor: **Digilab, Inc.**
**Canton, MA 02021 (US)**

(72) Inventors:
- **ZUCHT, Hans-Dieter**
  **30539 Hannover (DE)**
- **LAMERZ, Jens**
  **30169 Hannover (DE)**
- **KHAMENIA, Valery**
  **30655 Hannover (DE)**

(74) Representative: **Kröncke, Rolf**
**Gramm, Lins & Partner GbR**
**Freundallee 13a**
**30173 Hannover (DE)**

(56) References cited:
**WO-A-20/04008371** **DE-A- 10 038 694**

- **VAIDYANATHAN SEETHARAMAN ET AL: "Flow-injection electrospray ionization mass spectrometry of crude cell extracts for high-throughput bacterial identification." JOURNAL OF THE AMERICAN SOCIETY FOR MASS SPECTROMETRY. UNITED STATES FEB 2002, vol. 13, no. 2, February 2002 (2002-02), pages 118-128, XP002273858 ISSN: 1044-0305**
- **PEVZNER PAVEL A ET AL: "Efficiency of database search for identification of mutated and modified proteins via mass spectrometry" GENOME RESEARCH, vol. 11, no. 2, February 2001 (2001-02), pages 290-299, XP002273859 ISSN: 1088-9051**
- **WILKES JON G ET AL: "Defining and using microbial spectral databases." JOURNAL OF THE AMERICAN SOCIETY FOR MASS SPECTROMETRY. UNITED STATES JUL 2002, vol. 13, no. 7, July 2002 (2002-07), pages 875-887, XP002273860 ISSN: 1044-0305**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 704 507 B1

## Description

### Technical Field of the Invention

The invention relates to the field of high-throughput analysis of samples with peptide content and especially computer implemented methods and a system implementing these methods for identifying and characterizing peptides and their functional relationships by use of measures of correlation.

### Background of the Invention

The success of the Human Genome Project in mapping the human genetic code offers astonishing potential for medical research. A prerequisite for using this information, however, is the identification of gene products, especially proteins and peptides. Peptides are the family of molecules formed from the linking, in a defined order, of various amino acids. The link between one amino acid residue and the next is an amide bond, and is sometimes referred to as a peptide bond. Peptides occur in nature and are responsible for a variety of functions, many of which are not understood. They differ from proteins, which are also long chains of amino acids, by virtue of their size.

Parallel to the world-wide efforts in Genomics, a variety of discovery technologies have been developed for analyzing samples with peptide content. Just as Genomics focuses on decoding the human genome, these technologies strive for an comprehensive analysis of the myriad of biologically relevant proteins and peptides with a molecular mass between about 0.5 and 20 kDa, among which insulin is a prominent example.

Profiling of peptides and proteins of human body fluids and tissues by mass spectrometry reveals a large number of peptide signals. Such high-throughput analytical processes demand highly sophisticated bioinformatic approaches to understand and analyze biological and pharmaceutical coherences in huge sets of data.

Conventional computer implemented methods for assisting the mass spectrometric identification of peptides and small proteins interpret the spectra and generate proposals for the identity of the candidate peptide signal by determining the differences of masses of the fragments in one spectra and assigning these differences to missing amino acids. A string of missing amino acids is then composed to a proposed amino acid sequence that is thereafter queried in a huge database containing tens of thousands of the known protein sequences, such as the Swiss-Prot database. However, if the analyzed peptide or protein is not abundant and/or in a complex mixture, such an approach turns out to be not very effective and, thus, time consuming concentration or fractionation steps of the sample have to be performed.

More sophisticated approaches take the knowledge of a known sequence in a spectrum into consideration. Here, proteolytic digests of the known sequence are proposed "in silico", and a hypothetic resulting spectrum is then correlated with the actually measured one. However, these approaches are successful only if the sources of the spectra contain only a few different analytes, as their fragment signals alter the calculations and lower the correlation coefficients of the hypothesized calculated spectra with the actually measured one. If many possible protein precursors exist for a given peptide, then creating such a hypothetic spectrum for each unknown peptide and each possible precursor, the correlation process of hypothetic and measured spectra often turn out to be quite laborious and at times even unsuccessful.

Eng et al (Journ. Am. Soc. Mass Spectrom. 5, 976-989, 1994) for instance describe a statistical scorer for tandem mass spectrometry, that relies on cross-correlating experimental spectra with predicted spectra of peptides from a database (Havilio et al, Anal. Chem. 75[3], 435-444, 2003). No additional information (e.g. enzyme specificity used to create the peptides) about the peptide except the mass of the peptide is used. In a first step the tandem mass spectrometry data is reduced, whereby all but the most abundant signals are removed. In a second step protein sequences are queried from a database for combinations of amino acids that match the mass of the peptide, wherein the search algorithm only considers mass changes typical for a post-translational modification at every occurrence of the modification site. In a third step, the preliminary matches are scored by summing the number of fragmented ions that match the ions observed in the spectrum. Immonium ions are considered if the sequence contains the amino acids Tyrosine, Tryptophan, Methionine or Phenylalanine. This and the sum of fragments are being taken into account in the scoring function. Finally, a spectrum is reconstructed from the putative amino acid sequences and the highest scoring predictions are assessed by a cross-correlational analysis. The cross-correlation function measures the coherence of the reconstructed and the measured spectrum signals by, in effect, translating one signal across the other. Well-known applications such as SEQUEST and Sonar make use of this approach. However, a disadvantage of this approach is that the peak intensity strongly depends on the ion type, the ion mass and other experimental parameters and that many factors are not fully understood yet that contribute to peptide fragmentation.

Perkins et al (Electrophoresis 20[18], 3551-3567, 1999) describe a statistical scorer that evaluates the probability of finding a collection of detected fragments in a protein database (Havilio et al, Anal. Chem. 75[3], 435-444, 2003). Applications such as Mascot, MOWSE, Protocall are based on this approach. However, a disadvantage of this approach is that the signal intensities of the measured spectra are not being considered for the data analysis.

Weinberger et al (United States Patent Application 2002/0182649) describe essentially two approaches. In the first approach a protein candidate is identified by providing the mass spectrum to a protein database mining tool which identifies at least one protein candidate for the test protein in the database based on a closeness-of-fit measure between the mass spectrum and the theoretically calculated mass spectra of proteins in the database. In the second approach, the protein candidate is directly sequenced using mass spectrometry. In this method the unknown peptide is directly fragmented during mass spectrometry and the masses of the generated fragments are determined by mass spectrometry and are used to calculate the sequence of the unknown peptide.

The approaches according to Eng et al and Weinberger et al have in common that a closeness-of-fit analysis or a cross-correlation is performed over all signals of two spectra, i.e. the measured spectrum and the predicted spectrum. A fundamental disadvantage of these methods is that they rely on predicted mass spectra.

Thus, all of the above approaches have their disadvantages in that at times they turn out to be not very effective, quite laborious, time consuming and often unsuccessful.

DE 100 38 694-A and Wilkes, J. et al. ("Defining and using microbial spectral databases."JOURNAL OF THE AMERICAN SOCIETY FOR MASS SPECTROMETRY, vol. 13, July 2002, pages 875-887), both, disclose a method for detection/identification of microorganisms via MALDI-TOF-MS and pyrolysis MS, respectively. A similarity analysis of a sample spectrum with the reference spectra in a database comprising synthetic reference spectra is performed.

Vaidyanthan, S. et al. ("Flow-injection electrospray ionization mass spectrometry of crude cell extracts for high-throughput bacterial identification." JOURNAL OF THE AMERICAN SOCIETY FOR MASS SPECTROMETRY. UNITED STATES FEB 2002, vol. 13, no. 2, February 2002 (2002-02), pages 118-128) discloses a method for detection/identification of microorganisms via MALDI-TOF-MS and FI-ESI-MS, respectively. Each sample is a crude cell extract comprising proteins, phospholipids and glycolipids. The different samples are clustered by a principal component analysis such that similarity of the samples can be identified.

Pevzner, P et al. ("Efficiency of database search for identification of mutated and modified proteins via mass spectrometry" GENOME RESEARCH, vol. 11, no. 2, February 2001 (2001-02), pages 290-299) discloses a database search tool, in which the correlation between an experimental spectrum and a tehoretical spectrum of one single peptide is calculated.

WO 2004/008371 A describes a peptide identification method by performing tandem mass spectrometry on a single sample and reducing the resulting spectrum to a peak list. Therefater the peak list is interpreted and structured and the bets peptide match is determined by matching to a strucutred reperesentation of a database.

There is thus a need for methods for analyzing samples with peptide content and a system implementing these methods overcoming or at least mitigating the disadvantages associated with the prior art.

Summary of the Invention

The following methods according to the present invention are based upon the concept of Correlation Associated Networks and peptide topologies as will be apparent from the detailed description in the sections further below.

According to the present invention a method based on CANs is provided for providing a representative, non-redundant overview of the peptide content of a sample type by analyzing a plurality of samples using its peptide topology, wherein the method comprises the steps of providing a respective mass spectrum for each sample of said plurality of samples, wherein signal intensity peaks correspond to potential peptides, computing the measures of correlation between the signal intensities of said potential peptides, grouping potential peptides together, which exhibit a degree of correlation among each other above a certain threshold, thereby providing a plurality of correlation associated networks of potential peptides, and assigning one representative potential peptide out of each correlation associated network as a representative peptide to said correlation associated network of said sample type.

Preferred embodiments of the present invention are disclosed in the dependent claims.

Brief Description of the Drawings

FIGURE 1 shows schematically the hardware components and software modules according to the present invention, their interfaces as well as the flow of information between the hardware components and the software modules.

FIGURE 2 shows an averaged Peptide Mass Fingerprint of cerebrospinal fluid (CSF) samples from several patients. Each of the 96 chromatographic fractions of each sample is analyzed by MALDI-ToF-mass spectrometry and all 96 mass spectra generated from one sample are visualized as a "2-D gel-like picture", wherein the x- and y-axis correspond to the mass-to-charge ratio (m/z) and the chromatographic fraction (F), respectively. The bars represent peptide peaks, wherein the color intensity represents the mass spectrometric signal intensity. Some identified peptides including amino acid numbers are identified in this map.

FIGURE 3 shows a diagram exemplifying the correlational behavior of functionally related peptides. Four traces of spectra from four different samples are focused on the signals of a human osteopontin peptide being comprised of the amino acids 249-314 of human osteopontin (m/z = 7653.6 Da) and its phosphorylated derivatives, carrying one (m/z = 7733.5 Da), two (m/z = 7813.5 Da), three (m/z = 7893.4 Da) or more phosporylated residues. The conserved concentration ratios of the peptides between samples lead to high degrees of correlation of the signal intensities of the respective peptide pairs.

FIGURE 4 shows a schematic example of a correlation associated network (CAN) according to the present invention. Any CAN starts from a hub peptide and any member of a 1$^{st}$ order neighborhood of such a hub peptide of 1$^{st}$ order can be a hub peptide for the next order neighborhood and so forth.

FIGURE 5 shows a flow chart schematizing the procedural steps of an application of the CAN Module according to the present invention.

FIGURE 6 shows a graphical representation of an exemplary peptide topology of a sample, wherein peptides are represented by bullets and their mutual relations by lines connecting these bullets. Such a peptide network can be projected onto a peptide map like Figure 2 for a more intuitive analysis of the results.

FIGURE 7 shows a flow chart schematizing the procedural steps of an interaction of the Sequence Network Module with the CAN Module according to the present invention.

FIGURE 8a shows a flow chart schematizing the process of checking whether a predicted sequence matches the experimental properties of an unknown peptide.

FIGURE 8b shows a flow chart exemplifying the generation of sequence predictions, which are checked according to Figure 8a.

FIGURE 8c shows a flow chart schematizing the query of all unknown peptides P2 which are related to a known peptide P1. Sequence predictions are generated for any unknown peptide P2 according to Figure 8b.

FIGURE 8d shows a flow chart exemplifying the iteration of the process as demonstrated in Figure 8c for any peptide P1 with a known sequence.

FIGURE 9 shows a table of the monoisotopic and the average mass changes of a peptide upon the respective modification.

FIGURE 10 shows a table with exemplified motifs of chemical and enzymatic reactions, their respective mechanism/ enzyme and the resulting average mass difference of the modified peptide.

FIGURE 11 shows a table listing the most common amino acids, their three- and one letter codes as well as their monoisotopic and average mass in their dehydrated form.

FIGURE 12 shows a table listing the common amino-terminal and carboxy-terminal groups of peptides, as well as the chemical composition, their respective monoisotopic and average mass.

FIGURE 13 shows a table that refers to the fraction shifts of peptides that are caused by addition of the respective amino acid to the peptide sequence under the described experimental settings with cerebrospinal fluid as sample source.

FIGURE 14a shows a table with amino acids and their empirically derived occurrence before the N-terminal cleavage position (start position) of a peptide in a precursor sequence, the respective overall occurrence of the given amino acid in all determined sequences and the ratio thereof.

FIGURE 14b shows a table with amino acids and their empirically derived occurrence after the N-terminal cleavage position (start position) of a peptide in a precursor sequence, the respective overall occurrence of the given amino acid in all determined sequences and the ratio thereof.

FIGURE 14c shows a table with amino acids and their empirically derived occurrence before the C-terminal cleavage position (end position) of a peptide in a precursor sequence, the respective overall occurrence of the given amino acid in all determined sequences and the ratio thereof.

FIGURE 14d shows a table with amino acids and their empirically derived occurrence after the C-terminal cleavage position (end position) of a peptide in a precursor sequence, the respective overall occurrence of the given amino acid in all determined sequences and the ratio thereof.

FIGURE 15 shows a flow chart schematizing the procedural steps of an interaction of the Differential Network Module with the CAN Module according to the present invention.

FIGURE 16 shows a flow chart schematizing the procedural steps of an interaction of the Marker Panel Network Module with the CAN Module according to the present invention.

FIGURE 17 shows a flow chart schematizing the procedural steps of an interaction of the Surrogate Network Module with the CAN Module according to the present invention.

FIGURES 18a and 18b show a table of the signal intensity values of the peptides with coordinates Fraction 54; m/z 2743.0, Fraction 54; m/z 1371.5, Fraction 56; m/z 2927.2 and Fraction 20; m/z 1114.3 taken from 74 samples. Furthermore, the number of related peptides k with a Spearman's Rank Order Correlation Coefficient threshold of $|r| \geq 0.8$ is shown.

FIGURE 19 shows a table with the measures of correlation of the signal intensities of the peptide with coordinates Fraction 54; m/z 2743.0 with some exemplary peptides using different measures of correlation.

FIGURE 20 shows a histogram of Spearman's Rank Order correlation coefficient probabilities. The value of a correlation coefficient of a peptide-to-peptide relation (x-axis) is plotted versus the probability for that peptide-pair to achieve that value (y-axis). Peptide-to-peptide pairs with low absolute correlation coefficients are most likely not related. This is expressed by the maximum at zero of peptide-to-peptide relations from random data (P(r) Simulation). True positive relations are very likely to be found at higher absolute correlation coefficients. Therefore the plot of correlation coefficients of peptide-to-peptide relations from measured data (P(r) Measurement) deviates from P(r) Simulation, because correlation coefficients of functionally related peptides are most likely higher than those obtained from random data. Such a plot should be generated when a threshold for a given CAN has to be chosen in order to exclude as much false positive peptide-to-peptide relations while including as many true ones as possible.

FIGURE 21 shows a table of identified peptides related to Chromogranin A 97-131, the Spearman's Rank Order Correlation coefficient value of the said peptide with the related peptide, their relative monoisotopic mass and their amino acid sequence.

FIGURE 22 shows a graph exemplifying the usability of a Differential Network of the peptides SG | 88-132 and Chromogranin A 97-131. In hypothetic patients before prostatectomy (black triangles) a correlation between these peptides is present (r = 0.97), and a signal intensity ratio of about 10/1 is conserved. In hypothetic samples after prostatectomy (white squares) this ratio is not present and the Secretogranin I/Chromogranin A relation is "broken".

FIGURES 23a and 23b show a table of the signal intensity values of the peptides with coordinates Fraction 54; m/z 1371.5, Fraction 56; m/z 2927.2 and Fraction 20; m/z 1114.3 of 74 samples after removal of the variance of the signal intensity of the peptide with coordinates Fraction 54; m/z 2743.0. Furthermore, the number of related peptides k with a Spearman's Rank Order Correlation Coefficient threshold of $|r| \geq 0.8$ after the removal of said variance is shown.

FIGURE 24a shows a graph plotting the signal intensity of the peptide in fraction 54 with a mass-to-charge-ratio of 2743.0 (F 54; m/z 2743.0) versus the signal intensity of the peptide in fraction 20 with a mass-to-charge-ratio of 1114.3 (F 20; m/z 1114.3). This plot exemplifies a pair of peptides showing no correlation.

FIGURE 24b shows a graph plotting the signal intensity of the peptide in fraction 54 with a mass-to-charge-ratio of 2743.0 (F 54; m/z 2743.0) versus the signal intensity of the peptide in the same fraction with a mass-to-charge-ratio of 1371.5 (F 54; m/z 1371.5). This plot exemplifies a correlation between a peptide-to-peptide pair consisting of a single charged and a double charged peptide ion.

FIGURE 24c shows a graph plotting the signal intensity of the peptide in fraction 54 with a mass-to-charge-ratio of 2743.0 (F 54; m/z 2743.0) versus the signal intensity of the peptide in fraction 56 with a mass-to-charge-ratio of 2927.2 (F 56; m/z 2927.2). This plot exemplifies a peptide-to-peptide pair exhibiting a functional relation.

FIGURE 25a shows a graph plotting the studentized signal intensity of the peptide in fraction 54 with a mass-to-charge-ratio of 2743.0 (F 54; m/z 2743.0) versus the studentized signal intensity of the peptide in fraction 20 with a mass-to-charge-ratio of 1114.3 (F 20; m/z 1114.3), i.e. the peptide pair of Figure 24a. A minimum spanning tree algorithm was performed to connect the nearest vertices. The path with the most vertices, i.e. the MST diameter, has been higlighted by a bold line. In this example, the path comprises 29 vertices.

FIGURE 25b shows a graph plotting the studentized signal intensity of the peptide in fraction 54 with a mass-to-charge-ratio of 2743.0 (F 54; m/z 2743.0) versus the studentized signal intensity of the peptide in the same fraction with a mass-to-charge-ratio of 1371.5 (F 54; m/z 1371.5), i.e. the peptide pair of Figure 24b. This plot exemplifies a correlation between a peptide-to-peptide pair consisting of a single charged and a double charged peptide ion. A minimum spanning tree algorithm was performed to connect the nearest vertices. The path with the most vertices, i.e. the MST diameter, has been highlighted by a bold line. In this example, the path comprises 50 vertices.

FIGURE 25c shows a graph plotting the studentized signal intensity of the peptide in fraction 54 with a mass-to-charge-ratio of 2743.0 (F 54; m/z 2743.0) versus the studentized signal intensity of the peptide in fraction 56 with a mass-to-charge-ratio of 2927.2 (F 56; m/z 2927.2), i.e. the peptide pair of Figure 24c. This plot exemplifies a peptide-to-peptide pair exhibiting a functional relation. A minimum spanning tree algorithm was performed to connect the nearest vertices. The path with the most vertices, i.e. the MST diameter, has been highlighted by a bold line. In this example, the path comprises 40 vertices.

FIGURE 26 shows a table where the peptides with coordinates Fraction 54; m/z 1371.5 and Fraction 56; m/z 2927.2 were tested according to a method of the present invention, whether the given peptides are potentially n times charged ions of the peptide with coordinates Fraction 54; m/z 2743.0.

FIGURE 27 shows the precursor sequence of the "Hypothetical Precursor" (HP) using a one-letter code. The sequence of the peptide HP 25-48 is underlined, the sequence of HP 25-50 is in bold letters.

FIGURE 28 shows an Averaged Peptide Display (peptide map) of CSF samples from 66 patients as discussed in Example 6. Each chromatographic fraction of each sample is analyzed by MALDI-ToF mass spectrometry; panel A: all 96 fractions generated from one sample are visualized as a "2-D gel-like picture" as shown in Figure 2. The x- and y-axis are mass-to-charge ratio (m/z) and chromatographic fraction, respectively; grey-scale bars in the "2D gel-like picture" represent the peptide peaks where the intensity of the grey scale corresponds to the mass spectrometric signal intensity, which corresponds to the relative quantity of the peptide measured by MALDI. The insets B and C provide an enlarged view of the marked boxes in panel A, indicating that some peptides are present in more than one fraction.

FIGURE 29 shows the Correlation-Associated Network of VGF 26-58 (1) as the network hub with VGF 177-191 (2), VGF 350-370 (3), VGF 26-59 (4, 5, adjacent fractions), VGF 23-59 (7), VGF 26-61 (8), VGF 26-62 (9), VGF 25-62 (10), VGF 485-522 (11) and VGF 373-417 (12-14, adjacent fractions). (6) is not a VGF peptide. Threshold of correlation was $|r| \geq 0.68$. The peptides are represented as bullets, and a peptide-to-peptide relation is displayed as a line, connecting two bullets. The network is projected onto the pertaining CSF peptide map of Figure 28. The numbers in the brackets are also shown in the figure (without brackets).

FIGURE 30 shows the network members of Figure 29 mapped onto the protein precursor sequence of VGF. The numbers correspond to those of Figure 29. The arrows span the beginning and end of the respective sequences of the peptides. The VGF 26-58 Peptide Network covers different parts spreading over the whole protein precursor.

FIGURE 31 shows the Correlation-Associated Network of Albumin 25-48 (1) as the network hub with Albumin 25-45 (2), Alpha-1-antitrypsin 397-418 (3), Albumin 25-48 (4,5, adjacent fractions), Albumin 27-50 (6,7, adjacent fractions) and Albumin 25-50 (8,9, adjacent fractions). Threshold of correlation was $|r| \geq 0.67$. The network is projected onto the pertaining CSF peptide map of Figure 28.

FIGURE 32 shows a table listing correct and false precursor predictions for any peptidepeptide relation as a function of threshold of correlation coefficient r.

FIGURE 33 shows the nomenclature used for peptide cleavages.

FIGURE 34 shows a table listing the numbers and percentages of amino acid residues found before amino-terminal, after amino-terminal (N+1), before carboxy-terminal (C-1) after carboxyterminal cleavage site (C+1) and at any position in all protein precursor sequences, e.g. Percentage(N-1)=n(N-1)/n(any position). Three out of the 139 peptides started at the beginning of the protein precursor, 34 peptides ended at the protein precursor sequence, therefore the sum of n(N-1) and n(C-1) deviate from 139. The percentage of an amino acid to be found in the respective positions is compared to the percentage at any position, indicating an x-fold increase or decrease of probability of a cleavage adjacent to that particular amino acid, e.g. x(N-1) = Percentage(N-1) /Percentage(any position).

FIGURE 35 shows a table listing the numbers and percentages of selected pairs of amino acids found before/after amino-terminal / carboxy-terminal cleavage sites and at any position in the protein precursor sequence. The percentage of a pair of amino acids to be found in the respective positions was compared to the percentage at any position, indicating an increase or decrease of probability, that this constellation influences cleavage.

FIGURE 36 shows a table listing two examples for predictions of peptide coordinates that were confirmed by the results of the ESI-MS/MS identifications.

FIGURE 37 illustrates the evaluation of the predictive power of the models: 139 peptides, all previously identified by ESI-MS/MS, were split into two groups. The first group of 70 peptides predicted the putative sequence of the second group. The peptide sequence information of the second group was suppressed during the prediction process. After completion of calculations, the putative proposals were confirmed by the results of the ESI-MS/MS identifications. The 139 different peptides corresponded to 224 different peptide coordinates on the Peptide Mass Fingerprint, since abundant peptides are present in more than one fraction.

FIGURE 38 shows a table listing percentages of correct proposals of precursor proteins and start-stop positions for six different models separately assessed for any stored proposal and for the proposal with most bonus points. At $|r| \geq 0.75$, proposals were generated for 27 out of 112 peptide coordinates. Since any peptide coordinate can store a list of up to three proposals, 81 proposals were generated.

FIGURE 39 shows a table with the peptides correlating to the peptide albumin 25-48 of CSF samples taken from patients with different severe disruptions of the blood-CSF barrier. Shown are the correlations r to the albumin quotient (complet albumin protein) of patients with damaged brain barrier (example 7, Figure 40), correlations r to the albumin 25-48 mass spectrometric signal intensity of patients with damaged brain barrier (example 6), the name of the peptides (Albumin 25-48, 27-50, 25-51 and alpha-1-antitrypsin 397-418) the theoretical monoisotopic mass of the peptides and the sequences of the peptides.

FIGURE 40 shows five plots of the relative MALDI singnal intensities of the peptides Albumin 25-48, 27-50, 25-50, 25-51 and alpha-1-antitrypsin 397-418 relative to the albumin quotient measured in patients with damaged brain barrier (example 7). In all cases there is a near linear relation between the MALDI signal intensity and the albumin quotient.

Detailed Description of the Invention

Prior to giving a detailed albeit exemplary description of embodiments of the present invention the following definitions are provided to establish how the technical terms are to be understood herein.

**Definitions**

Unless defined otherwise, all technical and scientific terms used herein have the meaning commonly understood by a person skilled in the art to which this invention belongs. As used herein, the following terms have the meaning ascribed to them unless specified otherwise.

"Sample" refers to any material, substance or the like containing or potentially containing peptides.

"Peptides" refers to polymers of amino acids coupled by peptide bonds comprising at least two amino acids. These amino acids can be the twenty standard amino acids and additionally unusual amino acids as known in the arts including D- and L- amino acids. Peptides can contain additional modifications such as posttranslational, enzymatic and/or chemical modifications.

"Status of a sample or an organism" means that the status or type of a sample at the time of the generation of the sample, e.g. the drawing of blood, is reflected by the contents and the activities of the sample. The actual status of an organism at the time of sample generation (such as the drawing of blood) is reflected in the contents and activities present in the sample. The sample conserves the status similar to a snap shot picture. A status for example can represent the presence or absence of a certain disease, the presence or absence of pregnancy, the sex of the individual from which the sample originated, the presence of a certain genetic variation such as the knockout of a gene or a polymorphism, the over expression or increased activity of a certain gene or gene product (for example as a consequence of a drug or of the transfection of the gene coding for the gene product or by direct addition of the gene product, etc.), the suppression of the expression or activity of a certain gene or gene product (for example as a consequence of a drug, anti-sense nucleotides, RNAi (RNA interface) nucleotides, ribozymes, triplex-forming nucleotides, antibodies, etc.), the presence of genetic modified ingredients in food, cosmetics or other products, the age of the organism from which the sample originated, the species of the organism from which the sample originated, a certain treatment of the organism from which the sample originated (for example with a therapeutically active substance a food ingredient or substance present in cosmetics, treatment with insecticides, pesticides or other toxic substances, etc.), the geographic origin of the sample, the development stage of the organism from which the sample originated (for example the stage of a fertilized egg, an embryo, an adult, intracellular/extracellular bacteria/virus, egg/larva/pupal/adult-stage of for example butterflies, different development stages of plasmodium, etc.), the metabolic state of the organism from which the sample originated (for example hibernation, stages of the circadian rhythm, etc.), the point of time before, during or after the treatment of an organism with a substance, the localization (or tissue) within the organism, from where the sample was taken, and the like.

"Measurement parameter of a peptide" refers to any parameter known to or measurable by the investigator such as the molecular mass of the peptide, the mass/charge ratio of the peptide, the signal intensity of the measured peptide, the actual concentration of the measured peptide, the fraction-number in which the peptide is present as a consequence of a certain separation protocol subjected to the sample, or the measured activity of the peptide.

"Correlation" or "relation" refers to a hypothesized mutual dependency of at least one parameter of two peptides, may this dependency be symmetric or asymmetric, known or not known, statistically significant or not. Relations of two peptides can be caused by chemical and biochemical reactions from one peptide to the other, by concerted gene regulation of the analytes, by common precursor peptides and so on.

"Measure of correlation", "correlation measure" or "measure of association" refers to statistical means to describe the symmetric or asymmetric statistical dependency of measurement parameters of pairs of peptides in terms of their "relation". Examples for measures of correlation are: "Pearson Product-Moment Correlation Coefficient", "Spearman's Rank-Order Correlation Coefficient", "Kendall's Tau", "Kendall's Coefficient of Concordance", "Goodman and Kruskal's Gamma", "Manhattan distance", "Euclidean distance" and "Minimal Spanning Tree Diameter".

"Correlation associated network (CAN)" refers to the complete network of all measures of correlation identified within samples representing one status or identified within different groups of samples representing different statuses. It is possible that more than two peptides correlate to each other and a CAN contains at least two peptides correlating to each other. It should be noted that the peptide CAN based on "a sample" does not necessarily comprise results obtained from a single experiment. Rather, to completely determine a peptide CAN, multiple experiments are often needed, and the combined results of which are used to construct the peptide CAN for that particular sample. The results of the calculation of a CAN (CAN of first order) can be used for another round of calculation of measures of correlation and so on. The results of these kind of calculations are also termed CANs or more specifically CANs of second or higher order.

"Peptide-topology" refers to the entirety of measured and computed peptide data of a sample ("measurement parameter of peptides") comprising the masses of the peptides, the signal intensity of the peptides (preferably measured by mass spectrometry or another measurement method suitable to quantify peptides), the fraction number (if the sample was fractionated prior to mass spectrometry) and measures of correlation calculated using these data.

"Groups of samples" refers to a set of samples corresponding to a certain status. A group of samples for example could comprise 10 plasma samples of diabetic patients. The samples of a group need not to be of exactly the same origin. For example a group of samples may also comprise 5 plasma samples of diabetic patients and 5 urine samples of diabetic patients. The reason for this being that many peptides present in plasma are also present in urine and for example the same diabetes-specific peptides may be present in plasma and urine, as long as the sample originates from a diabetic patient.

"Known peptide" means that the peptide with that particular sequence or part of a sequence in the sample is known to the user of the invention. An unknown peptide is a peptide whose sequence is not known to the user of the invention, although the sequence of the peptide may be known from the literature or other sources of information such as sequence data bases.

"Potential peptide" refers to a mass spectrometric signal which most likely represents a peptide.

"Precursor of a peptide" refers to the longest amino acid sequence present in nature comprising the sequence of the peptide, i.e. form which the peptide can originate.

"Coordinate(s) of a peptide" refer to the mass-to-charge ratio and optionally further specific measurable properties obtainable by a detection or identification process that are involved in the identification and/or quantification of the said peptide/peptide ion. In the examples of this invention the peptide coordinates are the elution time/fraction number of a chromatographic process and the mass-to-charge ratio, thus comprising of two coordinates. In this invention, these coordinates often are written in a short form, such as "F 56; m/z 2873.0", which identifies the signal of a peptide found in fraction 56 with the mass-to-charge ratio 2873.0. Of course, further dimensions can be necessary, such as a previous capillary electrophoresis, or a downstream second mass spectrometric process. "Coordinates of a peptide", "signal coordinates" or "peptide" are often used synonymously.

"Fitness value" refers to an assessment of a predicted sequence based on the experimental properties of an unknown peptide. Any predicted sequence gains points for properties that match the experimental properties, such as the correct prediction of the fraction number. The higher the "fitness value", the more probable the correctness of the predicted sequence. According to the present invention fitness values are manually or automatically suggested for each sample type and empirically tested for suitability.

"Landmark peptides" refers to peptides that are related to numerous other peptide signals and least related to each other. The identification, e.g. sequencing, of these landmark peptides should be prioritized to gain a rapid overview about the peptide composition of a sample.

**Providing the data**

Figure 1 shows schematically the hardware components and software modules according to the present invention, their interfaces and the flow of information between the hardware components and the software modules. Although measurement data could be provided without fractionating the samples prior to performing mass spectrometry, such a fractionation e.g. by chromatography into e.g. 96 fractions is preferred. A "fraction" in terms of chromatography is a part or the effluent recovered during a separation step. Usually, several fractions are collected. Fractions usually contain different "subsets" of peptides from the sample. Suitable separations methods for peptides are chromatographic methods such as ion exchange, hydrophobic interaction, iso-electric focusing, gel filtration or affinity chromatography, electrophoretic methods such as native, iso-electric, denaturizing or SDS-gel electrophoresis using matrixes such as polyacrylamide or agarose gels, paper electrophoresis, thin layer chromatography, capillary electrophoresis, methods using centrifugation for separation such as sucrose or Caesiumchloride gradient centrifugation and the like. These chromatographic fractions are then subjected to a measurement of the mass spectrum providing 96 mass spectra, which can be visualized e.g. in a 2D gel-like format as shown in Figure 2. To this end all kinds of methods suitable to determine masses of peptides and preferably all kinds of mass spectrometry can be used in the present invention such as matrix-assisted laser desorption time of flight (MALDI-TOF) mass spectrometry, liquid chromatography electro-spray ionization (ESI) quadrupole time of flight mass spectrometry (LC-ESI qTOF) and the like. It is furthermore possible to analyze only selected but not all fractions by mass spectrometry.

Each bar in Figure 2 depicts a peak present in one of the 96 mass spectra, wherein the colour intensity of the bar corresponds to the intensity of the corresponding mass spectrometric signal. The x-axis of Figure 2 represents the mass to charge ratio m/z and the y-axis the chromatographic fraction number. The m/z values preferably range from 1.000 to 15.000, although higher or lower m/z values can be included as long as these values can be resolved by mass spectrometry or other methods. Within this range of m/z values the detected peptides can be comprised of only two amino acids at the lower end up to peptides of very large molecular mass such as alpha-2 macroglobuline having a molecular mass of 725 kDa.

Similar 2D gel-like maps are produced for every sample out of the set of samples to be analyzed. These maps can be averaged yielding an averaged peptide mass fingerprint map as shown in Figure 2. This averaged map serves as a template for the definition of usually about a thousand peak coordinates, i.e. the x-coordinate corresponding to the m/z value and the y-coordinate corresponding to the fraction number. In practice one selects those peak coordinates exhibiting a signal above a certain threshold value.

**Data Pre-processing**

In order to obtain measurement data that is suitable for a correlation analysis and that gives meaningful results preferably a pre-processing of the data is performed using methods such as baseline correction, spectra normalization, outlier detection and the like. Methods for baseline correction are well known in the art (e.g. Fuller et al, Applied Spectroscopy, 42, 217 1988). In a preferred embodiment the pre-processing of the data is performed by applying the baseline correction being part of the software RAZOR Library 4.0, Spectrum Square Associates, Ithaca NY, USA. Optionally a normalization of the mass spectra can be performed by using the signal intensities or the integrated mass spectra. Outlier samples can be identified by means of a principal component analysis as provided by the commercially available software package Pirouette 3.0, Infometrix Inc., WA, USA. Based on this principal component analysis individual mass spectra or even whole samples that exhibit a Mahalanobis distance $M_D$ above a critical threshold value of should not be considered for a further analysis and thus be discarded. In the examples described further below a Mahalanobis distance of $M_D > 11.5$ was chosen for 74 samples.

The preprocessing, processing and display of the data according to the present invention can be performed e.g. on a Apple G4 Computer, wherein the CPU consists of 2 processors with 800 MHz each and the memory size is 1.25 Gigabyte. The local data storage of peptide-to-peptide relations (measures of correlation, coordinates of peptides) is accomplished by a local Valentina Database system (Valentina 1.9 for Realbasic, Paradigma Software, Beaverton, Oregon, USA). The peptide sequence information is provided by a proprietary Interbase Server (Interbase 6, Borland Software Corp., Scotts Valley, CA, USA). Microsoft Internet Explorer 5.1 for Apple computer systems can be used for representation of results from internet resources. The CAN software launches the Internet explorer with a specific address that contained the keywords for querying the Swiss-Prot, the PubMed, and the US Patent database. Visualization of three-dimensional objects can be performed using a Realbasic RB3D engine (RealBasic 3.5, RealSoft, Austin, Texas, USA).

Other digital computer system configurations can also be employed to perform the methods of the present invention, and to the extent that a particular system configuration is capable of performing the method of this invention, it is equivalent to the representative digital computer system schematically shown in Figure 2. Once they are programmed to perform particular functions pursuant to instructions from program software that implements the methods of the present invention, such digital computer systems in effect become special-purpose computers particular to the methods of this invention.

Computer programs implementing the methods according to the present invention will commonly be distributed to users on a distribution medium such as floppy disk or CD-ROM. From there, they will often be copied to a hard disk or a similar intermediate storage medium. When the programs are to be run, they will be loaded either from their distribution medium or their intermediate storage medium into the execution memory of the computer, configuring the computer to act in accordance with the method of this invention. All these operations are well-known to those skilled in the art of computer systems. The term "computer-readable medium" encompasses distribution media, intermediate storage media, execution memory of a computer, and any other medium or device capable of storing a computer program implementing the methods of this invention for later access by a computer.

**Correlation Associated Network Module**

As exemplified by the arrows in Figure 1 the raw measurement data or preferably the preprocessed measurement data is supplied to the so called Correlation Associated Network (CAN) Module 42. Of the modules 40 of the present invention the CAN Module 42 is the most fundamental one. Basically the CAN Module 42 scans the measurement data obtained for example from Liquid Chromatography-Mass Spectrometry (LC-MS) experiments 22. On the basis of this data correlations of peptides are searched for by calculating measures of correlation between for example their relative concentration as measured by mass spectrometry.

Measures of correlation can be used to represent the degree of relationship between two variables throughout many observations. These variables can be either correlated, not correlated or anti-correlated. In the context of the present invention measures of correlation are used to detect such correlated, not correlated or anti-correlated peptides in a set of samples. This can be done e.g. by calculating Spearman's rank-order correlation coefficient of the signal intensities of two peptides measured in several samples. Preferably this is done for all pairs of peptides. Once these measures of correlation have been calculated only those pairs of peptides are selected that exhibit a certain behaviour, i.e. a certain degree of correlation, a certain degree of anti-correlation or a certain degree of no correlation at all. The parameters of such selected peptide pairs, e.g. the coordinates of the two peptides of each peptide pair, the measure of correlation, etc., can be stored, displayed on a display device or further processed. Preferably the data is stored in a database, as a text file or in another computer-readable form. Alternative measures of correlation to Spearman's rank order correlation coefficient are Pearson Product-Moment Correlation Coefficient, Kendall's Tau, Kendall's Coefficient of Concordance, Goodman and Kruskal's Gamma and Minimal Spanning Tree diameters.

A Minimal Spanning Tree (MST), also known as Minimum Spanning Tree, is defined by the collection of edges that joins together all points in a connected set of data points, with the minimum possible sum of edge values (e.g. Evan, Graph Algorithms, Computer Science Press, 1979). An edge can be graphically displayed by a line connecting two data points. A MST can be graphically displayed by a set of points (data points) connected by a minimum of lines to each other. Examples of MSTs are shown in Figures 25a to 25c as described in more detail further below. A MST also provides a plausible "connectionist" approach to solving the "Traveling Salesman" problem (e.g. Kruskal, Proc. American Math. Soc., 7, 48-50, 1956; Sun et al, Physica A, 199, 232-242, 1993), which identifies the minimum connected path between all data points. The MST diameter can be defined as the maximum number of edges in the paths of a graph of a MST. Usually a correlation, for example a Spearman's rank order correlation coefficient, is used to find a measure of correlation or association or dependency between variables, i.e. data points. A problem is that correlation is sensitive to linear trend, and linear trends are not always well presented for two associated variables. In the present invention, the diameter of the MST is used as an alternative measure of correlation between two variables. In order to use the diameter of the MST to analyze a given set of n statistical observations, all observations should be connected via the MST and then the MST diameter should be calculated. The larger the MST diameter is, the stronger is the association between two variables. In the context of mass spectrometry signal intensity data (in the present invention preferably MALDI mass spectrometry signal intensity data) it was found, that MST diameters > 0.425 times n indicate a noticeable association between signal intensities of peptide coordinates. In general all kinds of mass spectrometry signal intensity data, such as MALDI or ESI mass spectrometry data, can be used according to the present invention.

As already mentioned pairs of peptides are tested for their degree of correlation by estimating e.g. Spearman's rank order correlation coefficients between their signal intensities throughout many observations. It turns out that pairs of peptides which are biologically or functionally related surprisingly often exhibit correlation coefficients that are much higher than correlation coefficients that would be expected by chance. Unrelated pairs of peptides have low absolute values of correlation coefficients. Figure 3 exemplifies the correlational behaviour of related peptides. Four traces of spectra from four different samples are focused on the signals of a human osteopontin peptide being comprised of the amino acids 249-314 of human osteopontin (m/z = 7653.6 Da) and its phosphorylated derivatives, carrying one (m/z = 7733.5 Da), two (m/z = 7813.5 Da), three (m/z = 7893.4 Da) or more phosphorylated residues. The conserved concentration ratios of the peptides between samples leads to high degrees of correlation of the signal intensities of the respective peptide pairs.

Using the results of the above described computations of measures of correlation so called correlation associated networks (CANs) can be defined. A CAN, i.e. a network of peptide relations, comprises a peptide of interest, the so called hub peptide, and all those peptides and sample parameters that correlate to a certain degree with the hub peptide. The term hub is used in a similar manner in the theory of network topology and is to characterize the resemblance of a hub peptide to the hub of a wheel, the hub peptide being at the center of the spokes representing the peptide-to-peptide relations and the correlating peptides being at the respective ends of the spokes. In practice, the composition of a CAN is highly dependent on the threshold of correlation as selected by the user. This threshold is chosen according to the goal of a user. If a user is searching for peptides that strongly relate to a peptide of interest, such as peptides stemming from the same precursor, then he will select a threshold that will cause a selection of only the upper 5 % of the strongest correlations with the peptide of interest. The threshold value to be chosen for e.g. the Spearman's rank order correlation coefficient depends for the thus selected subset on the number of samples and the peptide of interest. In case the user is interested in finding functionally related peptides, such as e.g. peptides being co-secreted from vesicles, the user will choose a threshold value, that will select e.g. the upper 10 % of the strongest correlations.

The hub peptide and the peptides related thereto and selected as described above represent a CAN of first order. Depending on the objective it can be necessary to compute CANs of higher order due to the complexity of biological networks and pathways. As explained above, CANs connect directly related peptides which exhibit a high degree of correlation. Adjusting the threshold to lower values results in including more loosely related peptides into the network as well as increasing the probability of predicting false relations. For this reason a preferred embodiment of the present invention contemplates the computation of CANs of higher orders, such as e.g. second and third order. Since the direct members of a network of interest constitute the first order neighborhood, all these members are potential starting points for the calculation of second order neighborhoods as shown in Figure 4. Although computing CANs of higher order certainly will improve the results, the computational requirements set an upper limit, because the computational effort increases with the order of the CANs. A calculation of a CAN of the $n^{th}$ order, where n is greater than 5, can require more than several millions of calculations. Thus, this approach preferably should be used for the analysis of rather complex samples in order to include indirectly related peptides, thus avoiding having to decrease the value of the correlation threshold and possibly including false relations.

For any kind of sample the composition of peptides varies, novel peptide coordinates emerge, others disappear and many peptide coordinates have a different peptide sequence aligned to it. This results in dealing with many unknown peptide coordinates when operating with novel sample sources (types of samples). In order to accelerate analysis of an interest list or more general, to analyze the overall peptide composition of a sample, according to the present invention it is possible using CANs to accelerate the identification of peptides in complex biological samples by defining a list of representative peptides, so called landmark peptides, for further analysis such as peptide sequencing based on CANs described further below. The method comprises the following steps, which are shown in Figure 5. At step 80 mass spectra are provided as described above, wherein the peaks of the signal intensities in the mass spectra correspond to potential peptides. Then the measures of correlation between the measured signal intensities corresponding to potential peptides are computed (step 82). Thereafter at step 84 those peptides are grouped together that exhibit a degree of correlation above an adjustable threshold. These selected peptides constitute a CAN present in the samples analyzed. Finally, one peptide out of each determined CAN is assigned to represent that respective CAN at step 86. In doing so a plurality of landmark peptides is provided being representative of the analyzed samples. These landmark peptides have the properties of being hub peptides and they are least related to other peptides within the same type of samples.

Identifying a list of these landmark or prioritized peptides gives a rapid overview about the peptide composition present in complex biological samples, omitting the majority of similar peptides from for example the same precursor peptide. This is useful to obtain a general overview of the key peptides present in a sample or present in an interest list from that sample.

It is contemplated that a such generated interest list of prioritized landmark peptides will contain a set of n peptide coordinates, and for any peptide z the number of relations, which the peptide z has at a defined threshold r, $k_{z,r}$, will be determined. The peptide z with the highest value of $k_{z,r}$ will be defined as y and be rank 1 etc. on the prioritization list. Then the variance of signal intensities of that determined peptide coordinate y will be removed from the signal intensities of the related peptides x in a data matrix, for example by a combination of formulas 1, 2 and 3 shown below. Then this peptide will be removed from the data matrix. Calculations of any k and r start from the beginning to determine the representative peptide ranked number 2 in the prioritization list, and so on. Calculations end for example when the data matrix contains no more peptide coordinates, or no peptide has more than zero relations, or the number of peptide coordinates desired has been reached.

***Formulae 1 to* 3: *Removal of variance of the representative peptide coordinate y on peptide coordinate x***

$$X_{VR,p} = X_p - a_{xy} - b_{xy} Y_p \qquad (1)$$

where

$X_{VR,p}$ : Signal intensity of peptide x at observation p, Variance of peptide y removed
$X_p$ : Signal intensity of peptide x at observation p
$Y_p$ : Signal intensity of peptide y at observation p
m : number of observations

$$a_{xy} = \frac{\left(\sum_{p=1}^{m} X_p\right)\left(\sum_{p=1}^{m} Y_p^2\right) - \left(\sum_{p=1}^{m} Y_p\right)\left(\sum_{p=1}^{m} X_p Y_p\right)}{m\sum_{p=1}^{m} Y_p^2 - \left(\sum_{p=1}^{m} Y_p\right)^2} \qquad (2)$$

and

$$b_{xy} = \frac{\sum_{p=1}^{m} X_p Y_p - \frac{1}{m}\left(\sum_{p=1}^{m} X_p\right)\left(\sum_{p=1}^{m} Y_p\right)}{\sum_{p=1}^{m} Y_p^2 - \frac{1}{m}\left(\sum_{p=1}^{m} Y_p\right)^2} \qquad (3)$$

It is further contemplated that peptides being part of a CAN preferably are represented by graphical objects such as e.g. bullets and their mutual relations by lines connecting these bullets. In order to enable a more intuitive analysis of the results, this network can be projected onto a peptide map as shown in Figure 6. Peptides that have been identified can be provided with links to databases, with lists containing additional information about these peptides or with other sources of additional information regarding said peptides.

The coordinates or measurement parameters of related peptides can be queried in public, commercial and/or proprietary databases in order to identify further data about the potential identity, function or use of the corresponding peptides. Suitable public databases include e.g. the PubMed literature database, the OMIM disease database, the NCBI-Sequence database (all provided by the US National Library of Medicine, MD, USA), the Swiss-Prot and TrEMBL Sequence database, enzyme database, Swiss 3D image database, Prosite protein family and domain database (all provided the Swiss Institute of Bioinformatics, Switzerland), patent databases of the US, European, Japanese, German patent offices, the Gene Cards database of the Weizmann Institute, etc. Suitable commercial databases are for instance commercial patent databases containing patented amino acid or nucleic acid sequences such as DGENE (Thomson Derwent, USA) or REGISTRY (Chemical Abstracts Service, USA). A suitable proprietary database is the database of the user containing peptide sequences from various sources and species. This combination of the visualization of peptide networks and the connection to many sources of information alleviates the evaluation of the identified peptides for potential uses such as their use as therapeutic peptides or as biomarkers as will be described in more detail further below.

As is apparent from the above, correlation associated networks can be used to generate hypotheses about relations between structurally and/or biologically related peptides. These hypotheses are based on a correlational analysis of signal intensities and corresponding relative peptide concentrations from independent samples. The examples described in the sections further below will demonstrate that correlation associated networks are powerful tools for the systematic analysis and interpretation of large peptidomic and proteomic data in order to reveal functional relationships governing protein synthesis, posttranslational modifications and degradation. CANs support the discovery of novel bioactive and diagnostic peptides leading beyond the mere comparison of peptide concentration changes caused by a disease.

According to the present invention the CAN Module 42 is interacting with several application modules 44 comprising a Sequence Network Module 46, a Differential Network Module 48, a Marker Panel Network Module 50 and a Surrogate Network Module 52 as shown in Figure 1. These application modules 44 of the present invention and their interaction with the fundamental CAN Module 42 will be described in detail in the sections below.

**Sequence Network Module**

The interaction of the Sequence Network Module with the fundamental CAN Module according to the present invention allows to predict the amino acid sequences of unknown peptides with or without modifications of the sequence and/or to predict unknown modifications of a known or unknown peptide sequence. Although the identity of the peptide is unknown, certain physicochemical and biochemical properties of the signal of an unknown peptide are known and can be exploited for amino acid sequence prediction such as the mass-to-charge ratio (m/z) or the chromatographic behaviour (fraction number/retention time). Furthermore bioinformatic support data shown at 56 in Figure 1 such as the correlation associated network of related peptides, mass differences and differences in fraction number between the peptides of the correlation associated network, and the like are accessible as they can be computed using experimental data and the amino acid sequences of other members of the correlation associated network possibly already known.

Figure 7 shows a flow chart schematizing the procedural steps of an interaction of the Sequence Network Module with the CAN Module according to the present invention allowing the prediction of the sequence of peptides using the peptide topology of a plurality of samples containing a peptide having a known precursor. At step 80 a respective mass spectrum for each sample of said plurality of samples is provided, wherein the signal intensity peaks correspond to potential peptides. Thereafter at step 88 the peptide having a known precursor is identified using the mass of said peptide, wherein the sequence of the known precursor is known. Then measures of correlation between the signal intensity of the peptide having a known precursor and the signal intensities of the other potential peptides are computed at step 90. At step 92 those potential peptides are selected, which exhibit a degree of correlation with the peptide having a known precursor above a certain adjustable threshold, and finally the sequence of the potential peptides are predicted at step 94 by matching masses of putative fragments of the sequence of the known precursor with the masses of the potential peptides correlating with said peptide having a known precursor.

Alternatively after step 92 the mass differences between each of the potential peptides and the known peptide can be computed at step 96, and thereafter the sequence and/or the biologically, chemically or physically modified sequence of the potential peptides predicted at step 98 by using data about mass differences caused by biological, chemical or physical processes matching the mass differences determined in step 96.

The first of the above approaches is more comprehensive, since all plausible putative sequences are generated from the precursor sequence of the known peptide (steps 90-98). The second approach (steps 90-96, 100-102) generates fewer but more reliable predictions. It has been observed that related peptides very often have very similar sequences/ sequence modifications, and these predictions are promoted by the second approach. Nevertheless, since both approaches have steps 90-96 in common, computational power is "saved" if both approaches are combined in one operation, as contemplated in the present invention.

Mass differences may result from addition or removal of N- or C-terminal amino acid residues or of postranslational modifications of amino acid side chains such as phosphorylation, amidation, sulfatation, glycosylation, fatty acids or Ubiquitin modification, and the like or chemical modifications such as oxidation, disulfide bonding, and the like or Nor C-terminal modifications such as pyroglutamate modifications and the like. All of these modifications result in distinct increases or decreases of the molecular mass of the corresponding peptide. Also internal insertions or deletions or the exchange of one amino acid for another, so called point mutations, result in exactly predictable mass changes of the peptide.

According to the present invention the prediction of sequences is possible regardless of whether the identity of one of the related peptides is known or not. Especially if the identity of one peptide is known, mass differences corresponding to the molecular masses of amino acid residues allow to directly predict the complete sequence of the unknown peptide with high reliability. If the identity of no peptide is known, than for example it can be predicted that the unknown peptide 1 and the unknown peptide 2 are identical, except that for example peptide 2 contains an additional amino acid residue, for example a Tyrosine residue, or for example peptide 2 is the same peptide as peptide 1 except that it is phosphorylated, etc. The prediction is not always correct, but the more independent information is accessible, the more reliable the prediction becomes. For example if the mass difference fits to the addition of an Tyrosine amino acid residue and the peptide is present in a fraction, which fits to the prediction of the fraction-shift of a peptide with an additional Tyrosine residue, the overall reliability of the prediction increases.

For this embodiment the use of proprietary and/or commercial and/or public databases is possible. Suitable databases are for example databases containing amino acid or nucleic acid sequence information such as the NCBI sequence data base, Swiss-Prot, the EMBEL sequence data base, the DNA data base of Japan, data bases of patented sequences, and the like, data bases containing information about the structure of carbohydrates, such as PROSITE (Falquet et al, Nucleic Acids Res., 30, 235-238, 2002), data bases containing information about postranslational, enzymatic or chemical peptide modifications such as phosphorylation sites of peptides, glycosylation sites of peptides, positions of unusual amino acids such as hydroxy-proline or hydroxy-lysine within peptides, databases containing information about recognitions sites of proteases, ligases, phophatases, kinases, and the like within peptide sequences, databases containing information about the susceptibility of certain amino acids or sequences of amino acids towards chemical modifications such as oxidation, reduction, intra-molecular rearrangement, data bases containing data about three-dimensional structures about peptides, carbohydrates or other biological structures, and the like (Falquet et al, Nucleic Acids Res., 30, 235-238, 2002). All of these different kinds of databases enable to predict the structural difference between peptides, based on certain incremental increased or decreased molecular masses of these peptides. For example:

(i) amino acid sequences stored in databases allow the calculation of the masses of successive shortened or extended peptides or of peptides containing mutations of their sequence

(ii) databases containing for example recognition sites (sequences) of kinases allow to predict, that the molecular mass of a certain peptide, containing such a recognition site, may have a molecular weight increased or decreased by the mass of a phosphate group

(iii) data bases of recognition sites of proteases allow to predict the molecular masses of potential proteolytic fragments of a certain peptide

(iv) databases containing experimental data about physical properties of peptides such as elution times during for example hydrophobic interaction chromatography allow to predict, if a certain peptide sequence with a certain molecular mass is likely to elute at a certain time point during chromatography

(v) databases containing prediction values of chromatographic retention times or fraction numbers based on the amino acid composition and/or sequence of the peptide: if a certain chromatographic column is used, a peptide with an additional tyrosine residue would elute 3 fractions later than a peptide without that additional tyrosine residue. For example a peptide I in fraction x with mass y is known and a related peptide II within fraction x+3 has the molecular mass y plus the mass of a tyrosine residue. This would indicate with high reliability that peptide II is the same peptide as peptide I, except that it contains an additional tyrosine residue somewhere within its sequence

(vi) databases of three-dimensional structures of for example peptides allow to predict, if there is for example space enough at a certain amino acid side chain to be modified for example by a phosphate group or a sugar moiety, resulting in an increased molecular weight of the potential corresponding peptide

The prediction of physicochemical and biochemical properties of putative amino acid sequences fit surprisingly well to experimentally determined properties. This approach can be extended by utilizing knowledge about precursor amino acid sequences and posttranslational, chemical and enzymatic modifications of known related peptides as provided by the support data 56 shown in Figure 1 and as discussed above. Furthermore, information about a known peptide such as the name of its precursor, its precursor sequence, its start and end-position within the precursor sequence can be retrieved before or during the prediction processes. Information about protease recognition sites, predictions of domains, and structures sensitive to proteolytic digestions can also be retrieved. This information can be supplied manually, from databases or lists or from a comparable source of information. A conversion of mono-isotopic m/z ratio to average m/z ratio, from the m/z ratio of the charged ion to the m/z ratio of the un-charged ion within a reasonable error tolerance is known to those skilled in the art.

The invention comprises specific rules, which determine if a putative amino acid sequence derived according to one of the methods described above fits to the peptide signal coordinates of an unknown peptide. These rules which are schematically shown in Figures 8a to 8d can be applied in any order and it is not necessary to apply all of them in any given case:

Rule a:

This rule applies formula 4 (shown below) to check, whether the unknown peptide coordinate is an n-fold charged ion of the known peptide coordinate by the following condition, where n can be an integer number greater than 1, $m/z_{unknownpeptide}$ is the m/z ratio of the unknown peptide, $m/z_{knownpeptide}$ is the m/z ratio of the known peptide and $Mass_{Threshold}$ is a maximum difference of the calculated mass from the measured mass. A preferable $Mass_{Threshold}$ equals the mass precision of the instrument and the subsequent data processing routines. If this condition is met, the proposal is rewarded with a high fitness value and the proposal that the unknown peptide is the n-fold charged ion of the known peptide can be stored.

***Formula 4: Check for n times charged peptide ions***

$$\left| n * (m / z_{unknownpeptide} - 1) - m / z_{knownpeptide} \right| = Mass_{deviation} \leq Mass_{threshold}$$

wherein the asterix (*) indicates the mathematical operation of multiplication.

Rule b:

If the difference of the masses of a known hub peptide P1 and a related peptide P2 corresponds to a mass of an post-translational modification, as listed for example in the table "Mass Changes Due to Post-translational Modifications of Peptides and Proteins" shown in Figure 9 or as known from the prior art (Falquet et al, Nucleic Acids Res., 30, 235-238, 2002), then P2 is proposed to be the post-translationally modified derivative of P1. If the amino acid sequence of the known hub peptide P1 contains specific sites for posttranslational modifications or it is known that P1 is or can be posttranslationally modified, and if the mass difference between the known and the unknown peptide corresponds to the mass difference resulting from the presence or absence of that posttranslational modification, the fitness value is increased. The table shown in Figure 15 exemplifies motifs, enzymes recognizing these motifs and the resulting mass differences. Numerous other postranslational modifications or putative sequence motifs bearing certain post-translational modifications are known in the prior art and could be used as well such as N-glycosylation or O-glycosylation sites (motifs), phosphorylation sites, sulfatation sites, and the like (e.g. Alberts et al, Molecular Biology of the Cell, Garland Publications, 2002; Coligan et al, Short Protocols in Protein Science, John Wiley & Sons, 2003; Falquet et al, Nucleic Acids Res., 30, 235-238, 2002).

Rule c:

Putative sequences or putative fragments are generated from potential amino- and carboxy-terminal truncations or additions of amino acids of the known precursor sequence of the hub peptide and are checked whether they match the found m/z ratio of the unknown peptide coordinate. A putative sequence is generated by systematically and iteratively defining start-and end-positions, i and j, in the given precursor sequence of the hub peptide, as exemplified in Figure 8b. The mass of the putative amino acid sequence $M_{CALC}$ is calculated by summing up the masses of the amino acids, the hypothesized postranslational modifications of the amino acid residues and/or of the terminal groups of the putative amino acid sequence (see the tables in Figures 9, 11 and 11 and formula 5 shown below). Rule c defines that if the calculated mass differs from the measured mass $M_{FOUND}$ of the unknown peptide signal by less than a given mass threshold $T_{Mass,}$ this putative amino acid sequence plus posttranslational modifications are proposed and further rules d to i can be applied, otherwise this proposal is rejected. This can be done with one or more putative peptide sequences or with all hypothetically possible peptide sequences that can be deduced from the precursor sequence of the known related peptide signal coordinates.

***Formula 5: Calculation of Masses***

$$M_{CALC} = n_A * M_A + n_R * M_R + n_N * M_N + n_D * M_D + n_C * M_C + n_E * M_E + n_Q * M_Q + n_G * M_G + n_H * M_H + n_I * M_I + n_L * M_L + n_K * M_K + n_M * M_M + n_F * M_F + n_P * M_P + n_S * M_S + n_T * M_T + n_W * M_W + n_Y * M_Y + n_V * M_V + M_{N\text{-}Terminal\ Group} + M_{C\text{-}Terminal\ Group} + M_{Modifications}$$

wherein:

$M_{CALC}$ is the calculated mass of the peptide with the given/putative sequence,
$M_{ONE\ LETTER\ AMINO\ ACID\ CODE}$ is the mass of the appropriate amino acid,
$n_{ONE\ LETTER\ AMINO\ ACID\ CODE}$ is the number of the appropriate amino acid in the given/putative sequence,
$M_{N\text{-}Terminal\ Group}$ is the mass of the N-terminal group,
$M_{C\text{-}Terminal\ Group}$ is the mass of the C-terminal group, and
$M_{Modifications}$ is the mass change by modification(s), in the case of no modification
$M_{Modifications} = 0$.

Rule d:

The number and the identity of amino acids influence the elution time/fraction number, depending on the size and the kind of the chromatography column used and the chromatography conditions. The fraction number/elution time of a peptide can be surprisingly well predicted on the basis of its amino acid sequence by the so called Group Method of Data (GMDH, e.g. Mueller and Lemke, Self-Organising Data Mining Extracting Knowledge From Data, Trafford Publishing, 2003), multiple regression or comparable mathematic methods with a training set of peptides with known sequences, which are separated under the same chromatographic conditions as exemplified in Formula 6 shown below. In the said training set, the number of any amino acid residue type of a peptide is the independent variable whereas the fraction number of the peptide is the dependent variable. If the calculated fraction number (e.g. Formula 6) of the predicted amino acid sequence matches the derived fraction number of the unknown peptide within a given error tolerance, then the model fitness points are increased. If the mass differences are proposed to be resulting from distinct amino acid deletions/additions and if the differences in fraction number can be matched with these said amino acid sequence differences (see Figure 13), the model fitness points are increased.

***Formula 6: Estimation of fraction number based on proposed sequence***

$$F_{CALC} = 35.89 - 0.45 * n_S + 0.47 * n_E + 2.86 * n_I - 3.82 * n_H + 5.15 * n_L + 5.54 * n_F + 2.92 * n_Y - 1.72 * n_K - 0.85 * n_Q + 5.35 * n_W + 2.20 * n_V$$

wherein:

$F_{CALC}$ is the calculated Fraction number of the given sequence, and
$n_{ONE\ LETTER\ AMINO\ ACID\ CODE}$ is the number of the appropriate amino acid in the given sequence.

Rule e:

If the N-terminal position of the predicted amino acid sequence is the same as the N-terminal position of the known peptide, the fitness value is increased. This is because the known peptide and the unknown peptide of the underlying signals are related via a C-terminal proteolytic reaction, which is observed surprisingly often.

Rule f:

If the C-terminal position of the predicted amino acid sequence is the same as the C-terminal position of the known peptide signal, the fitness value is increased. This is because the known peptide and the unknown peptide of the underlying signals are related via an N-terminal proteolytic reaction, which is observed surprisingly often.

Rule g:

If the start position and/or the end-position of the predicted sequence is preceded or followed by sites of infrequent proteolytic events, the fitness value of this proposal is decreased. If the start position and/or the end-position of the predicted sequence is preceded or followed by sites of frequent proteolytic events, the fitness value of this proposal is increased. This is because it has been observed that peptides are often products of specific and/or unspecific proteases. Depending on the source and preparation procedure of the samples, proteases and intra-molecular rearrangements, such as disulfide bonding, can vary. With for example liquor cerebrospinalis (CSF) as sample source, the sequences "R-R" or "R-K" are frequently preceding a peptide’s N-terminal position in a precursor as they are recognition sites of the prohormone convertase PC2 in CSF. Next to known enzyme recognition sites, some amino acids are more frequently and others are less frequent. Positions preceding or following N-and C-terminal positions of peptides can be predicted on the basis of their mere percentage occurrence in a particular sample treated in that particular way. This kind of information can easily be determined empirically and an example for peptides present in human liquor cerebrospinalis is shown in the tables in Figures 14a to 14d. The tables "CSF: amino acid Before First Cleavage", "CSF: amino acid After First Cleavage", "CSF: amino acid Before Last Cleavage", and "CSF: amino acid After Last Cleavage" summarize empirically found Nor C-terminal amino acid frequencies as a result of proteolytic processes. Rule h increases the fitness value when those amino acids at the top of the tables shown in Figures 14a to 14d are present at the corresponding positions in the predicted sequence, while those amino acids at the bottom of these tables decrease the fitness value of the prediction. The tables shown in Figures 14a to 14d are suitable to predict the likelihood of the presence of certain amino acid residues at the N- or C-terminus of peptides present in human liquor cerebrospinalis as long as the CSF samples are treated in the same way as the CSF samples of the examples of the present invention. Tables similar to the ones shown in Figures 14a to 14d can be generated empirically for any sample such as whole blood, serum, plasma, urine and the like, and the treatment of the samples can be of any kind, as long as all samples are treated in the same way.

Rule h:

If the mass difference between the peptide coordinates of a known and an unknown peptide can be explained by the loss of one or more distinct N- or C-terminal amino acids, the fitness value of this prediction is increased.

Rule i:

If a prediction has been generated by one of the rules b to h or a combination thereof, proposing that the unknown peptide is a reactant or a product of a post-translational modification of the known peptide, this proposal is tested by determining in terms of accessibility of the reaction site within the protein sequence by an enzyme performing the given post-translational modification. Thus, if a look-up in a database storing three-dimensional data of peptides or proteins reveals that the proposed site is on the surface of the protein and/or its conformation sterically allows action of that enzyme, the fitness value of that prediction is increased. In the same way, if a region of a sequence is proposed to be modified by a post-translational modification process, the accessibility of that sequence region to enzymes is assessed by means of algorithms estimating the hydrophobicity of that particular region (Engelman et al, Ann. Rev. Biophys. Chem., 15, 321, 1986; von Heijne, Eur. J. Biochem., 116, 419, 1981). For example, a highly hydrophilic sequence region is more likely to be accessible by enzymes performing post-translational modifications than a hydrophobic sequence region, thus the fitness value of that prediction is increased.

The results computed by applying rules a to i and optionally additional rules can be stored in a list or a database in computer readable format and/or can be printed or displayed via an appropriate user interface such as a monitor. If more than one prediction for an unknown amino acid sequence fits the results obtained with the rules described above, then the predicted sequence can be ranked with the best fitting sequence for the unknown peptide on top as shown at step 148 in Figure 8b. If the known peptide P1 has more than one related, unknown peptide P2, than the approach described can be repeated for all unknown peptides P2 as shown in Figure 8c. The approach described above can be extended to any known peptide signal P1 in a list of peptides as exemplified in Figure 8d.

**Differential Network Module**

According to the present invention the interaction of the Differential Network Module with the fundamental CAN Module allows to identify peptides which independently from each other distinguish between a sample A and a sample B. A status can be young, old, healthy, diseased, sweet taste, bitter taste, transfected, non-transfected, yellow, green, male female, pregnant, non pregnant, smoker, non smoker or any other criterion defining a group or a subgroup of samples or organisms from which samples are derived. Optionally the Differential Network Module is linked with various databases, containing data such as the status of the samples, as well as with the other modules of the present invention and especially the basic CAN Module as shown in Figure 1. The Differential Network Module instructs the CAN Module to define subgroups of samples defined by distinct criteria, such as the status of the samples, and further to calculate separately the peptide-to-peptide relations for any status or any combination of more than one status. First, those peptide pairs that suffice a threshold of correlation in a group of samples representing the status A, second, those peptide pairs that suffice a threshold of correlation in a group of samples representing the status B, and third, relations can be defined on the basis of differences between the correlations of the compared status A and status B. If a user is interested in peptide-to-peptide relations, that are most different in samples from two different statuses A and B, then he will search for peptides where the correlation coefficients of the respective peptide-to-peptide relations is different, and where $\Delta r = |r_{Status\ A} - r_{Status\ B}|$ is preferably greater than 85% of all peptide-to-peptide $\Delta r$.

Figure 15 shows a flow chart schematizing the above described procedural steps of an interaction of the Differential Network Module with the CAN Module according to the present invention allowing for the identification of peptides suitable to be used as marker panels using the peptide topology of a plurality of samples taken from at least two different experimental groups representing a status A and a status B. At step 170 a respective mass spectrum for each sample of said plurality of samples is provided, wherein signal intensity peaks correspond to potential peptides. Then the measures of correlation between the signal intensities of said potential peptides are computed at step 172 for each plurality of samples within each experimental group separately. Finally pairs of potential peptides are selected at step 174, which exhibit a difference in the degree of correlation between the different experimental groups above a certain threshold, thereby providing peptides which are suitable to be used as marker panels for diagnostic purposes to distinguish between status A and status B.

The results of the Differential Network Module allow statements about the different relations of peptides within samples of status A compared to status B as follows: If the difference of correlation coefficients of peptide I with peptide II in status A minus the corresponding correlation coefficient in status B is greater than a given threshold, signal coordinates of the peptide pairs, their mutual distance within the observed status A and status B or the degree of difference or combinations of the latter information are stored in a database or list. The Differential Network Module optionally provides the same visualization methods as the other modules, that means peptide coordinates and their relations can be represented as bullets connected by lines, respectively, as shown in Figure 6, and identified peptides can be reviewed via convenient connections to databases or lists containing supportive data resources.

Another use of this aspect of the present invention is the comparison of the molecular masses of peptides present in at least three samples, representing one or at least two different states, status A with corresponding samples and status B with corresponding samples. For example samples from individuals with a certain disease versus samples from individuals without that certain disease, samples from pregnant versus samples from non-pregnant individuals, samples from bacteria transformed with an expression vector versus samples from non-transformed bacteria, samples from yoghurt with a strong acidic taste versus samples from yoghurt with a mild acidic taste, etc. might be compared by computing the correlation measures of peptides present in these samples. The comparison of measurement parameters of a peptide within two samples corresponding to two different states A and B may also indicate that the peptide is present only in samples of state A but not in samples of state B. Also in this case the measurement parameters of this peptide in status A and status B possibly can be related by a measure of correlation. If at least two different peptides, e.g. peptide I and peptide II, are identified, the measurement values of the parameters for peptide I and peptide II can be combined. Using measurement values of at least three samples being representative of status A and three samples being representative of status B, a mathematical function can be computed. This mathematical function describes the correlation-network of peptide I and peptide II. It is possible to include more than two different peptides in one correlation-network, e.g. to include more than two different peptides in one mathematical function describing a correlation-network. The resulting mathematical function describes which combinations of measures of correlation of at least two peptides (peptide I and peptide II) allow to distinguish status A from status B.

Furthermore, another use of this aspect of the present invention comprises the automated identification of sets of peptides that allow a prediction of a status of a sample by a regression model. The invention detects relations between at least two peptides, where the relations are representative for a given status A. In a next step, a linear or non-linear regression model is set up that uses input parameters of the found peptides, such as their respective MALDI signal intensities, and that fits these input parameters to an end point parameter, such as the diagnosis (yes/no = 1/0), or that fits to another parameter of a peptide of this derived set.

In order to check whether a sample of unknown status is a member of the status A, the input parameters of these peptides from that sample are applied to the derived model. If the output value obtained from that sample deviates in the range as other samples from status A from an expected value obtained by means of the determined function, than this unknown sample can be considered to be from status A. Otherwise, the sample most likely has another status.

**Marker Panel Network Module**

According to the present invention the interaction of the Marker Panel Network Module with the fundamental CAN Module allows to identify peptides which independently from each other distinguish between a sample representing status A and a sample representing status B. For example a disease is caused by different factors such as inflammation and an increased heart beat rate. Each of these disease factors might result in altered concentrations of distinct peptides in for example blood plasma of the patient. If a panel of for example two peptide markers is used for diagnosis of the disease it would be useful if one of the peptide markers indicates inflammation and the other peptide marker indicates increased heart beat rate. The combination of these two markers would increase the specificity and sensitivity of the marker panel to detect the disease caused by a combination of inflammation and increased heart beat rate. The Marker Panel Network Module selects those potential peptides which are related to the disease but are most likely associated to different disease factors (in this hypothetical case inflammation and increased heart beat rate), since these peptide coordinates have a low measure of correlation to each other but both have a high correlation to the disease. Thus the specificity and sensitivity of a diagnostic test can be improved by combining these complementary peptide coordinates to a marker panel.

For example a disease 1 (status A) which is associated with inflammation has to be distinguished from another disease 2 (status B) which is not associated with inflammation.

There are, for example, four peptides found, which distinguish disease 1 form disease 2. Peptide 1 and peptide 2 are fragments from the same protein, for example from TNF-alpha, peptide 3 is, for example, a fragment of IL-6 and peptide 4 is a fragment of an unknown protein. All of these four peptides differentiate between disease 1 and disease 2 by a measure of correlation, but peptide 1 and 2 correlate to each other, which is not surprising, as they originate from the same molecule (TNF-alpha). Additionally peptide 1 and peptide 3 correlate to each other, which is also not surprising, as TNF-alpha and IL-6 have similar pro-inflammatory functions. Consequently there are two groups of peptides, peptides 1, 2 and 3 belong to one group and peptide 4 represents the second group. To obtain a diagnostic test, with improved specificity and/or sensitivity combination of the detection of peptide 1 and 2 or 1 and 3 or 2 and 3 would not increase the specificity and/or sensitivity as much as combination of peptide 1 and 4 or 2 and 4 or 3 and 4 would do. This method allows to identify panels of peptides with additive or synergistic value (diagnostic, therapeutic, functional, etc.).

Figure 16 shows a flow chart schematizing the procedural steps of an interaction of the Marker Panel Network Module with the CAN Module according to the present invention allowing for the identification of peptides suitable to be used as marker panels using the peptide topology of a plurality of samples taken from at least two different experimental groups representing a status A and a status B. At step 180 a respective mass spectrum for each sample of said plurality of samples is provided, wherein signal intensity peaks correspond to potential peptides. Then potential peptides correlating with a parameter being representative of status A or status B are selected at step 182. Thereafter the measures of correlation between the signal intensities of said selected potential peptides for each plurality of samples are computed at step 184 and finally pairs of potential peptides which exhibit no correlation of their respective signal intensities above a certain threshold are selected at step 186, thereby providing potential peptides which are suitable to be used as complementing peptides in a marker panel for diagnostic purposes to distinguish between status A and status B.

In other words, the Marker Panel Network Module selects potential peptides which correlate with a parameter being representative for status A or status B. The Marker Panel Network Module then queries the Correlation Associated Network (CAN) Module for those pairs of selected peptide coordinates, which have a very low measure of correlation of their respective signal intensities to each other. The result are pairs of peptides which are related to the status A or B but not directly related to each other and can be combined for a marker panel to distinguish between status A and B. It is possible to combine two or more peptides to a marker panel.

The Differential Network Module described in the previous section discovers combinations of peptides, whose ratio of concentration indicate a certain state and deviations from that ratio indicate a different state. It is mandatory to measure the signal intensity (e.g. concentration) of both/any peptide to calculate said ratio. The relations between two peptides may be present only in state A, whereas the relations between the same two peptides may be different or absent in state B.

In contrast, any peptide found by the Marker Panel Network Module described in the present section could serve as a diagnostic marker alone, but a combination of both markers improves the sensitivity/specificity etc. of the diagnostic test. The members of a marker panel ideally should not correlate with each other in any of both states. If the members of a marker panel correlate with each other their combination most likely would not improve the sensitivity/specifity of the diagnosis.

**Surrogate Network Module**

The Surrogate Network Module relates to the identification of peptides (so called surrogate peptides) that can replace or complement established diagnostic or therapeutic peptides or peptides of other use. If for instance it is discovered that peptides correlate with known bioactive therapeutic peptides, these peptides might serve as surrogates for therapeutic measures or even may exhibit a higher/larger potency, efficacy, specificity, selectivity and/or less undesirable side effects. These kind of peptides can be found using the Surrogate Network Module in combination with the CAN Module according to the present invention by applying the steps shown in Figure 17. Initially a respective mass spectrum for each sample analyzed is provided, wherein signal intensity peaks correspond to potential peptides (step 190). Thereafter at step 192 measures of correlation between the signal intensity of a known peptide and the signal intensities of potential peptides are computed and finally those potential peptides are selected at step 194, which exhibit a degree of correlation with the known peptide above a certain threshold, thereby providing potential peptides suitable to replace or complement the known peptide. Two exemplary applications of the Surrogate Network Module are given below

For example a plasma sample is known to contain the peptide insulin and a potentially unknown peptide X within the same plasma sample correlates with the peptide insulin. In this case peptide X might have the same function as insulin, as its correlation measure indicates that it is related to insulin. The reason for this could be that peptide X is a derivative of insulin, for example a glycosylated form of insulin, or another peptide which is completely different from the amino acid sequence of insulin but which is involved in the same functional or metabolic cycles as insulin. In both cases peptide X could serve as an alternative to the use of insulin for example in treating diabetes. It might also turn out that peptide X in combination with insulin improves the therapeutic effect of insulin by itself.

In a further example a tissue sample of a prostate cancer patient contains the prostate-specific antigen (PSA) peptide, which is a known marker for prostate cancer. Another potentially unknown peptide Y is related by a correlation measure to the PSA peptide and consequently peptide Y might have the same diagnostic value as a biomarker for prostate cancer as the PSA peptide or the measurement of peptide Y might complement the prostate cancer diagnosis by PSA measurements.

**Interaction of Modules**

Though any of the modules described above can be used independently, any combination of these modules can be used and potentially can synergistically improve the result of one or more of the modules.

For example results of the Surrogate Network Module can be analyzed by the Sequence Network Module. In case the Surrogate Network Module yields peptide signals, which are not yet sequenced, a prediction of the sequence can give early hints for biological interpretation, thus accelerating validation processes of for example therapeutic or diagnostic peptides. However, a subsequent identification of these peptides by sequencing is recommended.

Results of the Differential Network Module can be analyzed with the Surrogate Network Module. If the Differential Network Module yields for example potential biomarkers, it is highly desirable to identify possible surrogate markers that show a similar behavior and therefore are of interest as well. Therefore a combination of the Surrogate Network Module with the Differential Network Module accelerates the discovery of novel therapeutic, diagnostic or other peptides and is highly synergistic.

Furthermore, results of the Differential Network Module can be analyzed with the Sequence Network Module. If the Differential Network Module yields peptide signals, which have not been sequenced yet, the prediction of the sequences of the unknown peptides can give early hints for biological interpretation, thus accelerating validation processes of potential therapeutic, diagnostic or other peptides. However, the later identification of these peptides by sequencing is recommended.

**Examples**

The following examples are intended to describe how the methods according to the present invention can be applied to real data. For the sake of a clarity only a limited number of exemplary measurement parameters are calculated and presented in the figures. However, as is readily observable by the person skilled in the art, the advantages of the methods according to the present invention become even more obvious when applied to large sets of data. On present computer systems commonly measures of correlation for data sets consisting of up to 6.000 potential peptides are commonly calculated and without undue effort data sets of up to 100.000 potential peptides can be analyzed by means of the methods according to the present invention.

**Example 1**

The basic CAN Module calculates to what extent a potential peptide for each individual potential peptide measured in a sample correlates to every other potential peptide in that sample. The CAN Module determines a network of correlations among the peptides which in case of some degree of correlation supposedly are related to each other for certain reasons such as a common precursor as the origin of the peptides or the same biological function of the different precursors of the correlating peptides.

In the present example the set of data, i.e. the data matrix, consists of 444.000 values comprising measurement parameters, in this case signal intensities, of 74 independent samples, each sample resulting in 6.000 peptide coordinates. The tables shown in Figures 18a, 18b list the corresponding raw data for four out of a total of 6.000 peptide coordinates. Four different methods to determine measures of correlation, namely, Spearman's rank order correlation, Pearson's product moment correlation, Kendall's rank correlation tau, and Minimal Spanning Tree (MST) diameter, are calculated for the three exemplified pairs of peptide coordinates comparing the peptide coordinate Fraction 54; m/z 2743.0 with three other peptide coordinates (Fraction 54; m/z 1371.5, Fraction 56; m/z 2927.2 and Fraction 20; m/z 1114.3) (see table shown in Figure 19). The definition of the threshold is an important step in the creation of Correlation Associated Networks and should be performed carefully as has been described in detail further above. In the data matrix 6.000 x 6.000 x 0.5 = 1.8 x $10^7$ possible peptide-to-peptide pairs can be combined, and each of these pairs exhibits a certain correlation coefficient r. Figure 20 shows a plot of the probability of a peptide pair P(r) to have a certain correlation coefficient r. A value of r of zero or close to zero describes relations which are completely random, whereas values of r close to 1 or -1 describes relations which respectively correlate or anti-correlate very strongly. The more peptide pairs are tested for a correlation by means of measures of correlations, such as Spearman's rank order correlation coefficient, the more peptide pairs by chance correlate to some extent with each other. This means that a correlation coefficient regarded as informative and real has to pass a higher threshold value. It is recommended to perform a plot like in Figure 20 to estimate the information content of a given correlation coefficient. One curve (black circles) in this figure plots the likelihood (y-axis) for a given correlation coefficient (x-axis) for all peptide-to-peptide pairs from the said data matrix comprising 6.000 peptide coordinates. The other curve in Figure 20 marked by the white squares describes the likelihood of correlations occurring by chance.

Most probable true positive relations can be found where the area under the curve is small, while the maxima of the curves represent the correlations coefficients which are most likely false positive relations. In case Spearman's rank order correlation coefficient is chosen as measure of correlation and $|t_{threshold}| \geq 0.8$ is chosen as threshold for a definition of a peptide-to-peptide relation, the peptide coordinate Fraction 20; m/z 1114.3 is not related to the peptide coordinate Fraction 54; m/z 2743.0 (see table shown in Figure 19). In contrast, the peptide coordinates Fraction 54; m/z 1371.5, and F 56; m/z 2937.3 are highly related to the peptide with coordinates Fraction 54; m/z 2743.0 (see table shown in Figure 19). These peptide relations could pass through a filter and be stored in a local Valentina Database file.

**Example 2**

Assuming that one is interested in finding surrogate markers for Chromogranin A in hypothetical prostate cancer patients and that some of the 74 samples described above originated from healthy male persons and some samples originated from prostate cancer patients. Under the further assumption that a peptide originating from Chromogranin A, amino acids 97-131, had been identified, the Surrogate Network Module would now query the basic CAN Module for peptide coordinates that are highly related by a correlation measure with the hub-peptide Chromogranin A, 97-131. This could be done for example by defining that the Spearman's rank order correlation coefficient of peptide-to-peptide relations |r| has to comply with the relation $|r| \geq 0.67$. Then the Surrogate Network Module would instruct the CAN Module to query the Valentina Database, and report that there are about 14 peptide coordinates matching this condition. These peptide coordinates are searched in databases for any known peptide fitting to these coordinates. In this way it would be found that three peptides known from the database and present in the list of 14 peptides belong to the Chromogranin/Secretogranin family as illustrated in the table shown in Figure 21. The Surrogate Network Module would project the peptide coordinates of the related peptides and the hub peptide as bullets on a two-dimensional or three-dimensional plane, such as a peptide map fingerprint of a serum sample as shown in Figure 6. Relations between peptide coordinates are depicted as lines between the bullets. Lines can be selected by a computer pointing device such as a mouse and a small information window will pop up containing information about the kind of correlation measure and the value for the measure of correlation of the connected peptide coordinates is shown. The bullets can also be selected by a computer mouse click, and an information window will provide information about the peptide coordinate, and if this peptide coordinate has already been identified, then the name of the precursor peptide, the start- and stop position of the identified peptide will be provided by retrieval of a "Sequence Information Database" as exemplified at 56 in Figure 1. Also links to other databases such as Swiss-Prot and GeneCard are provided and/or other databases such as the Patent database of the USPTO can be queried for the search terms "name of the peptide" and "diagnostic". An internet browser window could display the results from the US-Patent Database. The visualization of peptide-to-peptide relations and convenient connection and access to internet and intranet resources by the Surrogate Network Module significantly increases the speed of data acquisition that is needed for an evaluation of the results. The example of Chromogranin A indicates that other peptides originating from members of the secretogranin-chromogranin family are automatically found by the CAN Module. These peptides are listed in the table shown in Figure 21 and can serve as a diagnostic marker for the prediction of the therapeutic success in the hypothetic prostate cancer patients.

**Example 3**

In an exemplary hypothetic serum dataset 48 samples are derived from patients before prostatectomy and 26 samples from patients after prostatectomy. For the Differential Network Module a correlation measure, e.g. the Spearman's rank order correlation coefficient r, between the peptides is calculated for samples from patients before prostatectomy and for samples from patients after prostatectomy separately. The correlation coefficient of Chromogranin A 97-131 and Secretogranin I 88-132 for all 74 samples is r = 0.67, for those patients before prostatectomy is r = 0.23 and for those after prostatectomy is r = 0.97 (see Figure 22). This shows that the peptides Chromogranin A 97-131 and Secretogranin I 88-132 obviously are much less related after prostatectomy than before. This also explains the loss of correlation for all patients. For the given example this means that Secretogranin I 88-132 is a potential surrogate marker for Chromogranin A 97-131 only before prostatectomy, thereafter the relation is broken. This would have a significant impact on the design of a clinical evaluation of Secretogranin I 88-132 as a surrogate marker for Chromogranin A, and could save enormous costs. Furthermore, the ratio of concentration of Chromogranin A 97-131 and Secretogranin I 88-132 is a diagnostic parameter itself. If the ratio deviates from 10/1 significantly, then a prostatectomy has been accomplished. Figure 22 exemplifies the use of the ratio of the signal intensities of Chromogranin A 97-131 and Secretogranin I 88-132 as a diagnostic parameter: The ratio of 10/1 is present in all samples from patients before prostatectomy. In samples after prostatectomy this ratio is not present, i.e. the Secretogranin I/Chromogranin A relation is "broken".

**Example 4**

This section exemplifies the identification of representative peptides, also called "landmark peptides" and also refers to the given data matrix of 74 observations of 6.000 peptide coordinates already discussed in a previous example.

Two peptide coordinates are considered as related if the Spearman's rank order correlation of their signal intensities is above |r| > 0.8. The number of relations k a respective peptide has with different peptide coordinate is shown in the second row of the table shown in Figure 18a. From all peptide coordinates, Fraction 54; m/z 2743.0 has the most relations, i.e. k = 20. Therefore, this peptide coordinate would be No. 1 in a prioritization list. Then, the signal variance of Fraction 54; m/z 2743.0 is removed from the signal intensities of the 20 related peptide coordinates, wherein Formulas 1, 2 and 3 are applied. Then the data of Fraction 54; m/z 2743.0 is removed from the data matrix. The tables shown in Figures 23a and 23b show the values given in the tables shown in Figures 18a and 18b after the variance of Fraction 54; m/z 2743.0 on the related peptide coordinates has been removed. This process is iterated to determine the next peptide coordinate as a candidate for the sequencing prioritization list, until the number of peptides to be sequenced has been reached.

**Example 5**

In this example, the signal intensities of four fictive peptide coordinates of 74 samples, their respective mass-to-charge ratio and their fraction numbers are given (see table shown in Figure 18a). The calculation is performed using five fictive peptide coordinates using as the $5^{th}$ peptide coordinate F 53; m/z 2823.0. One of the five signal coordinates, the fictive peptide HP 25-48 in Fraction 54; m/z 2743.029, has already been identified, and guided by the rules for the Sequence Network Module, the identities of the four remaining, unknown peptides will be proposed.

The measure of correlation of the four unknown peptide coordinates with HP 25-48 has been calculated in the CAN Module by means of Spearman's rank order correlation coefficient:

r (HP 25-48 and F 20; m/z 1114.3) = +0.00
r (HP 25-48 and F 54; m/z 1371.5) = +0.92
r (HP 25-48 and F 56; m/z 2927.3) = +0.84
r (HP 25-48 and F 53; m/z 2823.0) = +0.87

As can be seen in Figures 24a to 24c and by the low correlation coefficient and MST diameter shown below, respectively, F 20; m/z 1114.3 is not related to HP 25-48, thus will not be hypothesized to be related to the HP precursor protein. The generation of proposals for this peptide coordinate stop at this point.

In the same manner, the MST diameter was calculated as a measure of correlation:

MST diameter (HP 25-48 and F 20; m/z 1114.3) = 29 (see Figure 25a)
MST diameter (HP 25-48 and F 54; m/z 1371.5) = 50 (see Figure 25b)
MST diameter (HP 25-48 and F 56; m/z 2927.3) = 38
MST diameter (HP 25-48 and F 53; m/z 2823.0) = 40 (see Figure 25c)

In contrast, peptide coordinates F 54; m/z 1371.5, F 53; m/z 2823.0 and F 56; m/z 2927.3 are highly related to HP 25-48 (see Figures 24b, 24c and Figures 25b, 25c). A proposal using the sequence of the precursor of the protein HP will be assigned to these peptide coordinates and the rules according to the Sequence Network Module of the present invention will be applied for sequence prediction.

Rule a determines whether the related peptide coordinate is a n-charged ion of HP 25-48. The calculation of $Mass_{Deviation}$ is exemplified with n = 1, 2, 3 or 4 and the mass-to charge ratios of F 54; m/z 1371.5 and F 56; m/z 2927.26 given in the table shown in Figure 26, using Formula 4. It is highly probable that F 56; m/z 1371.5 is a double charged ion of HP 25-48, as in the case of n = 2 $Mass_{Deviation} < Mass_{Threshold} = 0.5$, therefore it is proposed as HP 25-48$^{2+}$, i.e. the double charged ion of HP 25-48.

Rules b to i will now be applied to F 53; m/z 2823.0 and F 56; m/z 2927.3. Rule b assumes that the relation of the hub peptide P1 in fraction F 54; m/z 2743.029 of known identity with the unknown peptide P2 (peptide coordinate F 53; m/z 2823.0) is derived from a post-translational modification. In this case, the mass difference of the hub peptide P1 and the unknown peptide P2 $M_{DIFF} = |M_{P1}-M_{P2}| = 79.971$ might be caused by phosphorylation or sulphation (see table shown in Figure 9). Alignment of HP 25-48 with recognition sequence motifs of protein kinases, that are enzymes responsible for phosphorylation of proteins and peptides, identifies the sequence HP 35-37 to be "TYD", which is as a potential target of a hypothetic protein kinase HPKC. Therefore a proposal for F 53; m/z 2823.0 is HP 25-48 with one phosphorylation at the tyrosine residue on position 36 of the peptide HP 25-48.

As stated before, if the unknown peptide and the known hub peptide are related, it is hypothesized that the unknown peptide is derived from the same precursor protein and thus has the same precursor sequence as the known hub peptide. An algorithm systematically defines putative start and end positions, I an E, in the precursor sequence of the hub peptide P1 proposing a putative sequence fragment potentially derived from the precursor sequence, that could be the sequence of the unknown peptide P2 (see Figure 8b). Of course, the sum of masses of the amino acid residues, plus their amino- and carboxy-terminal ends and plus potential posttranslational modifications must match the measured m/z ratio $M_{found}$ of the unknown peptide P2 within a given threshold T. The Mass of the putative sequence is calculated by summing up the masses of the amino acid residues comprising the putative sequence for P2 plus the mass of a hydrogen and a hydroxyl group. Exemplary values of masses applying Formula 5 are given in the tables shown in Figures 11 and 12.

With HP 25-48 as the hub peptide and P2 having the peptide coordinate Fraction 56; m/z 2927.3 the Sequence Network Module searches for possible sets of start and end positions in the protein precursor sequence of HP as defined in Figure 27, that have a deviation of mass lower than the threshold $T_{MASS} = 0.5$.

One possible combination is a start position at amino acid No. 25 and an end position at amino acid No. 50 of HP resulting in the potential peptide HP 25-50:

$n_D = 2$, $n_A = 4$, $n_H = 2$, $n_K=2$, $n_S = 1$, $n_E = 3$, $n_V = 2$, $n_R = 1$, $n_F = 1$, $n_L = 3$, $n_G = 1$, $n_I =1$, $n_N = 1$,
$n_T = 1$, $n_Y = 1$ in Formula 5 results in $M_{CALC} = 2927.337$

This proposal is added to the list of proposals for P2.

The Sequence Network Module will now address the evaluation of the proposal HP 25-50 for P2 by applying rules c to i. In rule d, the chromatographic fraction of the proposed sequence $F_{CALC}$ is estimated and compared with the found peptide coordinate of P2 ($F_{FOUND}$). If $F_{CALC}$ deviates from $F_{FOUND}$ by less than the threshold for fractionation ($T_{FRACTION}$) then the proposal is awarded with 2 model fitness points. If Formula 6, "Estimation of fraction number based on proposed sequence", is applied to HP 25-50, the calculated Fraction results in $F_{CALC} = 56$.

As P2 HP 25-50 is found in fraction 56, the number of model fitness points for this proposal is increased by two points. Formula 6 was generated empirically from a mathematical model using data originating from liquor cerebrospinalis samples separated using a specific HPLC-column (as described in the patent application WO 03/048775 A2) using a specific software. Of course, for different types of samples and different separation methods other empirically determined models can be calculated in the same way.

Rule e rewards those proposals for P2, whose start-positions match the start positions of the hub peptide P1.

In the case of HP 25-48 as the P1 hub peptide and HP 25-50 as the proposal for the related peptide P2, the proposal HP 25-50 will be rewarded with 3 model fitness points.

Rule f rewards those proposals for P2, whose end-positions equal the end-position of the hub peptide P1. This is not the case with HP 25-50 as a proposal, therefore this rule does not increase the model fitness points of this proposal for P2.

Rule g will increase the model fitness points of the proposal HP 25-50 by three points as the start position 25 is preceded by the amino acid sequence "R-R" (written in 1-letter amino acid code). The sequence "R-R" is a recognition site of prohormone convertases, which commonly cleave after the second "R". In addition, rule g will increase the model fitness points for this proposal by another 3 points, as the "D-A" sequence is one of the preferred starts for peptide sequences present in liquor cerebrospinalis. Further sites of frequent proteolytic cleavage sites at start positions awarded by rule f are well known in the art.

Rule g assumes that the unknown peptide P2 is a product of N- or C-terminal proteolysis of the known hub peptide P1 or vice versa. The mass difference of P1 and P2 $M_{DIFF} = |M_{P1}-M_{P2}|$ is determined and aligned with the masses of the amino acids preceding and following the start- and end positions of P2 in the precursor sequence HP. In the example of HP 25-48 as P1 and HP 28-50 as P2 the mass difference is $M_{DIFF}$ = 184.2 and can be explained by the amino acids "I-A" ($M_I + M_A$ = 184.2) which are following the end position of P1. Therefore P2 fits the model and the model fitness points for this proposal for P2 are increased by 3 points.

Obviously, rules c to i can be examined in any order, and rules can be left out for biological considerations, but still any combinations and any omissions of these rules are within the scope of this invention.

The process described above can be repeated for all unknown peptides coordinates P2, which are related with HP 25-48.

**Example 6**

This example demonstrates the advantages associated with the methods according to the present invention by combining Correlation-Associated Peptide Networks with recognition of probable cleavage sites for peptidases and proteases in cerebrospinal fluid, resulting in a model able to predict the sequence of unknown peptides with high accuracy. On the basis of this approach, for instance the identification of peptide coordinates can be prioritized, and a rapid overview of the peptide content of a novel sample source can be obtained.

Cerebrospinal fluid (CSF) is in close contact with many parts of the brain. CSF aids to maintain a stable chemical environment for the central nervous system and is a route to remove products of brain metabolism. CSF distributes a multitude of biologically active substances within the central nervous system. It is acceptable to assume that CSF mirrors the physiological and pathophysiological status of the brain and, therefore, peptides from CSF represent a source of potential diagnostic and therapeutic target molecules.

Here the correlational behavior of peptides from CSF is analyzed, derived from the same protein precursor in more detail and correlational dependencies for the prediction of putative sequences of unknown peptides are exploited. If one assumes that a known peptide and an unknown peptide signal of a peptide-to-peptide pair might have a common protein precursor, the known protein precursor sequence is analyzed for proteolytic cleavage sites which could explain the generation of a signal with a mass corresponding to that of the unknown peptide. It will be shown that the combination of statistical analysis (CAN) and recognition of possible cleavage sites for peptidases and proteases in CSF results in a model with high predictive power for correct assignment of an unknown peptide signal to a protein precursor or even a sequence, thus reducing the number of peptides to be sequenced.

After approval by the local ethics committees, written informed consent was obtained from patients involved in this study. Human CSF was collected by lumbar puncture from neurological patients without cognitive impairment (n=39) and from patients suffering from dementia such as vascular dementia, Lewy-body dementia, frontotemporal dementia or Parkinson's disease (n=27). All CSF samples were prepared using mild conditions minimizing risk of sample alteration: The fluid was collected without aspiration and avoiding blood contamination. Samples were centrifuged for 10 min at 2000 g and the supernatant was stored at -80 ˚C until analysis.

Peptides were separated using reversed-phase C18 chromatography. 300 to 1500 μL CSF was diluted 1:3.75 with water and the pH was adjusted to 2-3. Samples were loaded onto RP silica columns (250 x 4 mm column, Vydac, Hesperia, CA, USA; HP-ChemStation 1100 Agilent Technologies, Palo Alto, CA, USA). Retained peptides were eluted using an acetonitrile gradient (4 to 80 %) in 0.05 % trifluoroacetic acid, collected into 96 fractions and lyophilized. Elution was monitored by UV detection. The retention time of major peptide peaks from repeatedly loaded extracts was used to confirm the reproducibility of the method.

After lyophilization, each HPLC fraction was resuspended in matrix solution (mixture of α-cyano-4-hydroxycinnamic acid and L-fucose (co-matrix) in 0.1 % acetonitrile/ trifluoroacetic acid (1:1 v/v) and applied to a matrix-assisted laser-desorption / ionization (MALDI) target, followed by ambient temperature air drying. Sample ionization was performed by application of repeated single laser shots over a representative area of the sample spot. The accelerated ions were analyzed in a time-of-flight (ToF) mass spectrometer (Voyager-DE STR, Applied Biosystems, Framingham, MA, USA) in linear mode.

Peptides of interest were identified by mass-spectrometric sequencing using nanoESI-qTOF-MS/MS (QSTAR pulsar, Sciex, Toronto, Canada) with subsequent protein database searching. The resulting peptide fragment spectra were acquired in product ion scan mode (spray voltage 950 V, collision energy 20-40 eV). Up to 200 scans per sample were accumulated. Data processing previous to database searching included charge-state deconvolution (Bayesian reconstruct tool of the BioAnalyst program package, Sciex, Concord, Canada) and de-isotoping (customized Analyst QS macro; Sciex, Concord, Canada). The resulting spectra were saved in MASCOT (Matrix Science, London, UK) generic file format and submitted to the MASCOT search engine. Cascading searches including several posttranslational modifications in Swiss-Prot (Version 39 or higher, www.expasy.ch) and MSDB (Version 030212 or higher, EBI, Cambridge, UK) were performed by MASCOT DAEMON client (Version 1.9, Matrix Science) that allows, beside sequence determination, identification of modified amino acids as well as determination of their position within the peptide's sequence.

All mass spectra with the same fraction number of chromatography were baseline-corrected averaged, and all 96 averaged mass spectra fractions were visualized in a "2D gel-like" format (peptide display), yielding an averaged peptide display (see Figure 28). Each peak (mass spectrometric signal) is depicted as a bar with its grey-scale intensity corresponding to the signal intensity of the corresponding MALDI-peak which corresponds to the relative amount of the peptide measured. The x-, y- and z-axis represent mass to charge ratios (m/z), chromatographic fraction and mass spectrometric signal intensity, respectively. Masses range from 1,000 to 15,000 m/z ratios (x-axis). The data matrix of an individual peptide display consists of 16 million data points, of which, for a given signal to noise ratio, signal coordinates are extracted. For all samples, an identical set of signal coordinates is thus present that is utilized for statistical analysis.

Data pre-processing of the acquired MALDI-ToF-mass spectra was performed applying baseline correction (RAZOR Library 4.0, Spectrum Square Associates, Ithaca, NY, USA) in combination with normalization of the mass spectra to a constant integral value. For the benefit of simplicity and uniformity, all m/z-ratios were stated as average masses of the uncharged analyte. Wherever necessary, data was made available for the model by transformation of m/z-ratio data into this format.

For the analysis of all peptide-to-peptide relations, calculations of correlation were performed with the signal intensities (i.e. relative peptide quantity) of all present (unknown) peptide coordinate data sets to any known peptide coordinate in peptide displays of patient samples:

> Any pair-wise relation of two peptides was rated by Spearman's rank order correlation of their respective signal intensities in all samples. Peptide pairs in combination with m/z ratios, chromatographic fraction and Spearman' rank order coefficients of correlation were stored in a local Peptide-to-Peptide database.

In an automated approach, all peptide coordinates were individually queried in a peptide sequence database. The following rules were applied

- For each peptide coordinate:
    - ○ Search for entry in peptide sequence database to match mass-to-charge ratio and chromatographic fraction within given thresholds.
    - ○ If peptide coordinate found in peptide sequence database:
        - ■ Retrieve information of peptide coordinate (sequence, average mass, name, precursor protein, start-stop position on precursor protein, precursor protein sequence).
        - ■ Store information in an individual list of identifications.

members of this list of identifications are now utilized as "hub peptides" for subsequent correlational analysis.

- For each peptide coordinate (= hub peptide) with entry in peptide sequence database:
    - ○ Create individual Correlation Associated Network by retrieving Peptide Coordinates with correlation coefficients above a given threshold in Peptide-to-Peptide database, thus becoming member of the CAN of the hub peptide.
    - ○ For each member of a CAN:
        - ■ If peptide coordinate of CAN member is not found in Peptide Sequence database:
            - • Analyze protein precursor sequence of hub peptide for putative sequences that approximately match the mass-to-charge ratio of the CAN member peptide coordinate: create a list of putative sequences that match the mass-to-charge ratio of the CAN member peptide coordinate within the range of mass accuracy (here: less than 500 ppm) by permuting the start- and end positions on the protein precursor sequence and simultaneously summation of masses of the amino acid residues of the putative sequences.

- For each putative sequence in generated list:

    ○ Evaluate putative Sequence (rules see below) with bonus points.

- Rank putative sequences according to the number of bonus points.

○ For each peptide coordinate

  ■ Present Top 3 putative sequences.

The determination of the bonus points is explained further below.
a) If the amino acid residue before/after amino-terminal/carboxy-terminal cleavage site of the putative peptide sequence on the precursor sequence corresponds to the following amino acid residues (one letter code), the proposal is awarded with the respective bonus points (bpt):

| | | | |
|---|---|---|---|
| 1. | before amino-terminal cleavage site: | M : 2 bpt; | R: 5 bpt |
| 2. | after amino-terminal cleavage site (N+1): | D : 3 bpt; | M: 2 bpt |
| 3. | before carboxy-terminal cleavage site (C-1): | no rules | |
| 4. | after carboxy-terminal cleavage site (C+1): | K : 3 bpt; | R: 4 bpt |

b) If the pairs of amino acids before/after amino-terminal/carboxy-terminal cleavage site of the putative peptide sequence on the precursor sequence correspond to the following amino acid pairs, the proposal is awarded with the respective bonus points (bpt):

| | | | |
|---|---|---|---|
| 1. | before amino-terminal cleavage site: | KR: 18 bpt; | RR: 22 bpt; |
| 2. | after amino-terminal cleavage site: | DA: 43 bpt; | GR: 11 bpt; |
| 3. | before carboxy-terminal cleavage site: | GA: 20 bpt; VN: 16 bpt | QK: 20 bpt; |
| 4. | after carboxy-terminal cleavage site: | KR: 22 bpt | |

c) If the putative sequence has the same start position as the known hub peptide, the proposal of this sequence is awarded with 69 bonus points. If the putative sequence has the same end position as the known hub peptide, the proposal of this sequence was awarded with 63 bonus points.The determination of the bonus points is explained further below.

The peptidome of 66 independent CSF samples was analyzed using a combination of chromatographic separation (96 fractions) and subsequent mass spectrometry, leading to a database containing 7104 MALDI-ToF preprocessed mass spectra. All mass spectra with the same fraction number were averaged, yielding an averaged peptide display (see Figure 28). 139 different peptides from 31 protein precursors have previously been identified by sequence determination from CSF-preparations carried out in an identical manner to the 66 CSF samples. The peptide coordinates were located on the averaged peptide display. Since abundant peptides can be found in more than one fraction, 224 instead of 139 peptide coordinates were located. The MALDI mass spectrometric signal intensities of the 224 peptides coordinates were determined in each of the 66 samples. Spearman's rank correlational analysis was performed for any given signal-to-signal combination resulting in $224^2/ 2$ correlations of peak signal intensities.

As described in detail further above, a network is defined as a collective of a peptide of interest, the so-called hub peptide, and peptides highly correlating with this peptide, selected from all peptides by exceeding an arbitrarily defined correlational threshold. This concept is exemplified by two networks of VGF and albumin peptides: The network of VGF 26-58 as a hub peptide (see Figure 29), calculated with a threshold correlation of $|r| \geq 0.68$, groups peptides derived from several regions of the VGF precursor protein (see Figure 30). The network with the hub peptide albumin 25-48 (see Figure 31), calculated with a threshold of correlation of $|r| \geq 0.67$, predominantly contains peptides derived from the amino-terminal region of the albumin protein precursor. The observation of networks of VGF 26-58 and albumin 25-48 gave rise to the hypothesis, that a network predominantly contains peptides derived from the same protein precursor, if the threshold of correlation is sufficiently high. This hypothesis was tested as follows: Networks of the 224 signal coordinates were created with increasing thresholds of correlation coefficients (see Figure 32). Members of these networks were predicted as derivatives from the same precursor protein, containing the hub peptide and the predictions matched with the peptides previously identified by ESI-MS/MS. The table of Figure 32 lists the number of correct and false predictions of protein precursors: Predictions with a high threshold of correlation yield few predictions of precursor proteins; predictions with low threshold of correlation coefficients yield several hundred predictions of precursor proteins. The correctness of precursor predictions reaches 100% at $|r| \geq 0.95$ and rapidly decreases with lower thresholds of correlation.

It was assumed that any member of a network is derived from the same protein precursor without experimental determination of the sequence of the unknown signal coordinate. Thus, start- and end positions on that precursor protein sequence were systematically permuted and iterated resulting in putative peptide sequences. Figure 33 gives an overview of the nomenclature for start and end of a peptide on a protein precursor. Any combination of start-/end- positions resulting in a putative sequence that matches the m/z-ratio of the unknown peptide coordinate within a given mass tolerance of less than 500 ppm was considered as a valid proposal. Combinations that do not fulfill this mandatory criterion were rejected. The following investigations showed that some valid proposals were more probable than others, since their start-/end positions are very likely sites of cleavage in human CSF. If a proposal meets one or more of the following criteria, its bonus points are increased.

Peptides are generated by cleavage of peptide bounds by proteolytic enzymes. These proteases recognize specific sites (amino acid sequence motifs), where a cleavage occurs. The probability of cleavage as a function of a particular amino acid and the position of the amino acid regarding the cleavage site was investigated and compared with the occurrence of the respective amino acid at any position in all precursor sequences. The table in Figure 34 shows the results obtained from the data set enrolling the 139 peptides. For example, in 31 % of all peptides, an arginine residue (R) preceeded the amino-terminal cleavage site, but the average content of arginine of the observed precursor sequences was only 6 %. It was concluded, that the probability of cleavage was 5 times increased if an arginine residue was found at the N-1 position. Rules were defined considering the different amino acids at the position of interest: an x-fold increase of probability of cleavage was awarded with x bonus points: For example, the bonus point score of an proposal with arginine at N-1 were increased by 5 points, reflecting the 5-fold increase of probability. The number of rules was limited in order to avoid excessive overfitting of the model: A rule had to be based on at least five peptides and a twofold increase of probability.

Besides investigations of single amino acids, pairs of amino acids were investigated for their influence on an increased probability of cleavage. The probability of cleavage as a function of a pair of amino acids and the position of such a pair with respect to the cleavage site was investigated and compared with the occurrence of the respective pair of amino acids at any position in all precursor sequences (see table in Figure 35). For example, arginine-arginine residues (RR) were found in 18 / 139 = 12.9 % of the peptides before the amino-terminal cleavage site, while RR was found only at 398 / 68516 = 0.58 % at any other position. Thus, the probability of cleavage after RR is 12.9% / 0.58 % = 22-fold increased compared to arbitrary positions. Consequently, a rule considering RR before amino-terminal cleavage increases the bonus point score of a corresponding proposal by 22 bonus points. Thus rules were defined considering pairs of amino acid at the position of interests: an x-fold increase of probability was awarded with x bonus points. Yet, a rule had to be based on at least five peptides and a 10-fold increase of probability, otherwise it was rejected.

Many related peptides were found to have the same start position on a precursor protein as exemplified by VGF 26-58, VGF 26-59, VGF 26-61 and VGF 26-62 (see Figures 29 & 30 #1, #4, #5, #8, #9) or Albumin 25-48, Albumin 25-45 and Albumin 25-50 (see Figure 31 #1, #4/#5, #2, #8/#9). Likewise, many sequences of related peptides stop with the same end position: VGF 25-59 and VGF 26-59 (see Figures 29 & 30 #4/#5, #7) or Albumin 27-50 (see Figure 31 #6/#7) and Albumin 25-50 (see Figure 31 #8/#9). It was found that 14.1 % of all peptides from the same precursor in the data set had the same start position and 12.7 % the same end position. The probability of two peptides having the same start- or end-position by chance was assumed as 1/n, where n is the length of the precursor. With an average precursor length of n=492 in the data set, the increase of probability was 69-fold for two peptides having the same start-position and 63-fold for two peptides having the same end position. Consequently, proposals with the same start-position as the hub peptide were awarded with 69 bonus points and those with the same end-position with 63 bonus points.

The application of the above described rules is exemplified by two proposals (see table in Figure 36), verified by ESI-MS/MS: According to the model, the known peptide VGF 26-58 predicts the unknown peptide coordinate with $m/z_{average}$ 3688.0 as VGF 26-62, because the calculated m/z-ratio of the putative sequence matches the found m/z-ratio (prerequisite condition). Hub peptide and putative sequence have the same start position (+69 bonus points), and the putative sequence terminates before an arginine residue (+4 bonus points) (see Figure 30 #1, #4). Moreover, VGF 26-58 predicts the unknown peptide with $m/z_{average}$ 2419.41 as VGF 350-370. The calculated m/z-ratio of the putative sequence matches the found m/z-ratio (prerequisite condition), a single arginine preceeds (+ 5 bonus points) and a dibasic site RR follows the putative sequence (+22 bonus points) (see Figure 30 #1, #3).

In order to asses the power of prediction of the described model, 139 peptides identified by ESI-MS/MS were split into a group of 70 peptides, that were used for the prediction of a second group of 69 peptides, whose sequence identity was suppressed during the prediction process (see Figure 37): For all 224 signal coordinates, the Correlation Associated Networks were calculated with a coefficient of correlation of |r| = 0.75. Seventy signal coordinates were used to predict the precursor protein, start- and stop position and thus the sequence of signal coordinates, which were members of the network of the corresponding predicting hub peptide. For any signal coordinate, up to three proposals were listed, having the proposal with most bonus points on top. Six models with increasing levels of complexity were compared regarding their power in prediction of precursor protein, start and end positions (see table in Figure 38). Predictions for the second group of 69 peptides were compared with identifications by ESI-MS/MS. The statistics were distinguished for any stored proposal and for the proposal with most bonus points: In all models, the percentages of correctly predicted start and end positions is better for the proposal with most bonus points compared to those of all proposals. The percentage of correct precursor sequence and start-/end-position increased with complexity of the model. Model 5, combining two sets of rules considering both the probability of cleavage near single amino acids and pairs of amino acids, yielded better results than model 2 and model 3, which applied only one set of rules. Best results were achieved by model 6, which incorporated all described rules, in particular those proposals with most bonus points: 85 % of all proposals were correct regarding the precursor sequence, the start- and stop positions, 89 % of all proposals yielded a correct protein precursor prediction, and only 11 % were wrong in both.

The above example thus demonstrates that related peptides are automatically grouped by CANs. The underlying algorithm exploits the fact that concentrations of peptides from different steps of the processing chain can display a conserved ratio, as shown previously for CSF-derived peptides. These conserved ratios of related peptides were reliably found by Spearman's rank order correlation analysis, which is the basis for the definition of CAN relations. The results show that CANs, can be used to automatically group intermediate products of peptide processing. At high thresholds of correlation coefficient, the number of predictions is low, but each having a high degree of accuracy. Decreasing thresholds delivers a growing number of predictions with false ones, finally outbalancing the correct predictions. The present example was based on the assumption/condition that a strict threshold delivers a network whose members are solely derived from the same protein precursor. This was the fundamental basis for the prediction of the sequence of unknown network members. Since the network was based on mass spectrometric data, all peptide signals were characterized by their mass-to-charge ratio. By iterating start and downstream end position on the protein precursor sequence of the previously sequenced hub peptide, putative peptide sequences were generated that matched experimental molecular masses of selected unknown MALDI mass spectrometric peptide signals. The mass accuracy of less than 500 ppm of the MALDI-ToF measurement in linear mode is sufficient to reduce the overwhelming number of theoretical combinations of start- and stop positions of a precursor to a concise selection. The putative sequences were evaluated by models based on the presumed protein precursor's sequence in combination with found proteolytic cleavage patterns in human CSF. In this approach, posttranslational modifications were not considered, considerably reducing the degree of freedom for possible predictions. However posttranslational, as well as other modifications in general can be used to search for correlation in peptide signals.

Six models were tested that were built on different sets of rules and combinations thereof. Since the cleavage of protein precursors is sequence- and tissue-specific, the sequence specificity of proteases in human CSF was investigated: Pairs of amino acids, the 'motifs', at the four positions, before and after amino-terminal and carboxy-terminal cleavage site, were differentiated for cleavage pattern analysis (Figure 38).

The described rules applied to sequence prediction are generic since they are based on the x-fold increase of the probability for the given event, and scoring the respective proposals with x bonus points. Combining the individual rules by summing the bonus points substantially increased the accuracy of prediction. This confirms that the rules of the different approaches are complementary and not contradictive. The magnitude of the bonus points was significantly different due the individual definition for every single parameter.

However, if the algorithm is applied to other sample matrices, the presented rules most likely will have to be redefined. The rules can be determined empirically using these other sample matrices. It is also recommended to test the parameters r and bonus points in a given data set with known peptides to determine the false positive rate prior to use for prediction of peptide sequences. The parameters should be readjusted until the false positive rate and prediction number match the design and requirements of the experimental purposes.

As a result of the combination of statistical analysis and peptide biology for the definition of a set of specific rules, a promising model is envisaged that predicts the sequence of peptides with high accuracy. A system of bonus points was used to select the prediction, which fitted the model best. Proposals with the highest sore of bonus points were compared with ESI-MS/MS identifications, and were found to be correctly predicting 85 % of protein precursors, start- and end positions and 89 % of precursor protein only. A further improvement is expected by using MALDI-ToF measurements in the reflectron mode with a mass accuracy of less than 30 ppm, and with broader sequence coverage to redefine the model.

As a consequence of the promising results of this proof-of-concept study the following procedure is suggested, in case a rapid overview of a peptide content of a new sample source has to be obtained: The peptide coordinates of a novel samples source are defined based on a representative peptide display. Thereafter, the related peptide coordinates are determined by calculating the CAN of any peptide coordinate: Hubs with the most network members are considered as related to a multitude of other peptide coordinates, thus being the most representative ones (Lamerz et al., 2005, Proteomics, 5:x-xx). These peptide coordinates should be identified first. On the basis of these identifications, CAN is used to predict sequence of the remaining not identified peptide coordinates. The identification of peptide signal coordinates that are adequately described by the model can be postponed or discarded from the identification list, leaving more resources for the identification of less abundant peptides or peptides unsatisfactorily described by the model. This procedure can be repeated several times with the additional sequence information generated during the process, resulting in a reduction of MS/MS identification work while achieving a comparably deep insight in the content of the novel sample source.

Posttranslational and other modifications of peptide sequences can be included by scanning the sequence of the hub peptide for specific motifs characteristic or susceptible for such modifications, such as phosphorylation, dephosphorylation, oxidation, reduction, glycosylation, deglycosilation, acetylation and other modifications known for peptides. Subsequently, the mass difference between the hub peptide and its related peptides can be analyzed to assess whether the mass difference corresponds to the respective posttranslational modification. This implies the implementation of many, even thousands of motifs, as documented in PROSITE (Falquet et al., 2002, Nucleic Acids Res., 30:235-238), and the scanning process can be computationally laborious.

## Example 7

CANs were also exploited for the discovery of surrogates of biomarkers. The whole albumin molecule is routinely used in diagnosis as gold standard to determine the integrity of the brain barriers (blood-brain barrier, blood-CSF barrier). The ratio of albumin concentration in CSF and blood, the 'albumin ratio', correlates with the extent of a barrier disruption (Reiber et al., 1980, J. Neurobiol., 224:89-99), leading to the increased transfer of "blood born" peptides and proteins into the CSF. Previous work shows, that the albumin peptide representing amino acids 25-48 of human albumin can serve as a marker for an impaired brain barrier (Heine et al., 2002, J. Chromatogr. B Analyt. Technol. Biomed. Life. Sci. 782(1-2):353-61). By way of this example (see Figures 39 and 40) it is demonstrated that members of the CAN calculated by use of the albumin 25-48 peptide as hub peptide have the same potential as surrogate markers to assess disruption of the brain barrier as the albumin 25-48 peptide and thus that CANs are suitable to identify surrogate markers of known markers (in this case of albumin 25-48).

This was tested in an independent experimental set-up using well-documented CSF samples taken from patients with different severe disruptions of the blood-CSF barrier. Subsequently to the identification of these potential surrogates for albumin 25-48, the surrogates identified were searched in the original dataset described in a previous work (Heine et al., 2002, J. Chromatogr. B Analyt. Technol. Biomed. Life. Sci. 782(1-2):353-61). It was confirmed, that the surrogates proposed (see Figure 39 and 40) are suitable to diagnose the impaired brain barrier of the patients analyzed in a previous work (Heine-et al., 2002, J. Chromatogr. B Analyt. Technol. Biomed. Life. Sci. 782(1-2):353-61). This shows, that CANs are suitable to identify surrogates of known markers, as claimed in this invention.

The samples used for the study performed with the present invention were e.g. human CSF collected by lumbar puncture from 74 patients suffering from vascular dementia, Lewy-body dementia, frontotemporal dementia, Parkinson's disease, depression, lumbago, facial paresis, vertigo, polyneuropathy or optic neuritis.

These samples were analysed by reversed-phase chromatography and MALDI mass spectrometry under the same conditions as the samples in example 6. Albumin 25-48 as the hub peptide displayed strong correlations ($|r| > 0.75$) with 25 different peptide signals and, most importantly, a significant correlation with the albumin ratio in the new sample set, which was determined using standard albumin-ELISA tests, as known in the art ($|r| = 0.73$). It was found that all network members correlated positively with the albumin quotient, and 16 out of 25 reached a significant level ($|r| > 0.7$, $n=9$, $p<0.05$). This positive correlation with the established and accepted albumin ratio as a measure for blood-CSF barrier disruption indicate the correctness of the predicted peptide-to-peptide relations in CSF. Five prominent network members were identified subsequently as structurally similar amino-terminal fragments of albumin, namely albumin 25-48, albumin 25-50, albumin 25-51 and albumin 27-50 by sequencing. The novel peptide alpha-1-antitrypsin 397-418 of the albumin 25-48 CAN correlated even stronger to the albumin quotient ($|r| = 0.83$) than the albumin fragment itself ($|r| = 0.73$). The identification of the alpha 1-antitrypsin 397-418 peptide as a member of the albumin CAN highlights the power of the claimed methodology for the identification of new chemically unrelated peptide surrogates with a high diagnostic potential. Interestingly, alpha 1-antitrypsin as whole protein is already described as a protein directly correlating with disturbances in the blood-brain barrier determined by assessment of the ratio of albumin in CSF to that in serum (Pearl et al., 1985, Arch. Neurolo. 42:775-777) further supporting that CANs are suitable to predict surrogates of known markers.

The person skilled in the art will appreciate form the foregoing that the range of application of CANs is expandable to any proteomic approach that allows a semi-quantitative analysis of components, for example data from two-dimensional gels (2D-gels). In such cases pair wise correlation coefficients of the components can be calculated, but it is of utmost importance to verify spot identity, avoiding inclusion of spots deriving from contaminating proteins. There, the precision of the two dimensions of peptidomics CAN, i.e. chromatographic fraction in RP-HPLC (usually better than 1 %) and in MALDI-MS (usually better than 100 ppm) is highly superior to separations obtained by 2D-gel electrophoresis (Schulz-Knappe et al., 2001, Comb. Chem. High Throughput. Screen., 4:207-217). On the other hand, CANs based on the approach described in the examples of the present invention are restricted to proteins < 15 kDa, while CAN based on 2D-gels can also address networks of larger proteins.

CANs are also applicable to peptide and protein quantification data from Isotope-Coded Affinity Tag (ICAT) mass spectrometric experiments. In ICAT experiments peptides and proteins present in the samples are isotopically labelled through a reactive group that specifically binds to cysteine residues. In a low molecular weight (peptidome) region, the number of peptides and small proteins that do not contain cysteins needed for ICAT labelling is higher compared to the proteomics field, thus decreasing the efficiency of ICAT. Novel labels such as iTRAQ which is an amine specific isotope labelling technique developed by Applied Biosystems, Foster City, CA, USA, will allow detection of all small proteins/peptides in CAN experiments.

It is envisaged that CANs will also support the interpretation of data from tryptic digestions of protein or peptide containing samples. Although the CAN methods presented here are based on undigested, native peptides, a similar clustering of different peptide species derived from the same precursor after tryptic digestion is possible. While this invention has been described with reference to preferred embodiments, it will be understood by those skilled in the art that various changes or modifications in form and detail may be made without departing from the scope of the invention as defined in the following claims.

For example it is readily apparent that the present invention can advantageously be utilized basically with all kinds of samples potentially containing peptides, such as samples from animals, plants, fungi, humans, parasites, microorganisms, such as bacteria, yeasts, viruses, and the like, samples from food or other agricultural materials such as meat, milk, grain, vegetables, wool, cotton, silk, samples from cosmetic products or other products containing peptides such as cleaning agents (often containing proteolytic enzymes), etc. Samples for example can be plasma, serum, hemofiltrate, whole blood, blood cells, tissues samples, in vitro grown cells, cell culture supernatants, urine, cerebrospinal fluid, lymph fluid, sputum, tear fluid, ascites, preparations of cell organelles, tissue homogenate or homogenates of a virus, a microorganism, a parasite, a multi-cellular organism, an animal, a fungus or a plant and the like or combinations thereof. Examples of combinations are in vitro cultured cells infected with a microorganism or treated with pharmaceutical substances, tissue samples of humans infected with a microorganism, food products containing microorganisms, tissue culture supernatants of cells treated with peptides or mixtures of peptides present in food or cosmetic products, and the like.

## Claims

1. A method of providing a representative, non-redundant overview of the peptide content of a sample type by analyzing a plurality of samples using its peptide topology, wherein the method comprises the steps of:

   a) providing a respective mass spectrum for each sample of said plurality of samples, wherein signal intensity peaks correspond to potential peptides,
   b) computing the measures of correlation between the signal intensities of said potential peptides,
   c) grouping potential peptides together, which exhibit a degree of correlation among each other above a certain threshold, thereby providing a plurality of correlation associated networks of potential peptides, and
   d) assigning one representative potential peptide out of each correlation associated network as a representative peptide to said correlation associated network of said sample type.

2. The method according to claim 1, wherein a plurality of minimal spanning tree diameters is computed as a measure of correlation using the signal intensity of said potential peptides in said samples, wherein the selection of potential peptides is done by using a minimal spanning tree diameter threshold, wherein the minimal spanning tree diameter for an association of two potential peptides has to be above an adjustable threshold of at least 0.425 times the number of samples.

3. The method according to anyone of the preceding claims, wherein the method comprises the additional step of at least one fractionating step of said samples prior to providing the mass spectra of said samples and wherein at least one fraction of said samples is used for providing said mass spectra.

**4.** The method according to anyone of the preceding claims, wherein the measures of correlation between the signal intensities of said potential peptides are computed by using at least one measure of correlation selected from the group consisting of "Pearson Product-Moment Correlation Coefficient", "Spearman's rank order Correlation Coefficient", "Kendall's Tau", "Kendall's Coefficient of Concordance", "Goodman and Kruskal's Gamma" and "Minimal Spanning Tree diameters".

**5.** The method according to anyone of the preceding claims using, wherein the mass spectra are calibrated by at least one of the group consisting of "Simple Offset Correction", "2-Point Baseline Correction", "Multi-Point Baseline Correction", "Interactive Polynomial Baseline Correction", "Function Fit Baseline Correction", and "GIFTS (Auto Leveling Method) Baseline Correction".

**6.** The method according to anyone of the preceding claims, wherein the method further comprises the step of identifying outlier samples by using at least one of the group consisting of "Principal Component Analysis", "multivariate calibration partial least-squares", and "Replicator Neural Networks".

**7.** The method according to anyone of the preceding claims, wherein the computation of the measures of correlation is repeated at least once using the peptide coordinates resulting from the previous round of computations of measures of correlation, thereby providing the measures of correlation of $2^{nd}$ or higher order neighborhood.

**8.** The method according to anyone of the preceding claims, wherein additional coordinates besides the mass are used, selected from the group consisting of fraction number, elution time, retention time, protein chip coordinates, peptide concentration, enzyme activities, structural properties, chemical properties and biological properties.

**9.** The method according to anyone of the preceding claims, wherein MALDI mass spectrometry or ESI mass spectrometry is used to generate the mass spectra.

**10.** The method according to anyone of the preceding claims, wherein the samples are homogeneous.

**11.** The method according to anyone of the preceding claims, wherein the computation of measures of correlation is done in advance prior to the analysis to accelerate the speed of the analysis using pre-determined values for the measures of correlation.

**12.** The method according to anyone of the preceding claims, wherein the necessary sequence information is provided by manual input or automatically queried from a database.

**13.** The method according to anyone of the preceding claims, wherein the corresponding results are automatically combined with data from other sources chosen from the group consisting of sequence databases, patent databases, literature databases, medical databases, 3D structure databases, databases containing information about enzyme recognition sites, postranslational modifications, genetic polymorphisms, clinical trials.

**14.** The method according to anyone of the preceding claims, wherein at least one step of data processing or data supply is done using a remote computer system and wherein the user is connected via an internet, intranet or other network to the remote computer system.

**15.** A digital computer system comprising means for carrying out a method according to any one of the preceding claims.

**16.** A computer readable medium storing a computer program adapted to perform a method according to any one of claims 1 to 14.

## Patentansprüche

**1.** Verfahren zur Bereitstellung eines repräsentativen, nicht-redundanten Überblicks über den Peptid gehalt einer Probenart durch einer Analyse einer Vielzahl von Proben unter Berücksichtigung ihrer Peptid-Topolgie, wobei das Verfahren die Schritte umfasst:

(a) Bereitstellen eines entsprechenden Massenspektrums für jede Probe dieser Vielzahl von Proben, wobei die Signalintensitätspeaks den potentiellen Peptiden entsprechen,

(b) Berechnen der Werte der Korrelation zwischen den Signalintensitäten dieser potentiellen Peptide,
(c) Gruppieren der potentiellen Peptide, die über einen gewissen Grenzwert einen Korrelationsgrad untereinander aufzeigen, und damit Bereitstellen einer Vielzahl von Korrelations-assoziierten Netzwerken potentieller Peptide, und
(d) Bestimmen eines repräsentativen potentiellen Peptids aus jedem dieser Korrelations-assoziierten Netzwerke als repräsentatives Peptid dieses Korrelations-assoziierten Netzwerkes der Probenart.

**2.** Verfahren nach Anspruch 1, wobei eine Vielzahl von minimalen Spannbaumdurchmesser (engl.: spanning tree diameters) als Werte der Korrelation unter Verwendung der Signalintensität dieser potentiellen Peptide in diesen Proben berechnet werden, wobei die Auswahl der potentiellen Peptide unter Verwendung des minimalen Grenzwertes des Spannbaumdurchmessers ausgewählt wird, wobei der minimale Spannbaumdurchmesser für eine Assoziierung von zwei potentiellen Peptiden oberhalb eines einstellbaren Grenzwertes von mindestens dem 0,425-fachen der Zahl der Proben liegt.

**3.** Verfahren nach einem der vorherigen Ansprüche, wobei das Verfahren den weiteren Schritt von mindestens einem Fraktionierungsschritt vor dem Bereitstellen der Massenspektren dieser Proben umfasst und wobei mindestens eine Fraktion dieser Proben zur Bereitstellung der Massenspektren verwendet wird.

**4.** Verfahren nach einem der vorherigen Ansprüche, wobei die Werte der Korrelation zwischen den Signalintensitäten der potentiellen Peptide berechnet wird unter Einsatz mindestens eines Werts der Korrelation ausgewählt aus der Gruppe bestehend aus Pearson Produkt-Moment Korrelation Koeffizient, Spearman's Rank Order Korrelationskoeffizient, Kendall's Tau, Kendall's Koeffizient der Konkordanz, Goodman und Kruskal's Gamma und Minimal Spannbaumdurchmesser.

**5.** Verfahren nach einem der vorherigen Ansprüche, wobei die Massenspektren kalibriert sind durch mindestens einer aus der Gruppe bestehend aus Einfach Offset Korrektion, 2-Punkt Basislinie Korrektion, Mehrpunkt Basislinie Korrektion, Interaktive polynominale Basislinie Korrektion, Funktion Fit Basislinie Korrektion und GIFTS (Auto Leveling Verfahren) Basislinie Korrektion.

**6.** Verfahren nach einem der vorherigen Ansprüche, wobei das Verfahren weiterhin den Schritt umfasst, des Identifizierens von Ausreißerproben unter Verwendung von mindestens einer Gruppe bestehend aus prinzipieller Komponentenanalyse, multivariater Kalibrierung von partial least-squares und Replicator neurale Netzwerke.

**7.** Verfahren nach einem der vorherigen Ansprüche, wobei die Berechnung der Werte der Korrelation mindestens einmal wiederholt wird unter Verwendung der Peptidkoordinaten, die sich aus der vorherigen Runde der Berechnungen der Werte der Korrelation ergibt, und damit Bereitstellen von Werten der Korrelation der zweiten oder höheren benachbarten Ordnung.

**8.** Verfahren nach einem der vorherigen Ansprüche, wobei zusätzliche Koordinaten neben denen der Masse eingesetzt werden ausgewählt aus der Gruppe bestehend aus Fraktionsnummer, Eluierungszeit, Retentionszeit, Protein Chip-Koordinaten, Peptidkonzentration, Enzymaktivitäten, strukturelle Eigenschaften, chemische Eigenschaften und biologische Eigenschaften.

**9.** Verfahren nach einem der vorherigen Ansprüche, wobei MALDI Massenspektrometrie oder ESI Massenspektrometrie zur Generierung des Massenspektrums verwendet wird.

**10.** Verfahren nach einem der vorherigen Ansprüche, wobei die Proben homogen sind.

**11.** Verfahren nach einem der vorherigen Ansprüche, wobei die Berechnung der Werte der Korrelation vor der Analyse durchgeführt wird, um die Geschwindigkeit zu erhöhen, unter Verwendung von vorbestimmten Werten für die Werte der Korrelation.

**12.** Verfahren nach einem der vorherigen Ansprüche, wobei die notwendige Sequenzinformation bereitgestellt wird durch manuelle Eingabe oder durch automatische Abfrage aus Datenbanken.

**13.** Verfahren nach einem der vorherigen Ansprüche, wobei die entsprechenden Ergebnisse automatisch kombiniert werden mit Daten von anderen Quellen ausgewählt aus der Gruppe bestehend aus Sequenzdatenbanken, Patentdatenbanken, Literaturdatenbanken, medizinischen Datenbanken, 3D-Strukturdatenbanken, Datenbanken, die Informationen über Erkennungsbereiche von Enzymen, postranslationale Modifikationen, genetische Polymorphismen, klinische Versuche enthalten.

**14.** Verfahren nach einem der vorherigen Ansprüche, wobei mindestens ein Schritt der Datenbearbeitung oder Daten-Zurverfügungstellung über einen entfernt angeordnetes Computersystem geschieht und wobei der Nutzer über Internet, Intranet oder anderem Netzwerk mit dem entfernt angeordneten Computersystem verbunden ist.

**15.** Digitales Computersystem umfassend Mittel zur Durchführung eines Verfahrens gemäß einem der vorherigen Ansprüche.

**16.** Computer-lesbares Medium, das ein Computerprogramm gespeichert hat, das an die Durchführung eines Verfahrens nach einem der Ansprüche 1 bis 14 angepasst ist.

## Revendications

**1.** Procédé pour fournir une vue d'ensemble représentative et non redondante de la teneur en peptide d'un type d'échantillon, par analyse de plusieurs échantillons utilisant sa topologie de peptide, le procédé comprenant les étapes suivantes :

a) fourniture d'un spectre de masse respectif pour chaque échantillon desdits plusieurs échantillons, les pics d'intensité du signal correspondant à des peptides potentiels,
b) calcul des mesures de corrélation entre les intensités de signal desdits peptides potentiels,
c) regroupement des peptides potentiels entre eux, qui présentent un degré de corrélation entre eux, supérieur à un certain seuil, fournissant ainsi plusieurs réseaux associés de corrélation de peptides potentiels, et
d) attribution d'un peptide potentiel représentatif en dehors de chaque réseau associé de corrélation, en tant que peptide représentatif dudit réseau associé de corrélation dudit type d'échantillon.

**2.** Procédé selon la revendication 1, dans lequel on calcule plusieurs diamètres d'arbre de recouvrement minimal en tant que mesure de corrélation utilisant l'intensité de signal desdits peptides potentiels dans lesdits échantillons, la sélection de peptides potentiels étant faite en utilisant un seuil de diamètre d'arbre de recouvrement minimal, le diamètre d'arbre de recouvrement minimal pour une association de deux peptides potentiels devant être supérieur à un seuil ajustable d'au moins 0.425 fois le nombre d'échantillons.

**3.** Procédé selon l'une quelconque des revendications précédentes, dans lequel le procédé comprend l'étape supplémentaire consistant en au moins une étape de fractionnement desdits échantillons avant de fournir les spectres de masse desdits échantillons, et au moins une fraction desdits échantillons étant utilisée pour fournir lesdits spectres de masse.

**4.** Procédé selon l'une quelconque des revendications précédentes, dans lequel on calcule les mesures de corrélation entre les intensités de signal desdits peptides potentiels, en utilisant au moins une mesure de corrélation sélectionnée dans le groupe consistant en « coefficient de corrélation du produit des moments de Pearson », « coefficient de corrélation de rang de Spearman », « tau de Kendall », « coefficient de concordance de Kendall », « gamma de Goodman et Kruskal », et « diamètre d'arbre de recouvrement minimal ».

**5.** Procédé selon l'une quelconque des revendications précédentes, dans lequel on calibre des spectres de masse grâce à au moins l'un des groupes consistant en « correction d'offset simple », « correction 2 points de ligne de base », « correction multipoints de ligne de base », « correction polynomiale interactive de ligne de base », « correction de ligne de base par fonction de régression », et « correction GIFT (procédé d'auto égalisation) de ligne de base ».

**6.** Procédé selon l'une quelconque des revendications précédentes, dans lequel le procédé comprend en outre l'étape d'identification d'échantillons aberrants, en utilisant au moins l'un des groupes consistant en « analyse de composant principal », « calibration multivariable des moindres carrés partiels », et « réseaux neuraux réplicateurs ».

**7.** Procédé selon l'une quelconque des revendications précédentes, dans lequel on répète le calcul des mesures de corrélation au moins une fois en utilisant les coordonnées de peptide résultant de la série précédente de calculs de mesures de corrélation, fournissant ainsi les mesures de corrélation d'ordre 2 ou supérieur.

8. Procédé selon l'une quelconque des revendications précédentes, dans lequel on utilise des coordonnées additionnelles en plus de la masse, sélectionnées dans le groupe consistant en numéro de fraction, temps d'élution, temps de rétention, coordonnées de fragment protéinique, concentration de peptide, activités enzymatiques, propriétés structurales, propriétés chimiques et propriétés biologiques.

9. Procédé selon l'une quelconque des revendications précédentes, dans lequel on utilise pour générer le spectre de masse une spectrométrie de masse MALDI ou une spectrométrique de masse ESI.

10. Procédé selon l'une quelconque des revendications précédentes, dans lequel les échantillons sont homogènes.

11. Procédé selon l'une quelconque des revendications précédentes, dans lequel on effectue le calcul de mesures de corrélation préalablement à l'analyse, pour accélérer la vitesse de l'analyse en utilisant des valeurs prédéterminées pour les mesures de corrélation.

12. Procédé selon l'une quelconque des revendications précédentes, dans lequel on fournit la séquence d'information nécessaire par entrée manuelle ou automatique demandée par une base de données.

13. Procédé selon l'une quelconque des revendications précédentes, dans lequel on combine automatiquement les résultats correspondants avec des données provenant d'autres sources choisies dans le groupe consistant en des bases de données de séquences, des bases de données de brevets, des bases de données de littérature, des bases de données médicales, des bases de données de structures 3D, des bases de données contenant des informations sur des sites de reconnaissance d'enzyme, des modifications post-translationnelles, des polymorphismes génétiques, des essais cliniques.

14. Procédé selon l'une quelconque des revendications précédentes, dans lequel on effectue au moins une étape de traitement de données ou mise à disposition de données en utilisant un système d'ordinateur distant, et dans lequel l'utilisateur est connecté au système d'ordinateur distant via un réseau internet, intranet, ou autre.

15. Système d'ordinateur numérique comprenant des moyens pour mettre en oeuvre un procédé selon l'une quelconque des revendications précédentes.

16. Media lisible par ordinateur stockant un programme d'ordinateur adapté pour exécuter un procédé selon l'une quelconque des revendications 1 à 14.

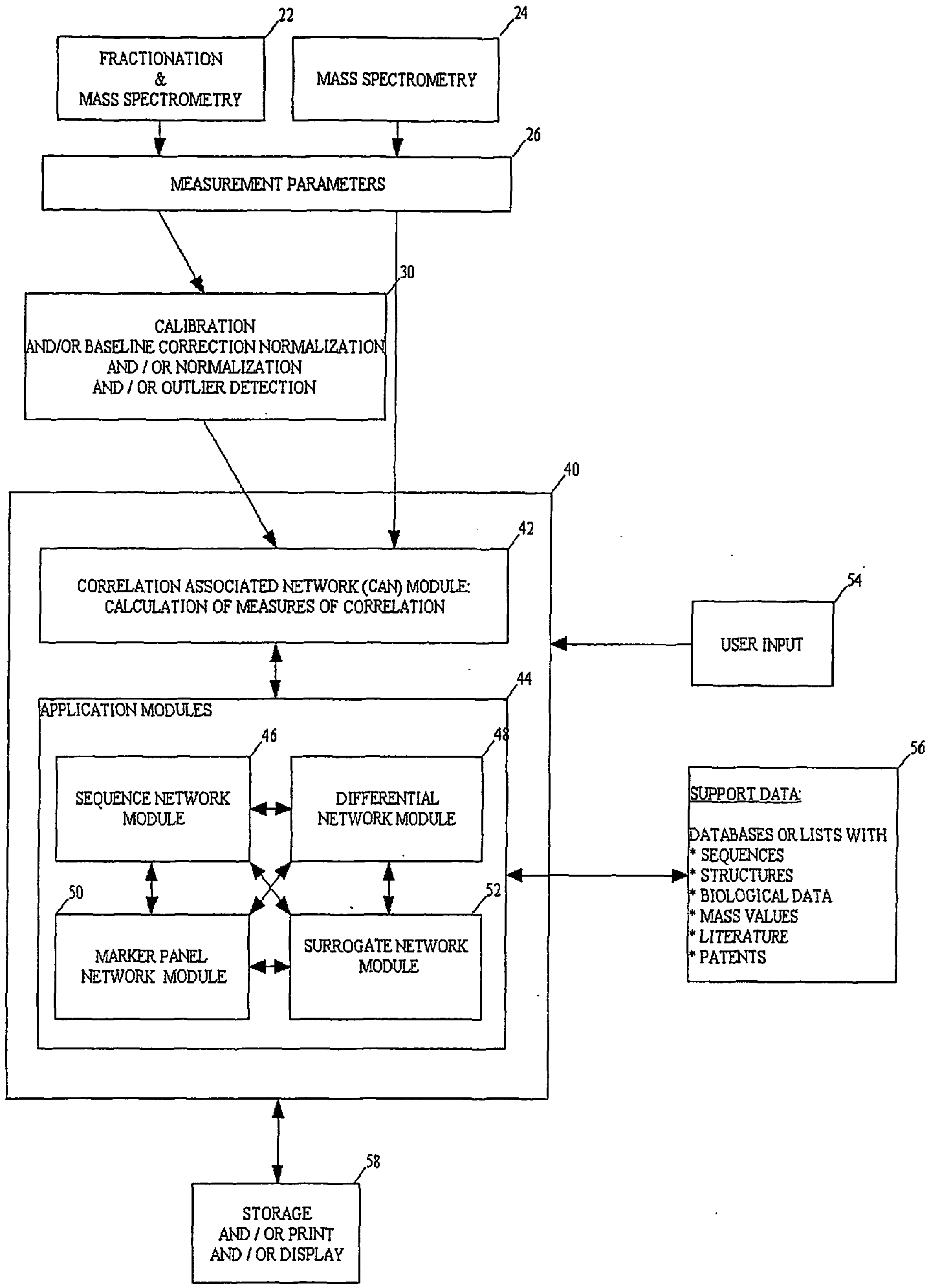
22
FRACTIONATION & MASS SPECTROMETRY
24
MASS SPECTROMETRY
26
MEASUREMENT PARAMETERS
30
CALIBRATION AND/OR BASELINE CORRECTION NORMALIZATION AND / OR NORMALIZATION AND / OR OUTLIER DETECTION
40
42
CORRELATION ASSOCIATED NETWORK (CAN) MODULE: CALCULATION OF MEASURES OF CORRELATION
54
USER INPUT
44
APPLICATION MODULES
46
SEQUENCE NETWORK MODULE
48
DIFFERENTIAL NETWORK MODULE
50
MARKER PANEL NETWORK MODULE
52
SURROGATE NETWORK MODULE
56
SUPPORT DATA:
DATABASES OR LISTS WITH
* SEQUENCES
* STRUCTURES
* BIOLOGICAL DATA
* MASS VALUES
* LITERATURE
* PATENTS
58
STORAGE AND / OR PRINT AND / OR DISPLAY

**Fig. 1**

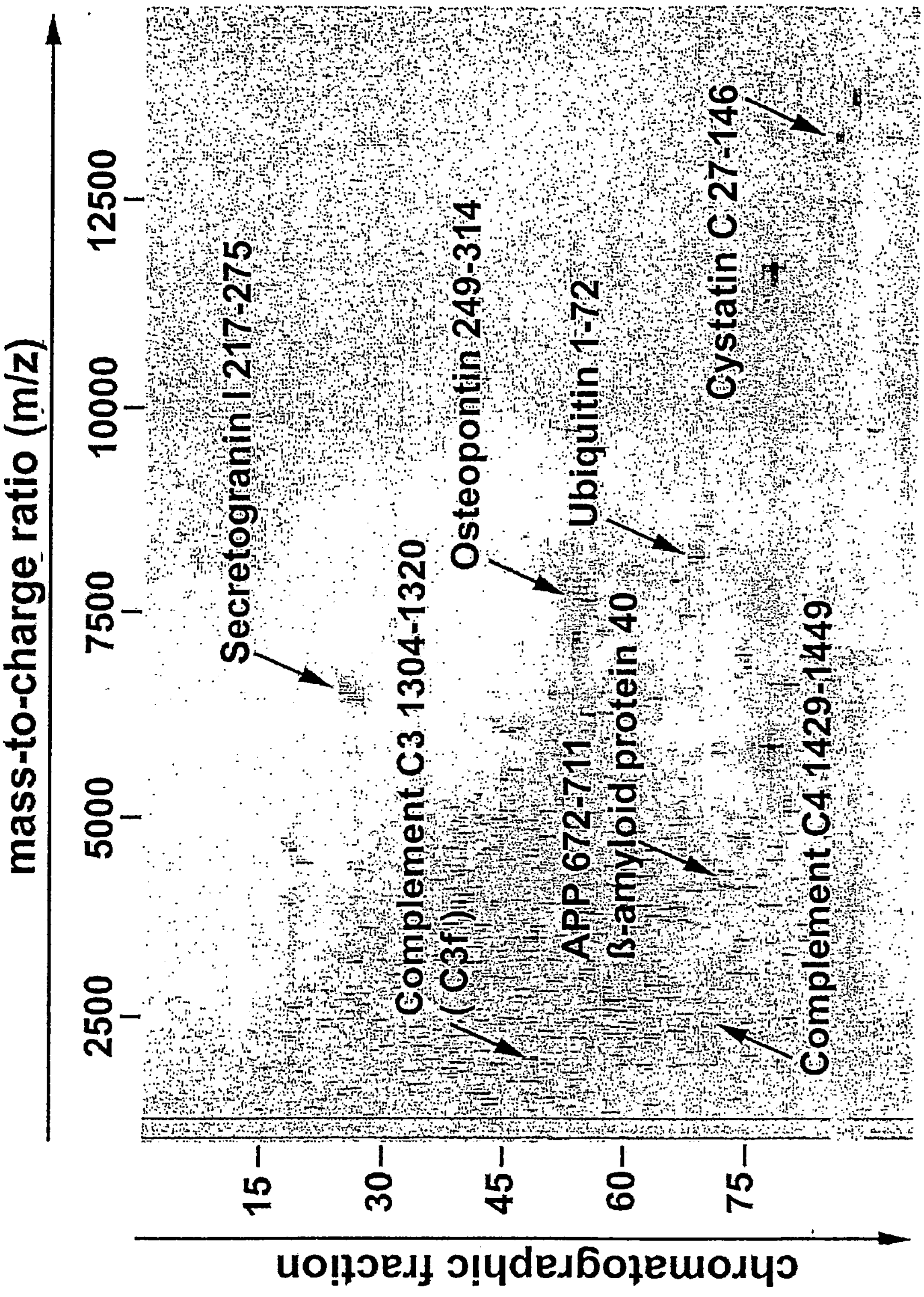
mass-to-charge ratio (m/z)
2500
5000
7500
10000
12500
Secretogranin I 217-275
Complement C3 1304-1320
(C3f)
Osteopontin 249-314
APP 672-711
ß-amyloid protein 40
Ubiquitin 1-72
Cystatin C 27-146
Complement C4 1429-1449
15
30
45
60
75
chromatographic fraction

Fig. 2

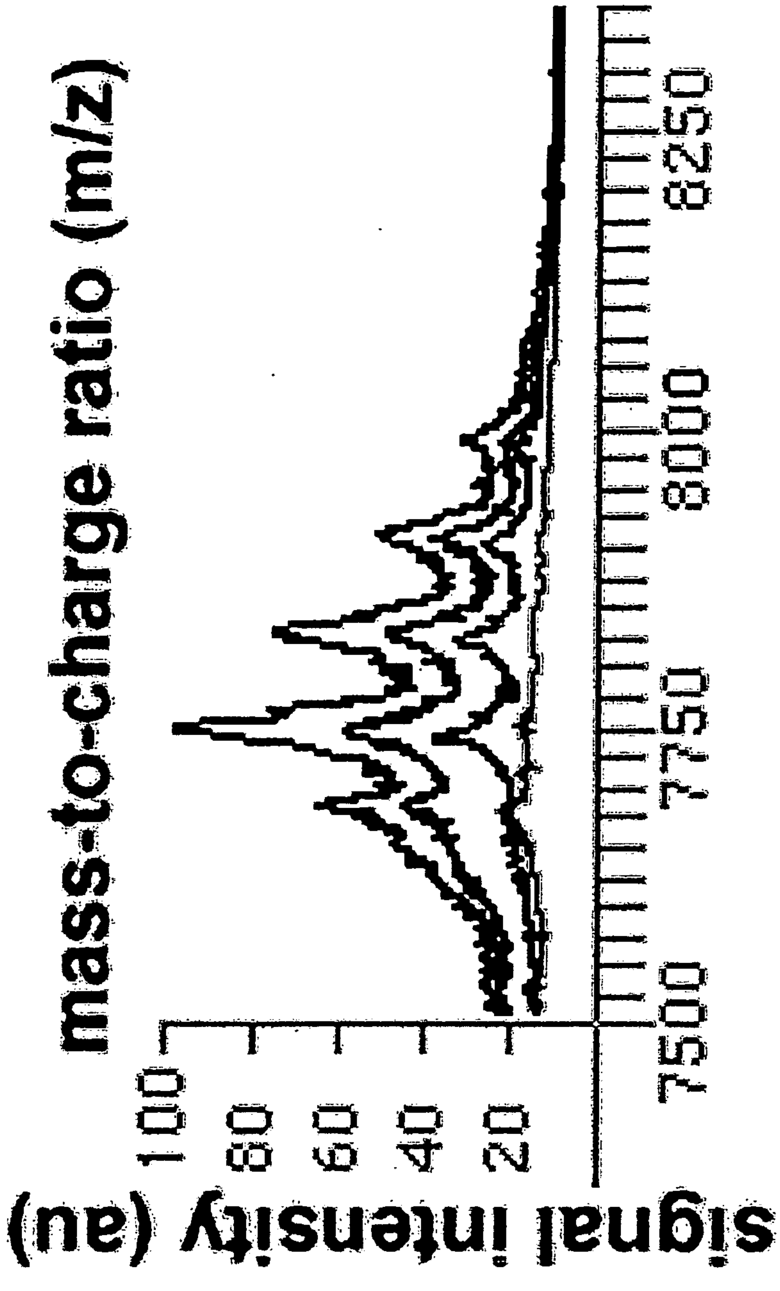
mass-to-charge ratio (m/z)
signal intensity (au)
100
80
60
40
20
7500
7750
8000
8250

Fig. 3

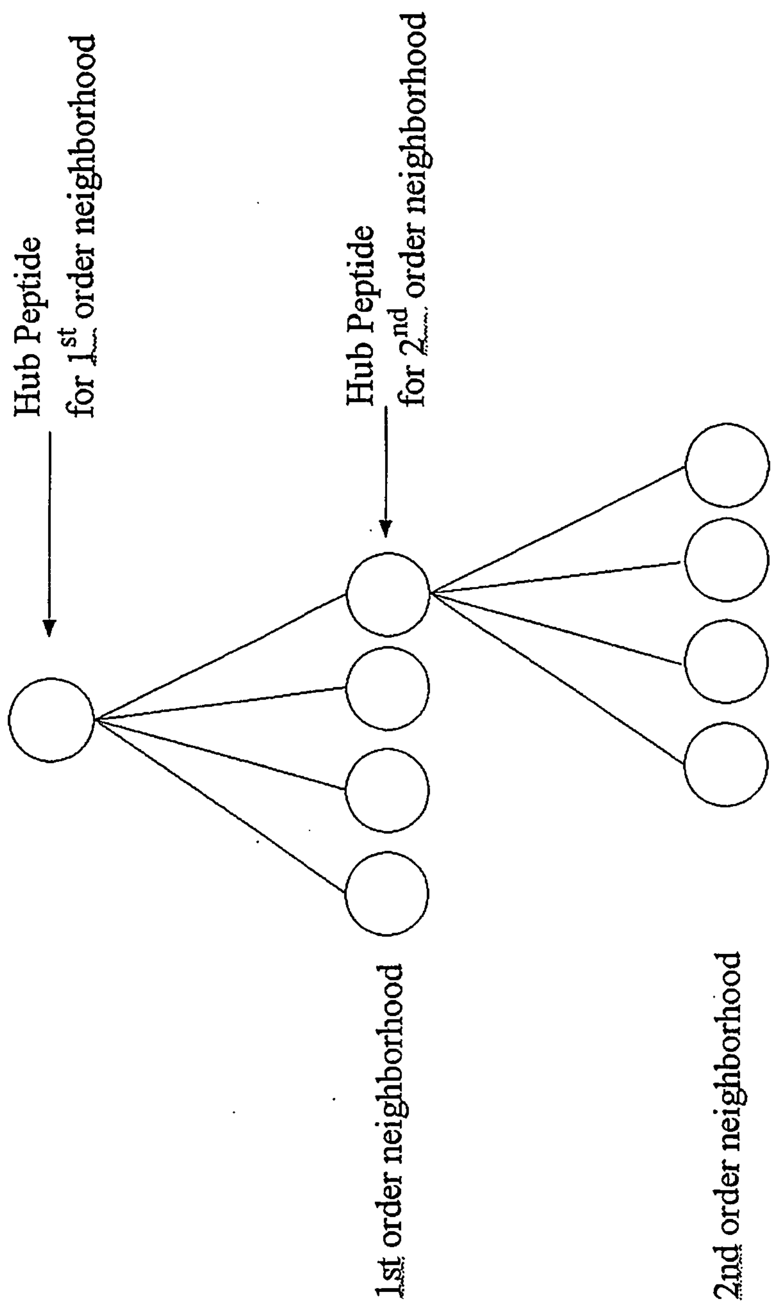
Hub Peptide
for 1st order neighborhood
Hub Peptide
for 2nd order neighborhood
1st order neighborhood
2nd order neighborhood

Fig. 4

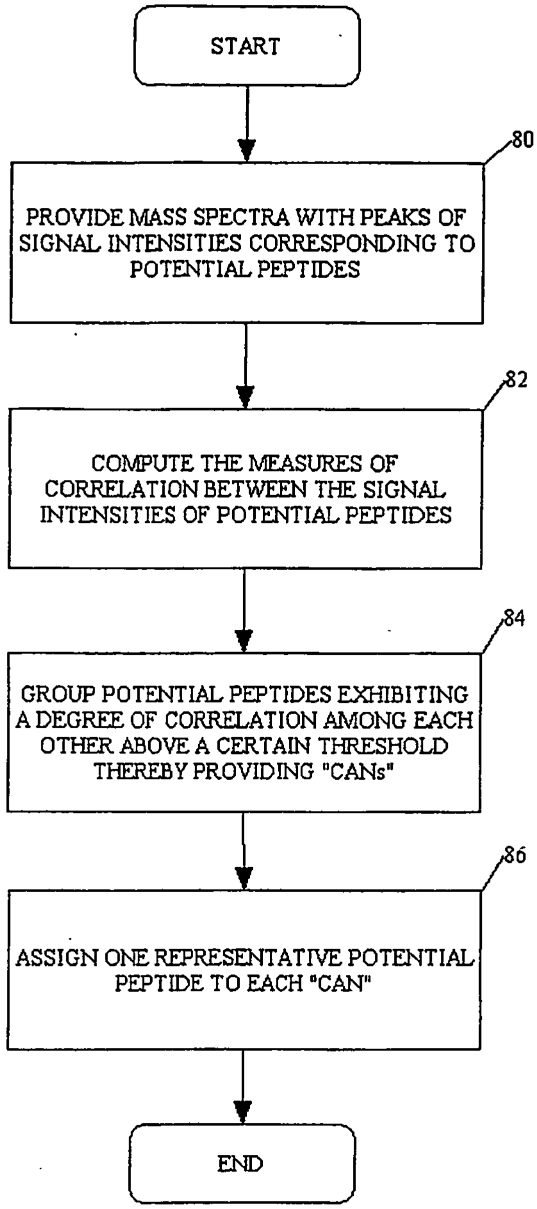
START
80
PROVIDE MASS SPECTRA WITH PEAKS OF SIGNAL INTENSITIES CORRESPONDING TO POTENTIAL PEPTIDES
82
COMPUTE THE MEASURES OF CORRELATION BETWEEN THE SIGNAL INTENSITIES OF POTENTIAL PEPTIDES
84
GROUP POTENTIAL PEPTIDES EXHIBITING A DEGREE OF CORRELATION AMONG EACH OTHER ABOVE A CERTAIN THRESHOLD THEREBY PROVIDING "CANs"
86
ASSIGN ONE REPRESENTATIVE POTENTIAL PEPTIDE TO EACH "CAN"
END

Fig. 5

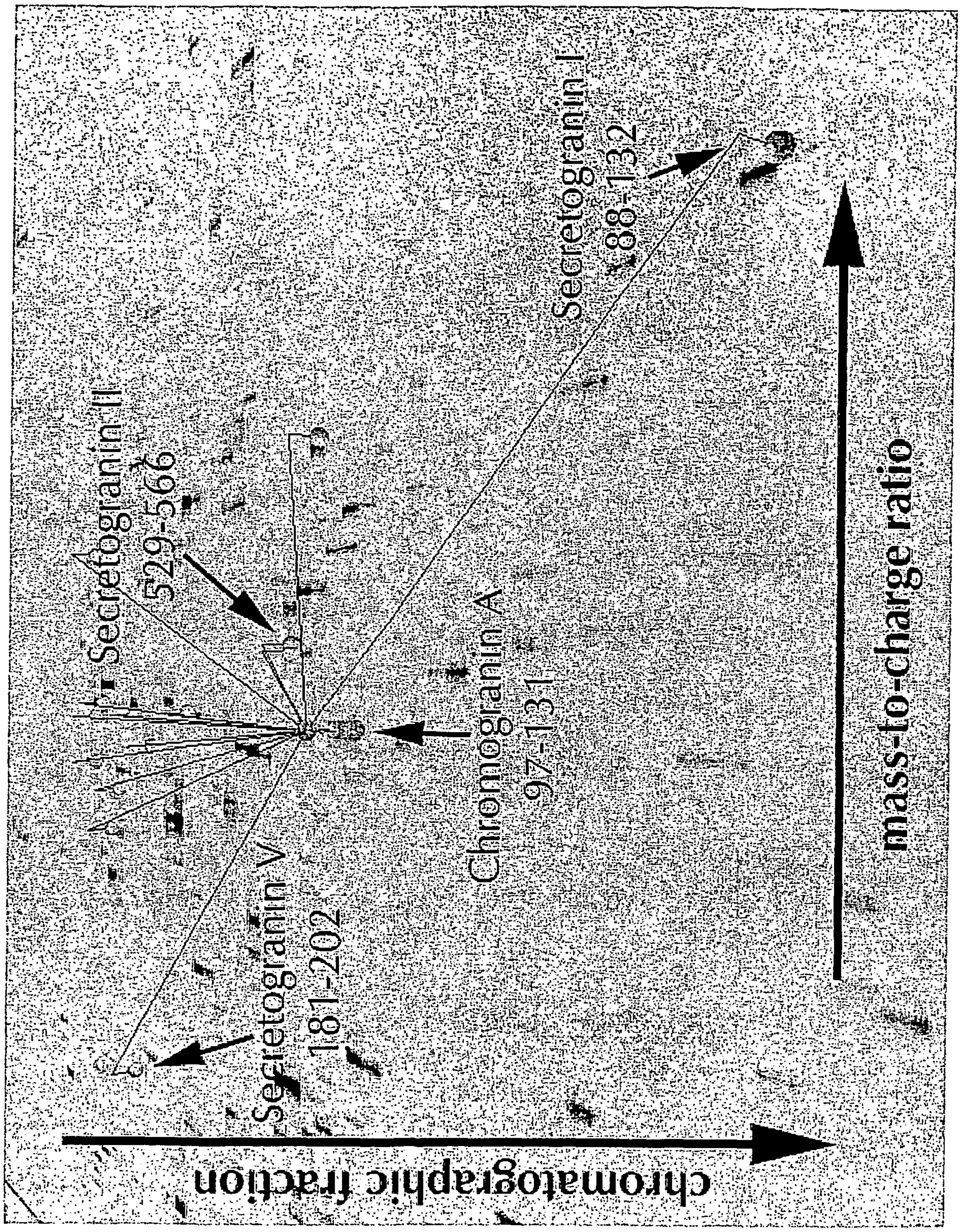
Secretogranin I
88-132
Secretogranin II
529-566
Chromogranin A
97-131
Secretogranin V
181-202
mass-to-charge ratio
chromatographic fraction

Fig. 6

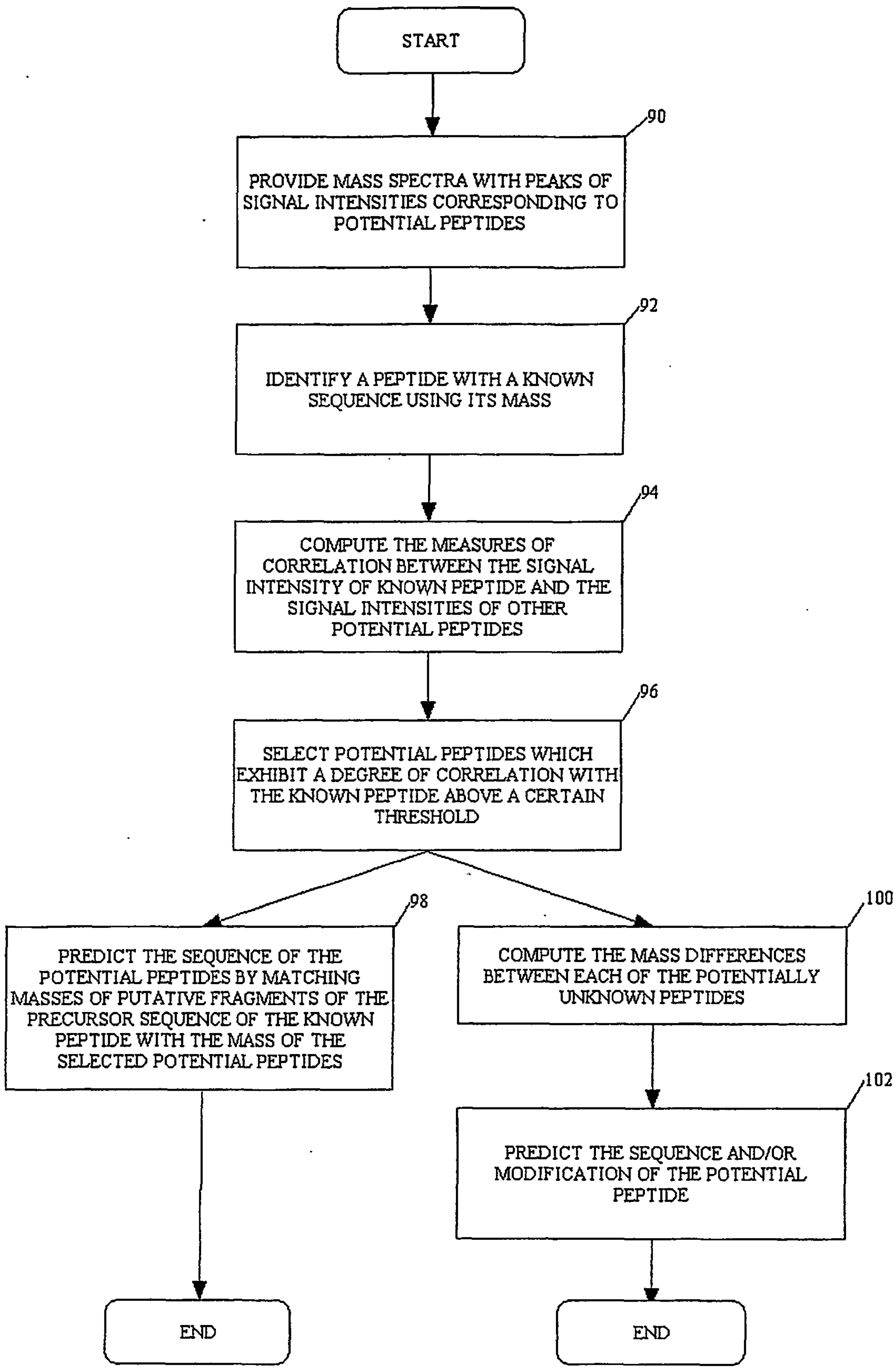
START
90
PROVIDE MASS SPECTRA WITH PEAKS OF SIGNAL INTENSITIES CORRESPONDING TO POTENTIAL PEPTIDES
92
IDENTIFY A PEPTIDE WITH A KNOWN SEQUENCE USING ITS MASS
94
COMPUTE THE MEASURES OF CORRELATION BETWEEN THE SIGNAL INTENSITY OF KNOWN PEPTIDE AND THE SIGNAL INTENSITIES OF OTHER POTENTIAL PEPTIDES
96
SELECT POTENTIAL PEPTIDES WHICH EXHIBIT A DEGREE OF CORRELATION WITH THE KNOWN PEPTIDE ABOVE A CERTAIN THRESHOLD
98
PREDICT THE SEQUENCE OF THE POTENTIAL PEPTIDES BY MATCHING MASSES OF PUTATIVE FRAGMENTS OF THE PRECURSOR SEQUENCE OF THE KNOWN PEPTIDE WITH THE MASS OF THE SELECTED POTENTIAL PEPTIDES
100
COMPUTE THE MASS DIFFERENCES BETWEEN EACH OF THE POTENTIALLY UNKNOWN PEPTIDES
102
PREDICT THE SEQUENCE AND/OR MODIFICATION OF THE POTENTIAL PEPTIDE
END
END

Fig. 7

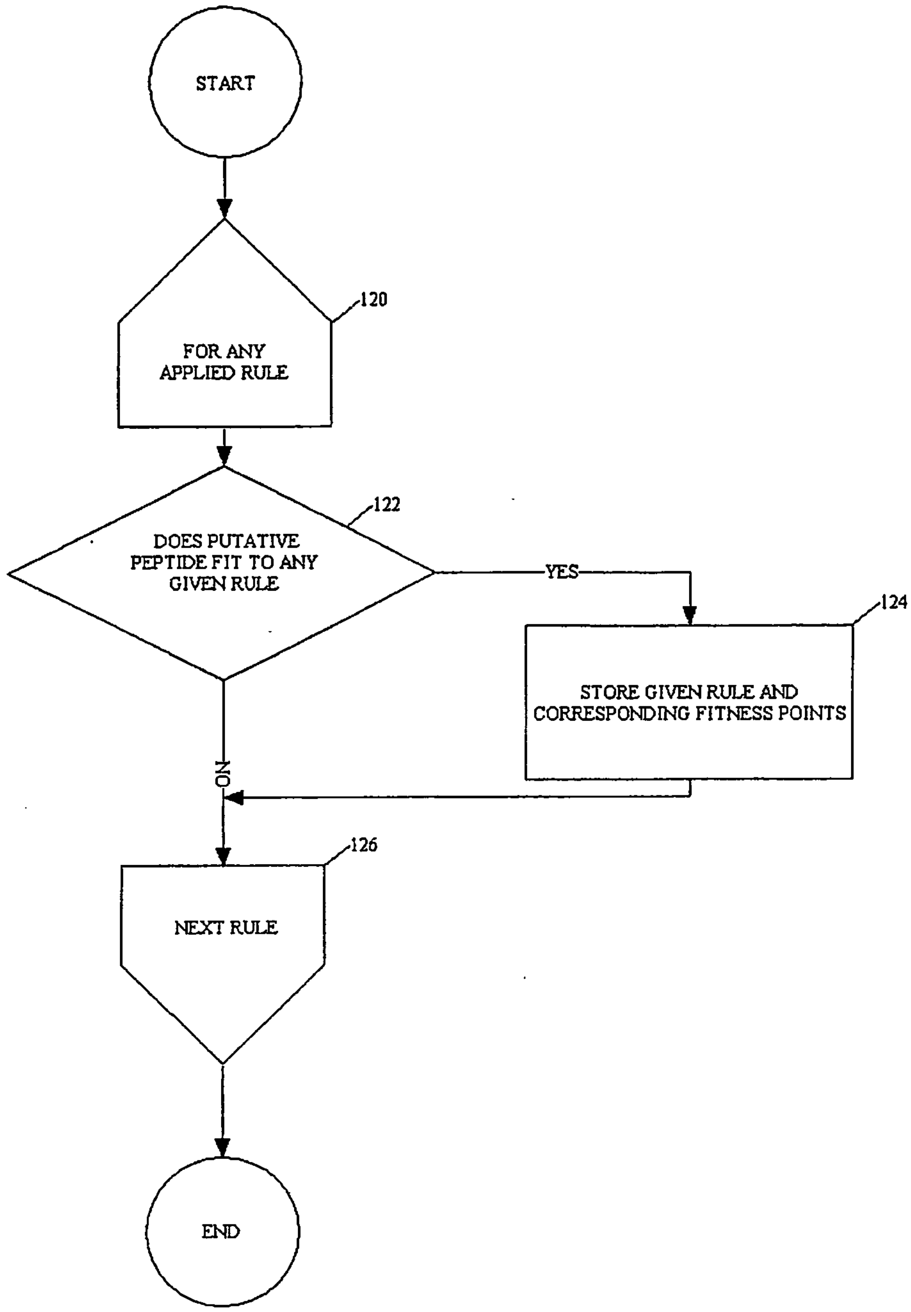
START
120
FOR ANY
APPLIED RULE
122
DOES PUTATIVE
PEPTIDE FIT TO ANY
GIVEN RULE
YES
124
STORE GIVEN RULE AND
CORRESPONDING FITNESS POINTS
NO
126
NEXT RULE
END

**Fig. 8a**

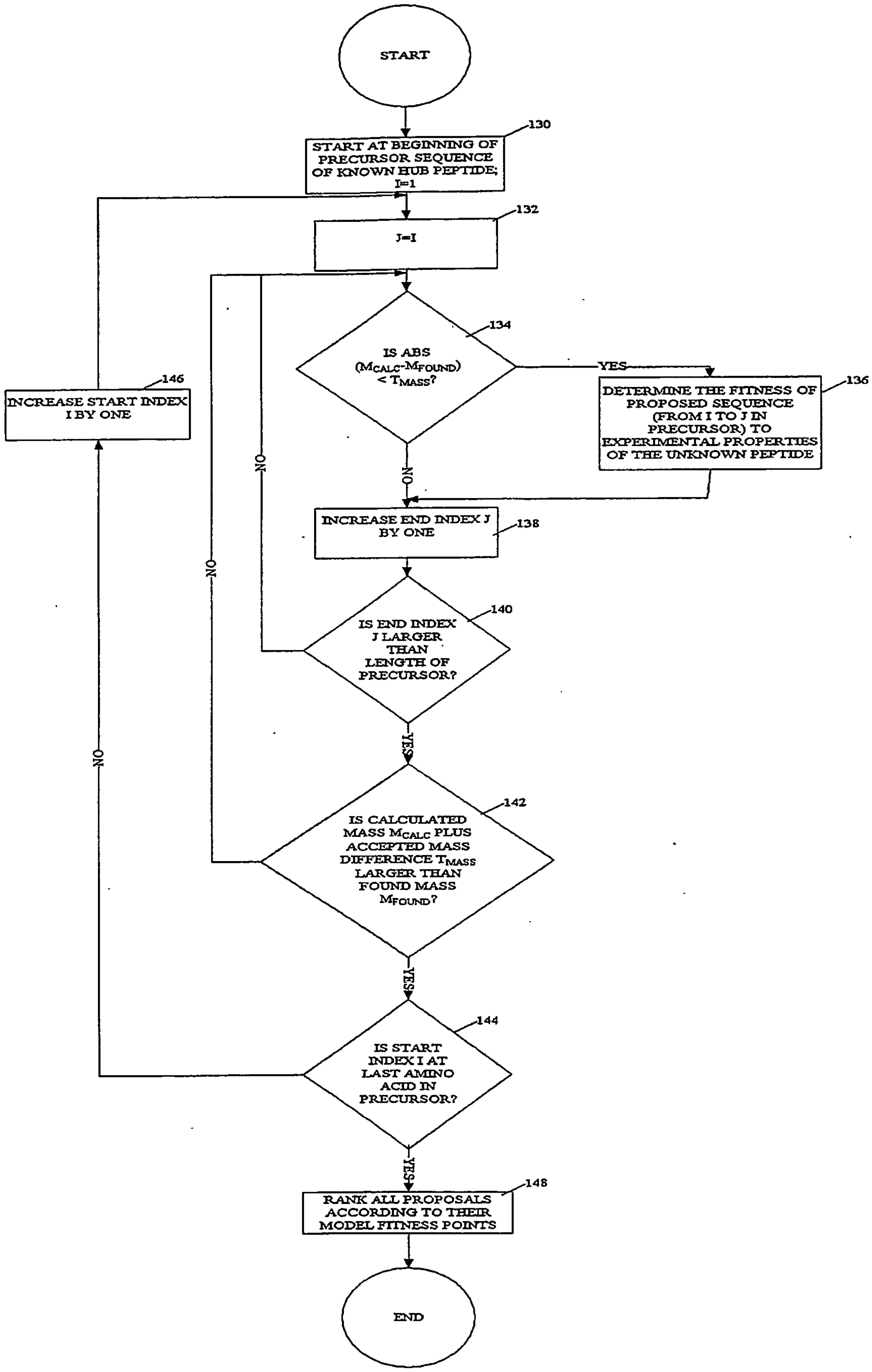
START
START AT BEGINNING OF PRECURSOR SEQUENCE OF KNOWN HUB PEPTIDE; I=1
130
J=I
132
IS ABS ($M_{CALC}$-$M_{FOUND}$) < $T_{MASS}$?
134
YES
DETERMINE THE FITNESS OF PROPOSED SEQUENCE (FROM I TO J IN PRECURSOR) TO EXPERIMENTAL PROPERTIES OF THE UNKNOWN PEPTIDE
136
NO
INCREASE END INDEX J BY ONE
138
IS END INDEX J LARGER THAN LENGTH OF PRECURSOR?
140
NO
YES
IS CALCULATED MASS $M_{CALC}$ PLUS ACCEPTED MASS DIFFERENCE $T_{MASS}$ LARGER THAN FOUND MASS $M_{FOUND}$?
142
NO
YES
IS START INDEX I AT LAST AMINO ACID IN PRECURSOR?
144
NO
INCREASE START INDEX I BY ONE
146
YES
RANK ALL PROPOSALS ACCORDING TO THEIR MODEL FITNESS POINTS
148
END

Fig. 8b

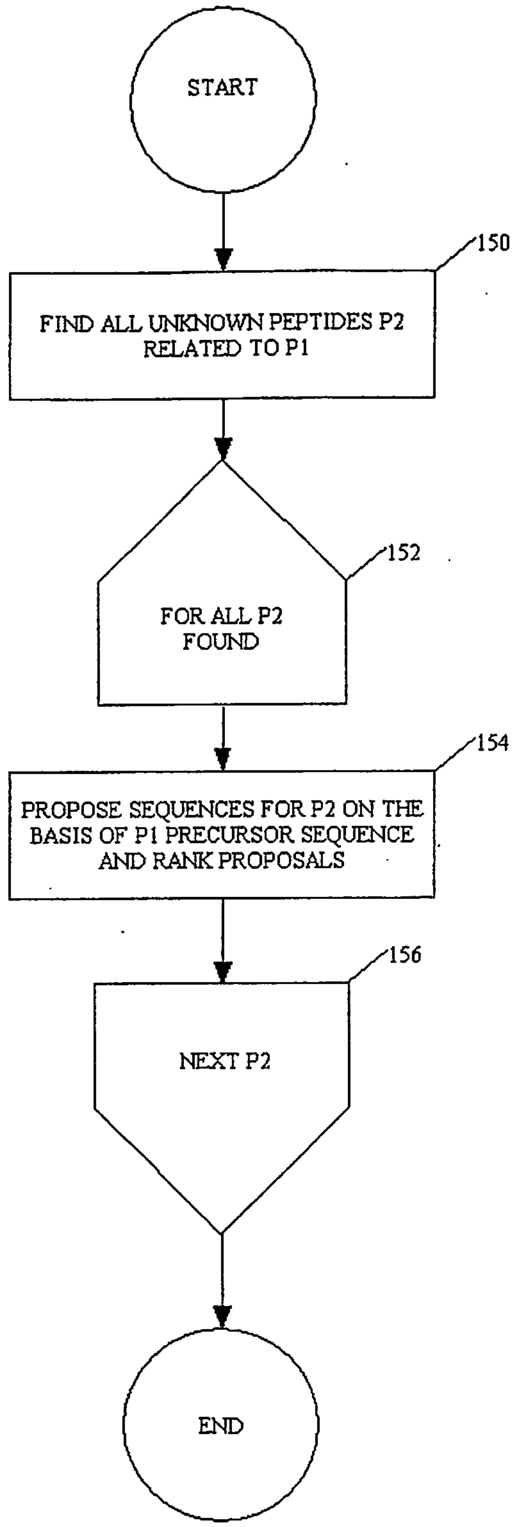
START
150
FIND ALL UNKNOWN PEPTIDES P2 RELATED TO P1
152
FOR ALL P2 FOUND
154
PROPOSE SEQUENCES FOR P2 ON THE BASIS OF P1 PRECURSOR SEQUENCE AND RANK PROPOSALS
156
NEXT P2
END

Fig. 8c

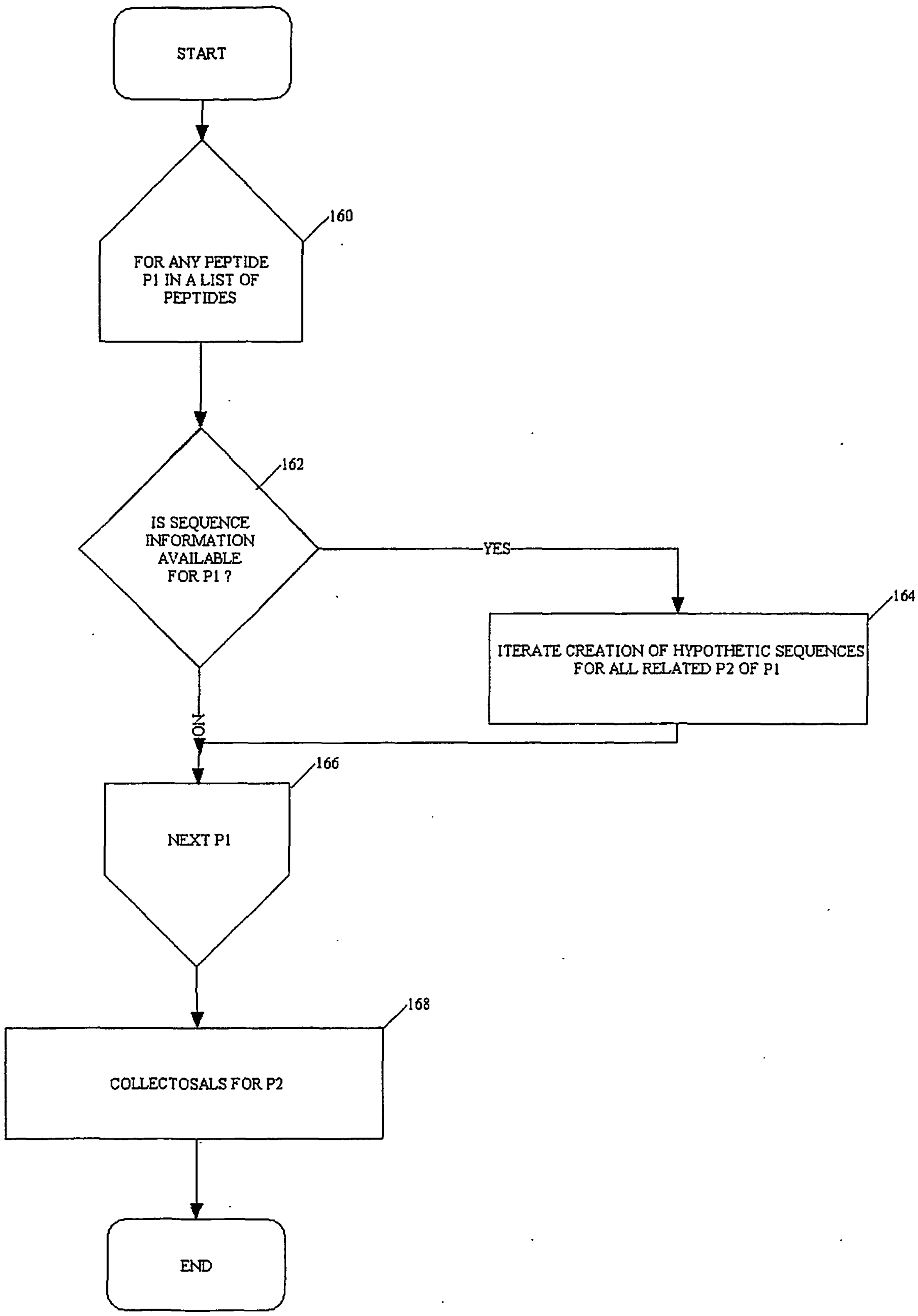
START
160
FOR ANY PEPTIDE
P1 IN A LIST OF
PEPTIDES
162
IS SEQUENCE
INFORMATION
AVAILABLE
FOR P1 ?
YES
164
ITERATE CREATION OF HYPOTHETIC SEQUENCES
FOR ALL RELATED P2 OF P1
NO
166
NEXT P1
168
COLLECTOSALS FOR P2
END

Fig. 8d

| Modification | Monoisotopic Mass | Average Mass |
|---|---|---|
| 4-Phosphopantetheine | 339.0780 | 339.3294 |
| S-Adenosylation | 329.0525 | 329.2091 |
| Acetylation | 42.0106 | 42.0373 |
| ADP-ribosylation (from NAD) | 541.0611 | 541.3052 |
| Biotinylation (amide bond to lysine) | 226.0776 | 226.2994 |
| Carboxylation of Asp and Glu | 43.9898 | 44.0098 |
| C-terminal amide formed from Gly | -0.9840 | -0.9847 |
| Cysteinylation | 119.0041 | 119.1442 |
| Deamidation of Asn and Gln | 0.9840 | 0.9847 |
| Deoxyhexoses (Fuc, Rha) | 146.0579 | 146.1430 |
| Disulphide bond formation | -2.0157 | -2.0159 |
| Farnesylation | 204.1878 | 204.3556 |
| Formylation | 27.9949 | 28.0104 |
| Geranylation | 272.2504 | 272.4741 |
| Glutathionylation | 305.0682 | 305.3117 |
| Hexosamines (GalN, GlcN) | 161.0688 | 161.1577 |
| Hexoses (Fru, Gal, Glc, Man) | 162.0528 | 162.1424 |
| Homoserine formed from Met by CNBr treatment | -29.9928 | -30.0935 |
| Hydroxylation | 15.9949 | 15.9994 |
| Lipoic acid (amide bond to lysine) | 188.0330 | 188.3147 |
| Methylation | 14.0157 | 14.0269 |
| Myristoylation | 210.1984 | 210.3598 |
| N-acetylhexosamines (GalNAc, GlcNAc) | 203.0794 | 203.1950 |
| N-acetylneuraminic acid (Sialic acid, NeuAc, NANA, SA) | 291.0954 | 291.2579 |
| N-glycolylneuraminic acid (NeuGc) | 307.0903 | 307.2573 |
| Oxidation of Met | 15.9949 | 15.9994 |
| Palmitoylation | 238.2297 | 238.4136 |
| Pentoses (Ara, Rib, Xyl) | 132.0423 | 132.1161 |
| Phosphorylation | 79.9663 | 79.9799 |
| Proteolysis of a single peptide bond | 18.0106 | 18.0153 |
| Pyridoxal phosphate (Schiff Base formed to lysine) | 231.0297 | 231.1449 |
| Pyroglutamic acid formed from Gln | -17.0265 | -17.0306 |
| Stearoylation | 266.2610 | 266.4674 |
| Sulphation | 79.9568 | 80.0642 |

**Fig. 9**

| Motif | Enzyme/ Reaction | Mass difference( average mass) |
| --- | --- | --- |
| W | photochemical | +16 |
| W | photochemical | +32 |
| W | photochemical | +4 |
| [ST]-X-[RK] | Protein kinase C | +79.9799 |

**Fig. 10**

| Symbols | | Monoisotopic Mass | Average Mass |
|---|---|---|---|
| Gly | G | 57.02146 | 57.05 |
| Ala | A | 71.03711 | 71.08 |
| Ser | S | 87.03202 | 87.08 |
| Pro | P | 97.05276 | 97.12 |
| Val | V | 99.06841 | 99.07 |
| Thr | T | 101.0476 | 101.1 |
| Cys | C | 103.0091 | 103.1 |
| Leu | L | 113.0840 | 113.2 |
| Ile | I | 113.0840 | 113.2 |
| Asn | N | 114.0429 | 114.1 |
| Asp | D | 115.0269 | 115.1 |
| Gln | Q | 128.0585 | 128.1 |
| Lys | K | 128.0949 | 128.2 |
| Glu | E | 129.0425 | 129.1 |
| Met | M | 131.0404 | 131.2 |
| His | H | 137.0589 | 137.1 |
| Phe | F | 147.0684 | 147.2 |
| Arg | R | 156.1011 | 156.2 |
| Tyr | Y | 163.0633 | 163.2 |
| Trp | W | 186.0793 | 186.2 |

**Fig. 11**

| | Composition | Monoisotopic Mass | Average Mass |
|---|---|---|---|
| **N-Terminal Groups** | | | |
| Hydrogen | H | 1.00782 | 1.0079 |
| N-Formyl | HCO | 29.00274 | 29.0183 |
| N-Acetyl | $CH_3CO$ | 43.01839 | 43.0452 |
| **C-Terminal Groups** | | | |
| Free acid | OH | 17.00274 | 17.0073 |

**Fig. 12**

| Additional Amino acid | +/- Fraction numbers |
|---|---|
| E, Glutamic acid | + 0.47 |
| F, Phenylalanine | + 5.54 |
| H, Histidine | - 3.82 |
| I, Isoleucine | + 2.86 |
| K, Lysine | - 1.72 |
| L, Leucine | + 5.15 |
| Q, Glutamine | - 0.85 |
| S , Serine | - 0.45 |
| V, Valine | + 2.2 |
| W, Tryptophane | + 5.35 |
| Y, Tyrosine | + 2.92 |

**Fig. 13**

| AminoAcid Before First Cleavage | | | | |
|---|---|---|---|---|
| | | % Cleavage | % Present | Δ |
| **A** | **24** | **15.5%** | **8.4%** | **184%** |
| **R** | **32** | **20.6%** | **6.3%** | **329%** |
| M | 4 | 2.6% | 1.5% | 171% |
| W | 3 | 1.9% | 1.2% | 168% |
| P | 14 | 9.0% | 6.2% | 145% |
| N | 7 | 4.5% | 3.3% | 135% |
| F | 6 | 3.9% | 3.2% | 122% |
| K | 10 | 6.5% | 6.0% | 107% |
| G | 11 | 7.1% | 6.6% | 107% |
| L | 12 | 7.7% | 8.5% | 91% |
| H | 3 | 1.9% | 2.7% | 71% |
| V | 5 | 3.2% | 5.3% | 61% |
| D | 5 | 3.2% | 5.5% | 58% |
| Q | 3 | 1.9% | 4.6% | 42% |
| C | 1 | 0.6% | 1.8% | 36% |
| I | 1 | 0.6% | 2.1% | 31% |
| T | 2 | 1.3% | 4.7% | 27% |
| **S** | **3** | **1.9%** | **7.8%** | **25%** |
| **E** | **4** | **2.6%** | **11.5%** | **22%** |
| **Y** | **0** | **0.0%** | **2.6%** | **0%** |
| PrecursorStart | 5 | 3.2% | | |

**Fig. 14a**

| AminoAcid after First Cleavage | | | | |
|---|---|---|---|---|
| | | % Cleavage | % Present | Δ |
| **S** | **26** | **16.8%** | **7.8%** | **214%** |
| **D** | **18** | **11.6%** | **5.5%** | **210%** |
| V | 13 | 8.4% | 5.3% | 158% |
| H | 6 | 3.9% | 2.7% | 143% |
| G | 14 | 9.0% | 6.6% | 136% |
| M | 3 | 1.9% | 1.5% | 128% |
| I | 4 | 2.6% | 2.1% | 124% |
| A | 16 | 10.3% | 8.4% | 122% |
| K | 9 | 5.8% | 6.0% | 97% |
| P | 9 | 5.8% | 6.2% | 93% |
| Q | 6 | 3.9% | 4.6% | 84% |
| L | 11 | 7.1% | 8.5% | 83% |
| T | 6 | 3.9% | 4.7% | 82% |
| N | 3 | 1.9% | 3.3% | 58% |
| Y | 2 | 1.3% | 2.6% | 50% |
| C | 1 | 0.6% | 1.8% | 36% |
| E | 5 | 3.2% | 11.5% | 28% |
| **R** | **2** | **1.3%** | **6.3%** | **21%** |
| **F** | **1** | **0.6%** | **3.2%** | **20%** |
| W | 0 | 0.0% | 1.2% | 0% |
| | | | | |

**Fig. 14b**

| AminoAcid Before Last Cleavage | | | | |
|---|---|---|---|---|
| | | % Cleavage | % Present | Δ |
| **R** | **26** | **16.8%** | **6.3%** | **267%** |
| E | 29 | 18.7% | 11.5% | 162% |
| N | 7 | 4.5% | 3.3% | 135% |
| D | 11 | 7.1% | 5.5% | 128% |
| A | 16 | 10.3% | 8.4% | 122% |
| Q | 8 | 5.2% | 4.6% | 111% |
| R | 10 | 6.5% | 6.3% | 103% |
| F | 5 | 3.2% | 3.2% | 102% |
| L | 13 | 8.4% | 8.5% | 98% |
| G | 10 | 6.5% | 6.6% | 97% |
| K | 9 | 5.8% | 6.0% | 97% |
| M | 2 | 1.3% | 1.5% | 85% |
| T | 6 | 3.9% | 4.7% | 82% |
| S | 9 | 5.8% | 7.8% | 74% |
| C | 2 | 1.3% | 1.8% | 72% |
| V | 5 | 3.2% | 5.3% | 61% |
| Y | 2 | 1.3% | 2.6% | 50% |
| H | 2 | 1.3% | 2.7% | 48% |
| **P** | **2** | **1.3%** | **6.2%** | **21%** |
| **I** | **0** | **0.0%** | **2.1%** | **0%** |

**Fig. 14c**

| AminoAcid After Last Cleavage | | | | |
|---|---|---|---|---|
| | | % Cleavage | % Present | Δ |
| **Precursor end** | **30** | **19.4%** | | |
| **R** | **26** | **16.8%** | **6.3%** | **267%** |
| **K** | **19** | **12.3%** | **6.0%** | **204%** |
| W | 3 | 1.9% | 1.2% | 168% |
| F | 8 | 5.2% | 3.2% | 162% |
| G | 12 | 7.7% | 6.6% | 117% |
| V | 9 | 5.8% | 5.3% | 110% |
| T | 7 | 4.5% | 4.7% | 96% |
| I | 2 | 1.3% | 2.1% | 62% |
| A | 7 | 4.5% | 8.4% | 54% |
| P | 5 | 3.2% | 6.2% | 52% |
| Y | 2 | 1.3% | 2.6% | 50% |
| M | 1 | 0.6% | 1.5% | 43% |
| Q | 3 | 1.9% | 4.6% | 42% |
| S | 5 | 3.2% | 7.8% | 41% |
| N | 2 | 1.3% | 3.3% | 39% |
| L | 5 | 3.2% | 8.5% | 38% |
| D | 3 | 1.9% | 5.5% | 35% |
| E | 6 | 3.9% | 11.5% | 34% |
| C | 0 | 0.0% | 1.8% | 0% |
| H | 0 | 0.0% | 2.7% | 0% |

**Fig. 14d**

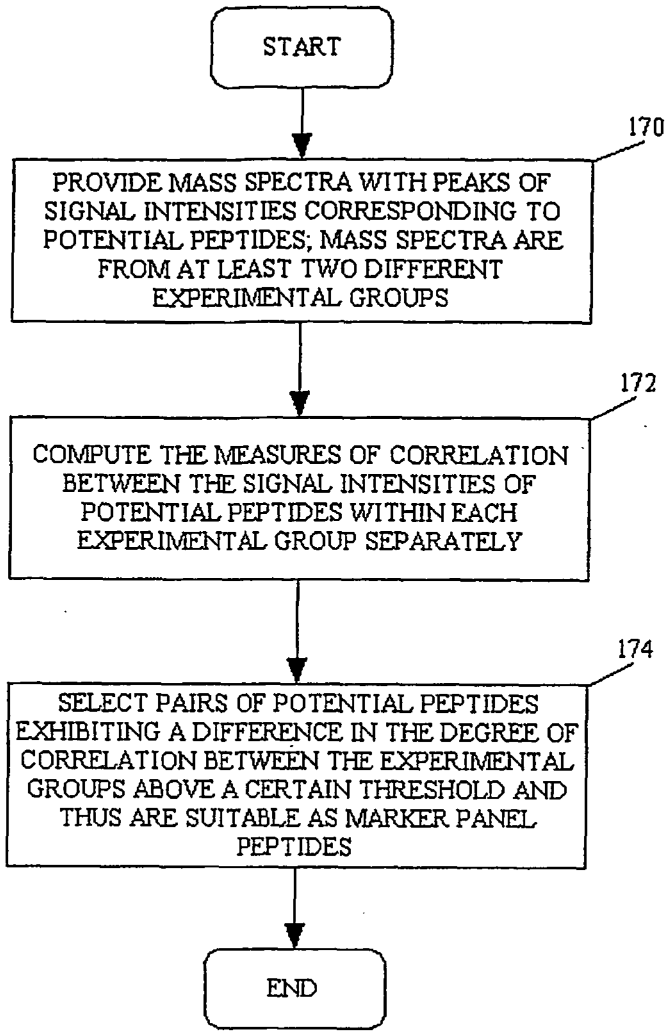
START
170
PROVIDE MASS SPECTRA WITH PEAKS OF SIGNAL INTENSITIES CORRESPONDING TO POTENTIAL PEPTIDES; MASS SPECTRA ARE FROM AT LEAST TWO DIFFERENT EXPERIMENTAL GROUPS
172
COMPUTE THE MEASURES OF CORRELATION BETWEEN THE SIGNAL INTENSITIES OF POTENTIAL PEPTIDES WITHIN EACH EXPERIMENTAL GROUP SEPARATELY
174
SELECT PAIRS OF POTENTIAL PEPTIDES EXHIBITING A DIFFERENCE IN THE DEGREE OF CORRELATION BETWEEN THE EXPERIMENTAL GROUPS ABOVE A CERTAIN THRESHOLD AND THUS ARE SUITABLE AS MARKER PANEL PEPTIDES
END

**Fig. 15**

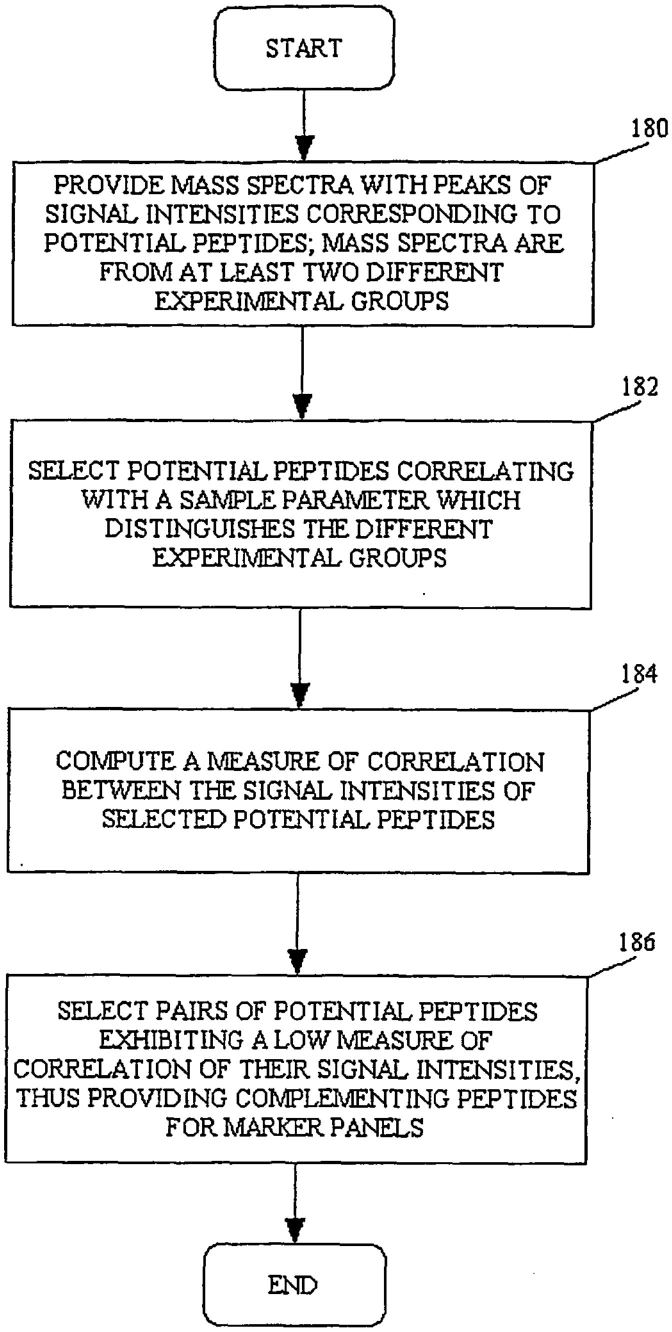
START
180
PROVIDE MASS SPECTRA WITH PEAKS OF SIGNAL INTENSITIES CORRESPONDING TO POTENTIAL PEPTIDES; MASS SPECTRA ARE FROM AT LEAST TWO DIFFERENT EXPERIMENTAL GROUPS
182
SELECT POTENTIAL PEPTIDES CORRELATING WITH A SAMPLE PARAMETER WHICH DISTINGUISHES THE DIFFERENT EXPERIMENTAL GROUPS
184
COMPUTE A MEASURE OF CORRELATION BETWEEN THE SIGNAL INTENSITIES OF SELECTED POTENTIAL PEPTIDES
186
SELECT PAIRS OF POTENTIAL PEPTIDES EXHIBITING A LOW MEASURE OF CORRELATION OF THEIR SIGNAL INTENSITIES, THUS PROVIDING COMPLEMENTING PEPTIDES FOR MARKER PANELS
END

**Fig. 16**

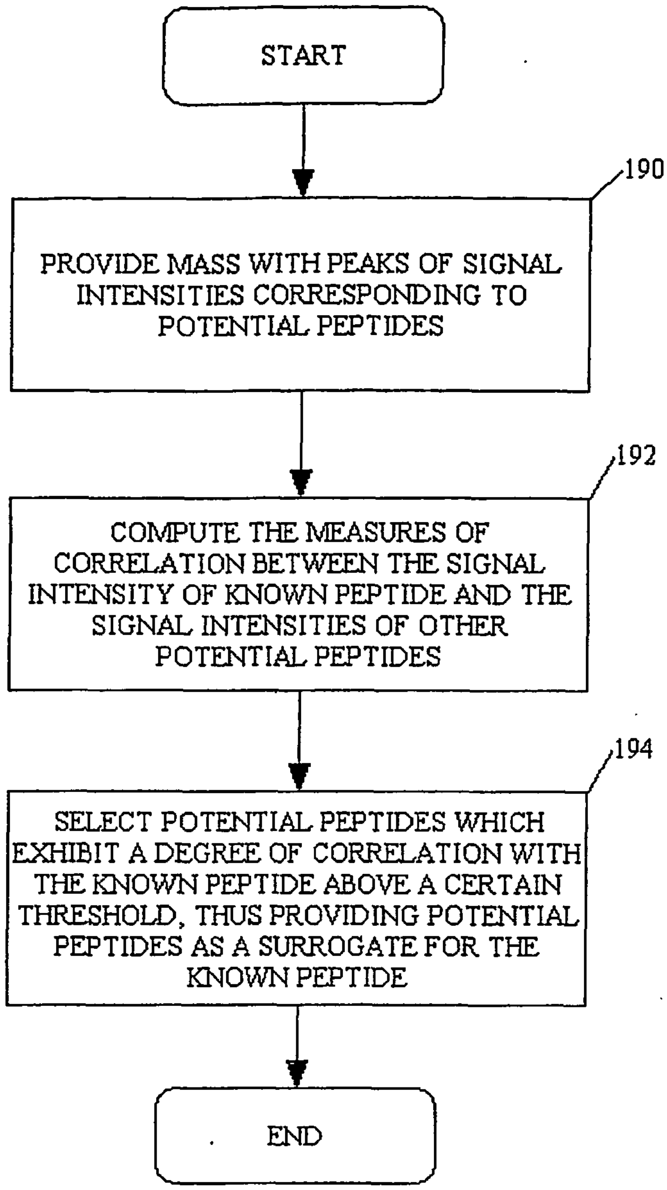
START
190
PROVIDE MASS WITH PEAKS OF SIGNAL INTENSITIES CORRESPONDING TO POTENTIAL PEPTIDES
192
COMPUTE THE MEASURES OF CORRELATION BETWEEN THE SIGNAL INTENSITY OF KNOWN PEPTIDE AND THE SIGNAL INTENSITIES OF OTHER POTENTIAL PEPTIDES
194
SELECT POTENTIAL PEPTIDES WHICH EXHIBIT A DEGREE OF CORRELATION WITH THE KNOWN PEPTIDE ABOVE A CERTAIN THRESHOLD, THUS PROVIDING POTENTIAL PEPTIDES AS A SURROGATE FOR THE KNOWN PEPTIDE
END

**Fig. 17**

| Case No. | Fraction 54 m/z 2743.0 | Fraction 54 m/z 1371.5 | Fraction 56 m/z 2927.2 | .... | Fraction 20 m/z 11143 | .... |
|---|---|---|---|---|---|---|
| | k=20 | k=19 | k=16 | | k=19 | |
| 1 | 21648 | 712 | 2620 | | 1452 | |
| 2 | 1830 | 1320 | 1199 | | 2554 | |
| 3 | 21353 | 2022 | 3159 | | 2139 | |
| 4 | 24223 | 2454 | 2431 | | 2169 | |
| 5 | 3725 | 2719 | 982 | | 1038 | |
| 6 | 13548 | 2956 | 1689 | | 1300 | |
| 7 | 16606 | 3260 | 1402 | | 2213 | |
| 8 | 20902 | 3435 | 624 | | 887 | |
| 9 | 10321 | 3444 | 427 | | 2145 | |
| 10 | 31047 | 3498 | 2238 | | 1516 | |
| 11 | 31142 | 3592 | 4405 | | 1255 | |
| 12 | 37241 | 3745 | 2785 | | 1739 | |
| 13 | 22656 | 3822 | 2264 | | 2576 | |
| 14 | 24366 | 3852 | 1752 | | 1139 | |
| 15 | 16638 | 3935 | 1147 | | 2182 | |
| 16 | 37171 | 4092 | 2393 | | 1069 | |
| 17 | 33188 | 4115 | 1578 | | 1681 | |
| 18 | 27596 | 4127 | 3228 | | 1463 | |
| 19 | 39668 | 4348 | 3604 | | 318 | |
| 20 | 12983 | 4362 | 1048 | | 3039 | |
| 21 | 14420 | 4488 | 899 | | 2676 | |
| 22 | 23261 | 4634 | 1965 | | 3071 | |
| 23 | 30507 | 4710 | 2715 | | 1247 | |
| 24 | 41494 | 4928 | 5343 | | 1238 | |
| 25 | 36664 | 5107 | 3914 | | 3156 | |
| 26 | 42465 | 5135 | 2729 | | 1768 | |
| 27 | 42551 | 5135 | 3010 | | 2500 | |
| 28 | 35473 | 5201 | 2242 | | 1978 | |
| 29 | 48611 | 5906 | 2381 | | 1075 | |
| 30 | 28413 | 5914 | 1855 | | 3189 | |
| 31 | 35258 | 5954 | 3368 | | 2140 | |
| 32 | 44774 | 6056 | 4167 | | 670 | |
| 33 | 46137 | 6465 | 7640 | | 1719 | |
| 34 | 40892 | 6531 | 1630 | | 1241 | |
| 35 | 48202 | 7076 | 11222 | | 3826 | |
| 36 | 43760 | 7183 | 4771 | | 1565 | |
| 37 | 50211 | 7316 | 5443 | | 2060 | |

Fig. 18a

| Case No. | Fraction 54 m/z 2743.0 | Fraction 54 m/z 1371.5 | Fraction 56 m/z 2927.2 | ... | Fraction 20 m/z 1114.3 | ... |
|---|---|---|---|---|---|---|
| 38 | 49824 | 7410 | 3004 | | 1113 | |
| 39 | 50785 | 7752 | 6412 | | 1616 | |
| 40 | 46200 | 7821 | 3689 | | 3725 | |
| 41 | 52471 | 7949 | 5395 | | 1837 | |
| 42 | 49299 | 8280 | 4623 | | 1207 | |
| 43 | 45032 | 8483 | 4881 | | 1566 | |
| 44 | 51224 | 8562 | 6481 | | 2194 | |
| 45 | 51901 | 8638 | 10081 | | 2047 | |
| 46 | 51084 | 8776 | 14193 | | 1478 | |
| 47 | 50928 | 8852 | 6635 | | 287 | |
| 48 | 50707 | 10097 | 8877 | | 1458 | |
| 49 | 52304 | 10259 | 6244 | | 1860 | |
| 50 | 48355 | 10661 | 5195 | | 3695 | |
| 51 | 51363 | 10685 | 11403 | | 1261 | |
| 52 | 54423 | 10846 | 11299 | | 2067 | |
| 53 | 55167 | 11041 | 12868 | | 1545 | |
| 54 | 55091 | 11539 | 5597 | | 2381 | |
| 55 | 56825 | 11912 | 7718 | | 2409 | |
| 56 | 53173 | 12022 | 8865 | | 1969 | |
| 57 | 51649 | 12057 | 7855 | | 1295 | |
| 58 | 51328 | 12095 | 9035 | | 2043 | |
| 59 | 53464 | 12641 | 6408 | | 856 | |
| 60 | 54542 | 12891 | 10363 | | 1858 | |
| 61 | 56950 | 13172 | 7586 | | 1802 | |
| 62 | 43273 | 14559 | 20080 | | 596 | |
| 63 | 57335 | 14922 | 12288 | | 2916 | |
| 64 | 55118 | 14997 | 10078 | | 1761 | |
| 65 | 57147 | 16164 | 7726 | | 2626 | |
| 66 | 55584 | 16216 | 17106 | | 2623 | |
| 67 | 59414 | 16550 | 15122 | | 539 | |
| 68 | 57093 | 16689 | 19689 | | 2078 | |
| 69 | 57841 | 18254 | 16079 | | 1659 | |
| 70 | 54084 | 18734 | 19524 | | 395 | |
| 71 | 56325 | 22730 | 10828 | | 2326 | |
| 72 | 58386 | 24159 | 16681 | | 1631 | |
| 73 | 54843 | 26671 | 44356 | | 3183 | |
| 74 | 53935 | 27937 | 30189 | | 1403 | |

**Fig. 18b**

| | Fraction 54 m/z 2743.0 and ... | | |
|---|---|---|---|
| Measure of Association | Fraction 54 m/z 1371.5 | Fraction 56 m/ 2927.2 | Fraction 20 m/z 1114.3 |
| Spearman's rank order correlation | 0.9298 | 0.8761 | -0.0044 |
| Pearson's product moment correlation | 0.7318 | 0.5855 | -0.0781 |
| Kendall's rank correlation tau | 0.7704 | 0.6919 | 0.0107 |
| MST diameter | 50 | 40 | 29 |

**Fig. 19**

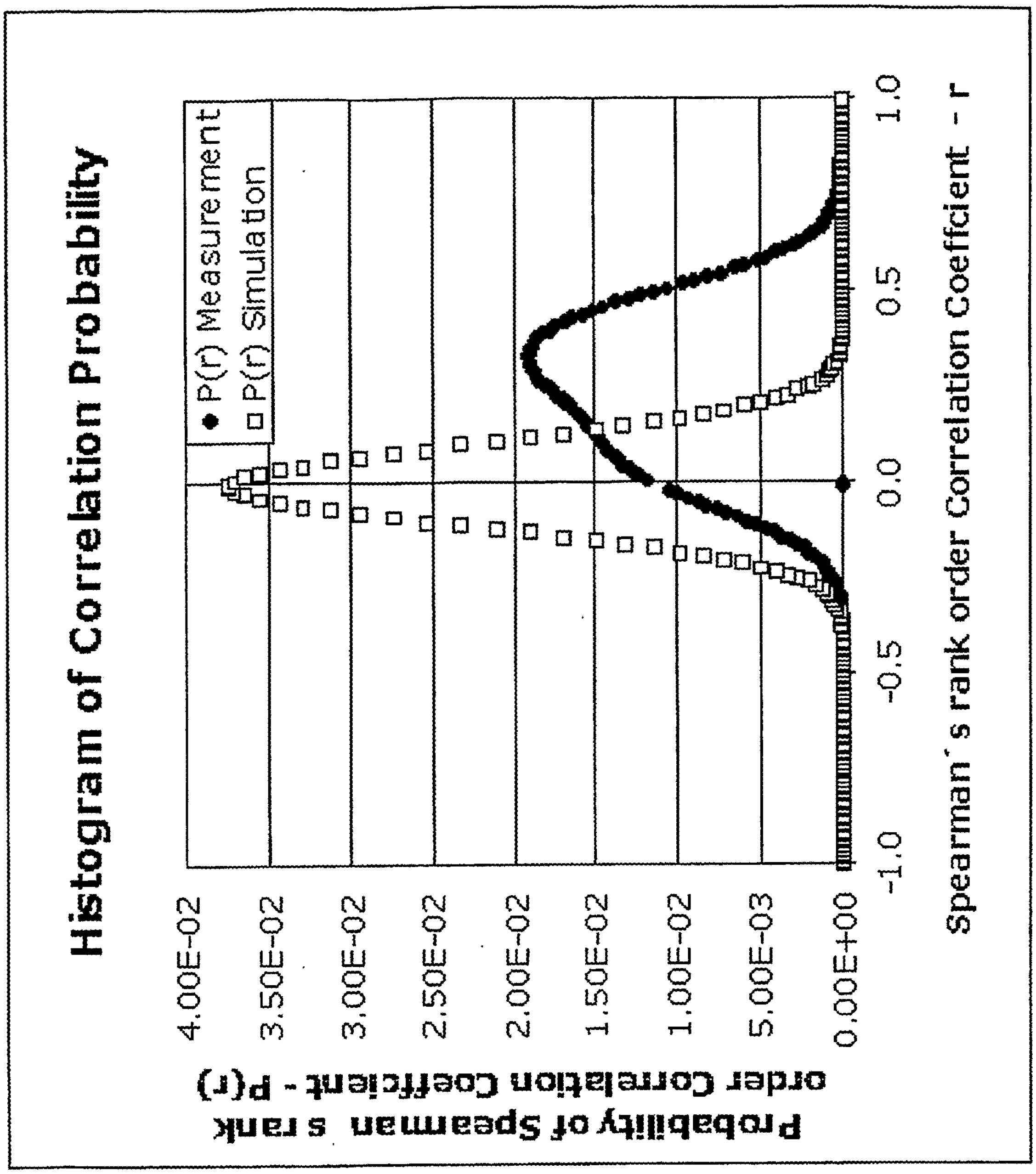

Histogram of Correlation Probability
P(r) Measurement
P(r) Simulation
4.00E-02
3.50E-02
3.00E-02
2.50E-02
2.00E-02
1.50E-02
1.00E-02
5.00E-03
0.00E+00
-1.0
-0.5
0.0
0.5
1.0
Spearman´s rank order Correlation Coeffcient - r
Probability of Spearman´s rank order Correlation Coefficient - P(r)


Fig. 20

| Hub - Peptide | Corre-lation | Related peptide | Relative Mono-isotopic mass [Da] | Amino Acid Sequence |
|---|---|---|---|---|
| **Chromogranin A 97 - 131** | | | 3905.764 | HSGF EDELSEVLEN QSSQAELKEA VEEPSSKDVM E |
| | r=0.67 | Secretogranin I 88-132 | 4605.025 | DPADASEAHESSSRGEAGAP GEEDIQGPTKADTEKWAEGG GHSRE |
| | r=0.71 | Secretogranin II 529-566 | 4152.921 | G QGSSEDDLQEE EQIEQAIKEH LNQGSSQETD KLAPVS |
| | r=0.72 | Secretogranin V 181-202 | 2448.334 | SVNPYLQGQRLDNVVAKKSV PH |

**Fig. 21**

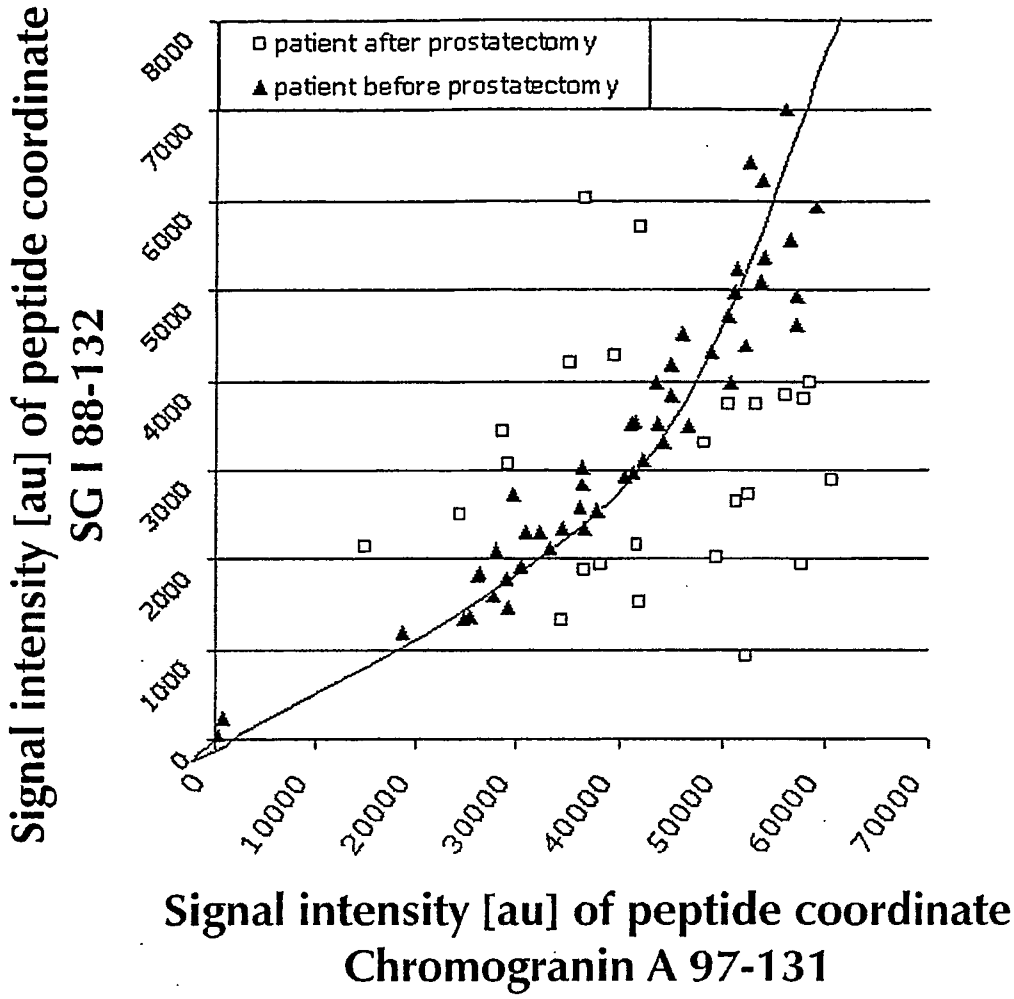
patient after prostatectomy
patient before prostatectomy
Signal intensity [au] of peptide coordinate SG I 88-132
Signal intensity [au] of peptide coordinate Chromogranin A 97-131
0
1000
2000
3000
4000
5000
6000
7000
8000
10000
20000
30000
40000
50000
60000
70000

Fig. 22

| | Fraction 54 m/z ~~2743.0~~ | Fraction 54 m/z 1371.5 | Fraction 56 m/z 2927.2 | .... | Fraction 20 m/z 1114.3 | .... |
|---|---|---|---|---|---|---|
| | ~~k=0~~ | k=1 | k=2 | | k=19 | |
| case 1 | ~~21648~~ | 3747 | 7051 | | 1452 | |
| case 2 | ~~1830~~ | 4355 | 5630 | | 2554 | |
| case 3 | ~~21353~~ | 5057 | 7590 | | 2139 | |
| case 4 | ~~24223~~ | 5489 | 6862 | | 2169 | |
| case 5 | ~~3725~~ | 5754 | 5413 | | 1038 | |
| case 6 | ~~13548~~ | 5991 | 6120 | | 1300 | |
| case 7 | ~~16606~~ | 6295 | 5833 | | 2213 | |
| case 8 | ~~20902~~ | 6470 | 5055 | | 887 | |
| case 9 | ~~10321~~ | 6479 | 4858 | | 2145 | |
| case 10 | ~~31047~~ | 6533 | 6669 | | 1516 | |
| case 11 | ~~31142~~ | 6627 | 8836 | | 1255 | |
| case 12 | ~~37241~~ | 6780 | 7216 | | 1739 | |
| case 13 | ~~22656~~ | 6857 | 6695 | | 2576 | |
| case 14 | ~~24366~~ | 6887 | 6183 | | 1139 | |
| case 15 | ~~16638~~ | 6970 | 5578 | | 2182 | |
| case 16 | ~~37171~~ | 7127 | 6824 | | 1069 | |
| case 17 | ~~33188~~ | 7150 | 6009 | | 1681 | |
| case 18 | ~~27596~~ | 7162 | 7659 | | 1463 | |
| case 19 | ~~39668~~ | 7383 | 8035 | | 318 | |
| case 20 | ~~12983~~ | 7397 | 5479 | | 3039 | |
| case 21 | ~~14420~~ | 7523 | 5330 | | 2676 | |
| case 22 | ~~23261~~ | 7669 | 6396 | | 3071 | |
| case 23 | ~~30507~~ | 7745 | 7146 | | 1247 | |
| case 24 | ~~41494~~ | 7963 | 9774 | | 1238 | |
| case 25 | ~~36664~~ | 8142 | 8345 | | 3156 | |
| case 26 | ~~42465~~ | 8170 | 7160 | | 1768 | |
| case 27 | ~~42551~~ | 8170 | 7441 | | 2500 | |
| case 28 | ~~35473~~ | 8236 | 6673 | | 1978 | |
| case 29 | ~~48611~~ | 8941 | 6812 | | 1075 | |
| case 30 | ~~28413~~ | 8949 | 6286 | | 3189 | |
| case 31 | ~~35258~~ | 8989 | 7799 | | 2140 | |
| case 32 | ~~44774~~ | 9091 | 8598 | | 670 | |
| case 33 | ~~46137~~ | 9500 | 12071 | | 1719 | |
| case 34 | ~~40892~~ | 9566 | 6061 | | 1241 | |
| case 35 | ~~48202~~ | 10111 | 15653 | | 3826 | |
| case 36 | ~~43760~~ | 10218 | 9202 | | 1565 | |

Fig. 23a

| | Fraction 54 m/z 2743.0 | Fraction 54 m/z 1371.5 | Fraction 56 m/z 2927.2 | .... | Fraction 20 m/z 1114.3 | .... |
|---|---|---|---|---|---|---|
| case 37 | 50211 | 10351 | 9874 | | 1113 | |
| case 38 | 49824 | 10445 | 7435 | | 1616 | |
| case 39 | 50785 | 10787 | 10843 | | 3725 | |
| case 40 | 46288 | 10856 | 8120 | | 1837 | |
| case 41 | 52471 | 10984 | 9826 | | 1207 | |
| case 42 | 49299 | 11315 | 9054 | | 1566 | |
| case 43 | 45082 | 11518 | 9312 | | 2194 | |
| case 44 | 51224 | 11597 | 10912 | | 2047 | |
| case 45 | 51981 | 11673 | 14512 | | 1478 | |
| case 46 | 51084 | 11811 | 18624 | | 287 | |
| case 47 | 50928 | 11887 | 11066 | | 1458 | |
| case 48 | 50707 | 13132 | 13308 | | 1860 | |
| case 49 | 52304 | 13294 | 10675 | | 3695 | |
| case 50 | 48255 | 13696 | 9626 | | 1261 | |
| case 51 | 51363 | 13720 | 15834 | | 2067 | |
| case 52 | 54423 | 13881 | 15730 | | 1545 | |
| case 53 | 55167 | 14076 | 17299 | | 2381 | |
| case 54 | 55091 | 14574 | 10028 | | 2409 | |
| case 55 | 56825 | 14947 | 12149 | | 1969 | |
| case 56 | 53173 | 15057 | 13296 | | 1295 | |
| case 57 | 51649 | 15092 | 12286 | | 2043 | |
| case 58 | 51328 | 15130 | 13466 | | 856 | |
| case 59 | 53464 | 15676 | 10839 | | 1858 | |
| case 60 | 54542 | 15926 | 14794 | | 1802 | |
| case 61 | 56950 | 16207 | 12017 | | 596 | |
| case 62 | 43273 | 17594 | 24511 | | 2916 | |
| case 63 | 57335 | 17957 | 16719 | | 1761 | |
| case 64 | 55118 | 18032 | 14509 | | 2626 | |
| case 65 | 57147 | 19199 | 12157 | | 2623 | |
| case 66 | 55584 | 19251 | 21537 | | 539 | |
| case 67 | 59414 | 19585 | 19553 | | 2078 | |
| case 68 | 57093 | 19724 | 24120 | | 1659 | |
| case 69 | 57841 | 21289 | 20510 | | 395 | |
| case 70 | 54084 | 21769 | 23955 | | 2326 | |
| case 71 | 56325 | 25765 | 15259 | | 1631 | |
| case 72 | 58386 | 27194 | 21112 | | 3183 | |
| case 73 | 54843 | 29706 | 48787 | | 1403 | |
| case 74 | 53935 | 30972 | 34620 | | 1113 | |

**Fig. 23b**

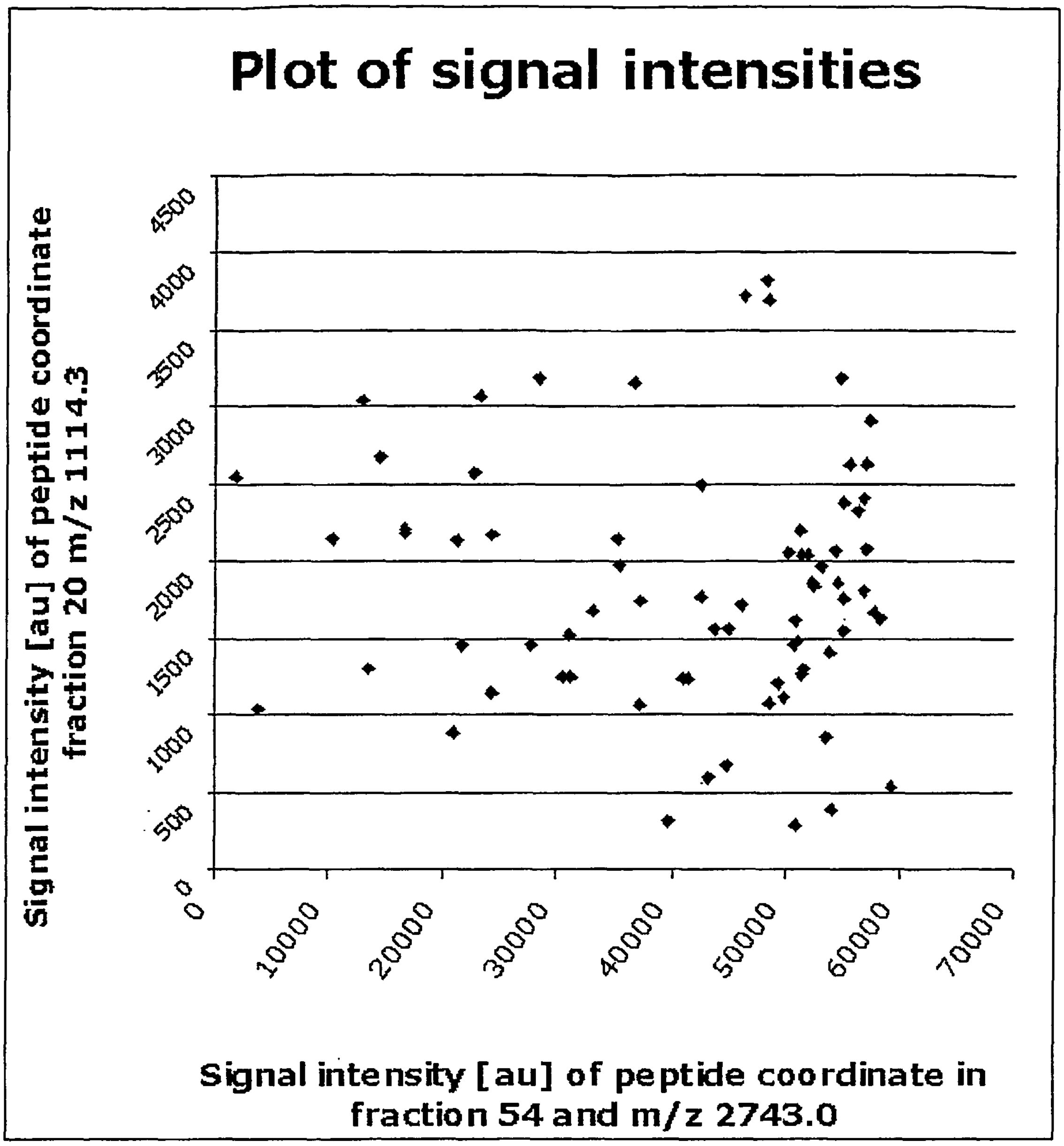
Plot of signal intensities
Signal intensity [au] of peptide coordinate fraction 20 m/z 1114.3
0
500
1000
1500
2000
2500
3000
3500
4000
4500
0
10000
20000
30000
40000
50000
60000
70000
Signal intensity [au] of peptide coordinate in fraction 54 and m/z 2743.0

Fig. 24a

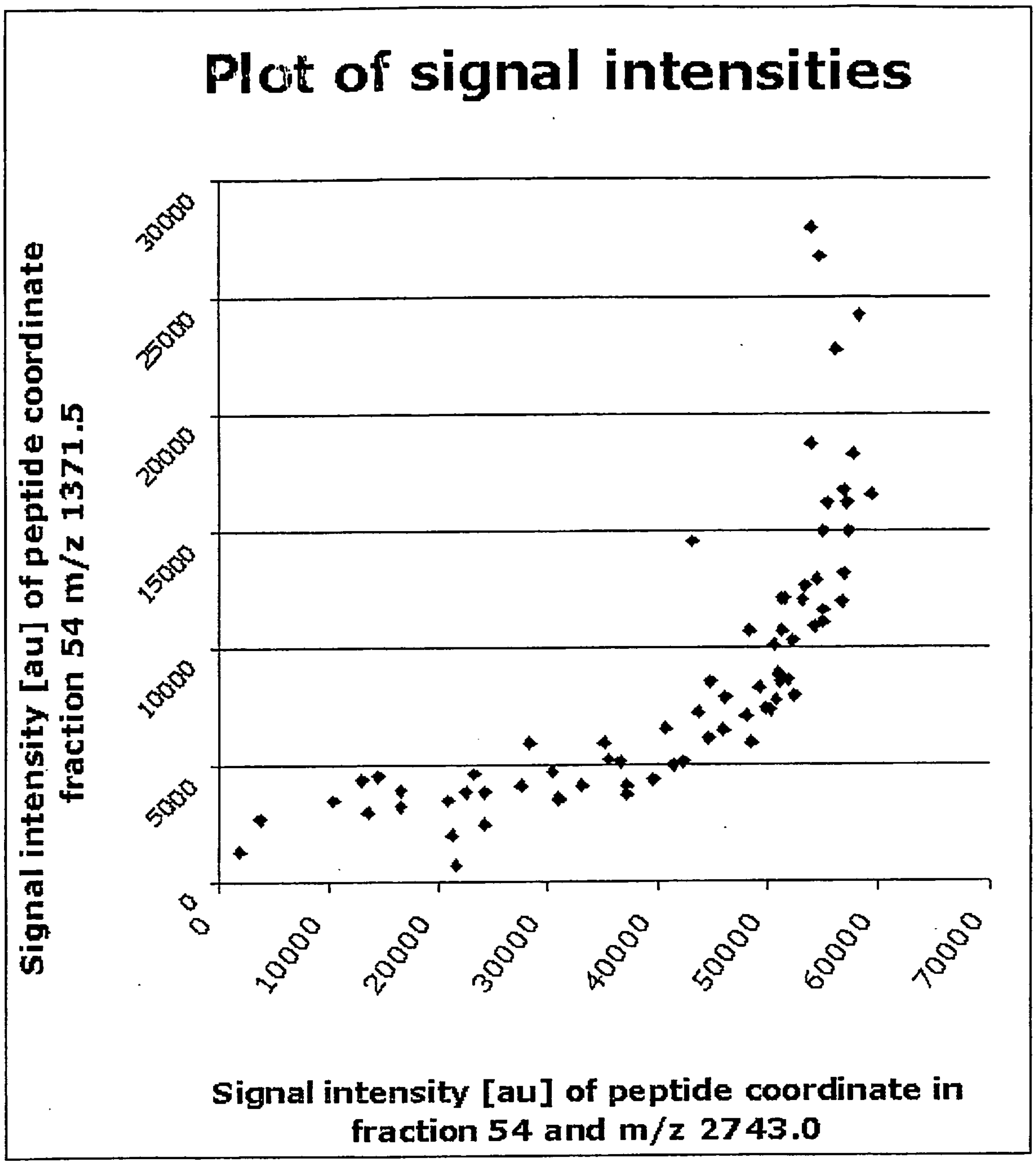
Plot of signal intensities
Signal intensity [au] of peptide coordinate fraction 54 m/z 1371.5
0
5000
10000
15000
20000
25000
30000
0
10000
20000
30000
40000
50000
60000
70000
Signal intensity [au] of peptide coordinate in fraction 54 and m/z 2743.0

Fig. 24b

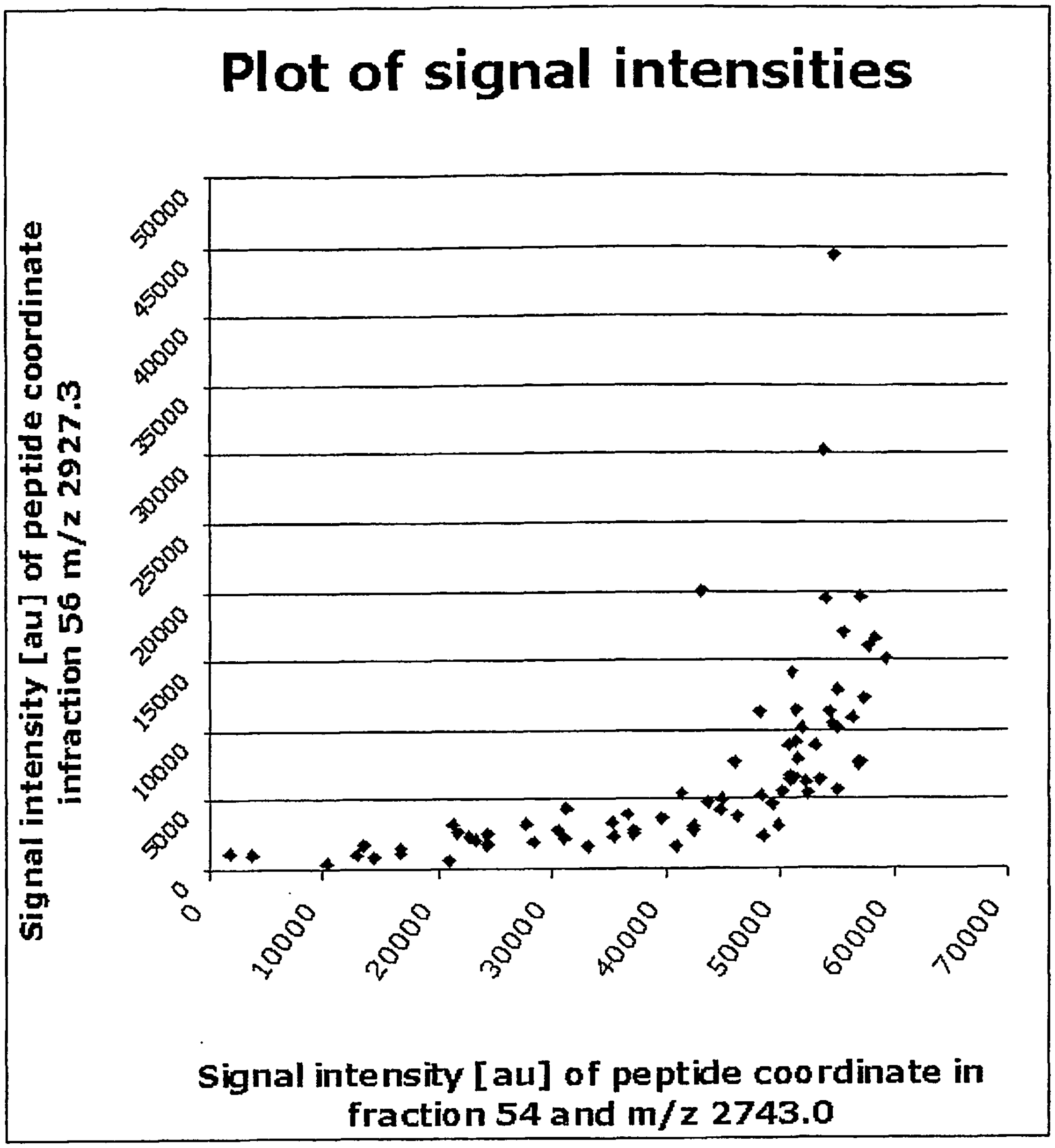
Plot of signal intensities
Signal intensity [au] of peptide coordinate infraction 56 m/z 2927.3
0
5000
10000
15000
20000
25000
30000
35000
40000
45000
50000
0
10000
20000
30000
40000
50000
60000
70000
Signal intensity [au] of peptide coordinate in fraction 54 and m/z 2743.0

Fig. 24c

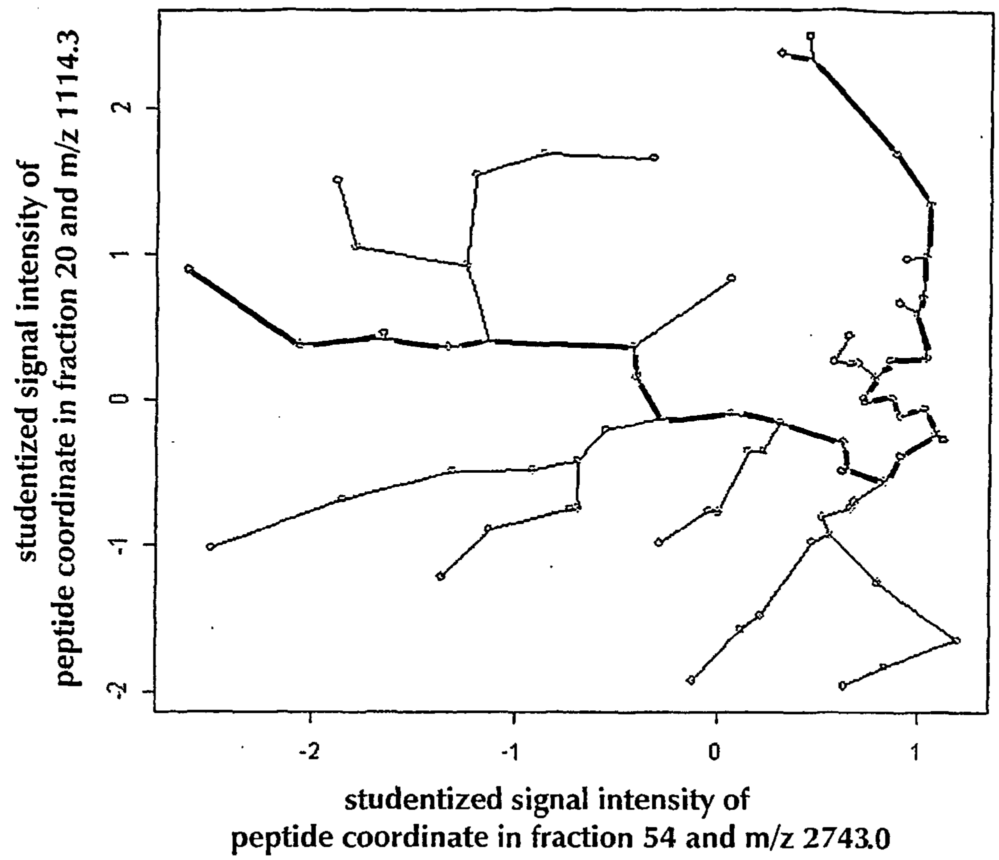
studentized signal intensity of
peptide coordinate in fraction 20 and m/z 1114.3
2
1
0
-1
-2
-2
-1
0
1
studentized signal intensity of
peptide coordinate in fraction 54 and m/z 2743.0

Fig. 25a

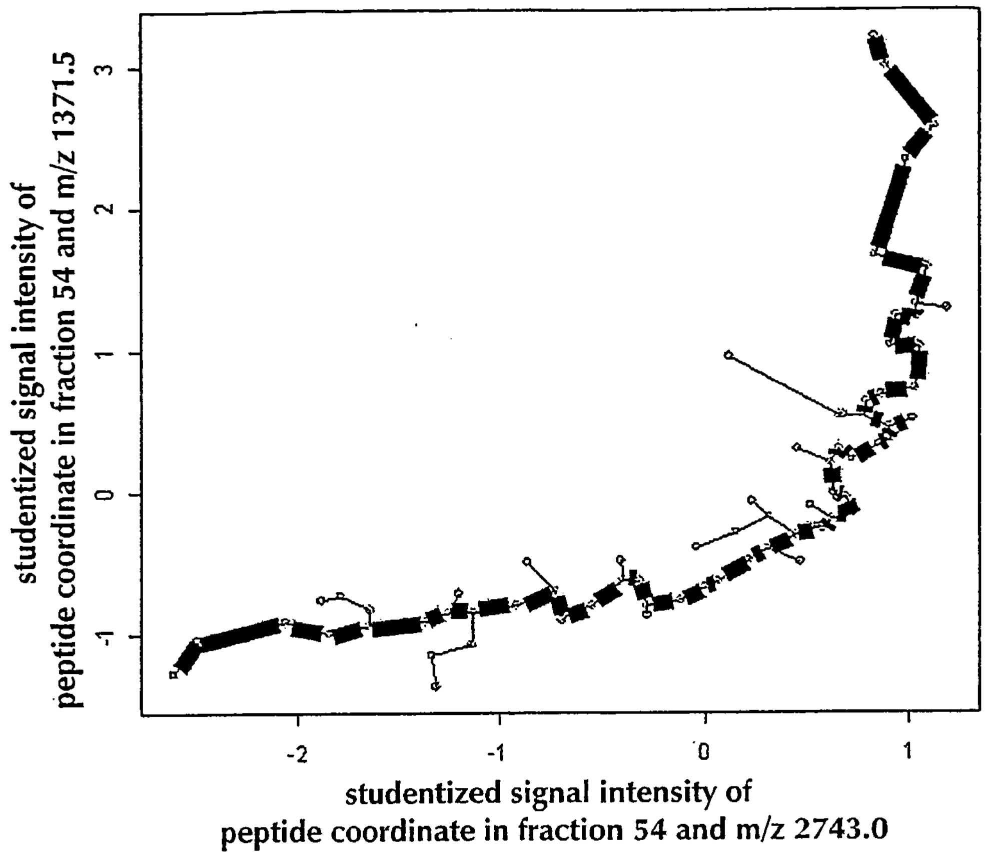
studentized signal intensity of
peptide coordinate in fraction 54 and m/z 1371.5
3
2
1
0
-1
-2
-1
0
1
studentized signal intensity of
peptide coordinate in fraction 54 and m/z 2743.0

Fig. 25b

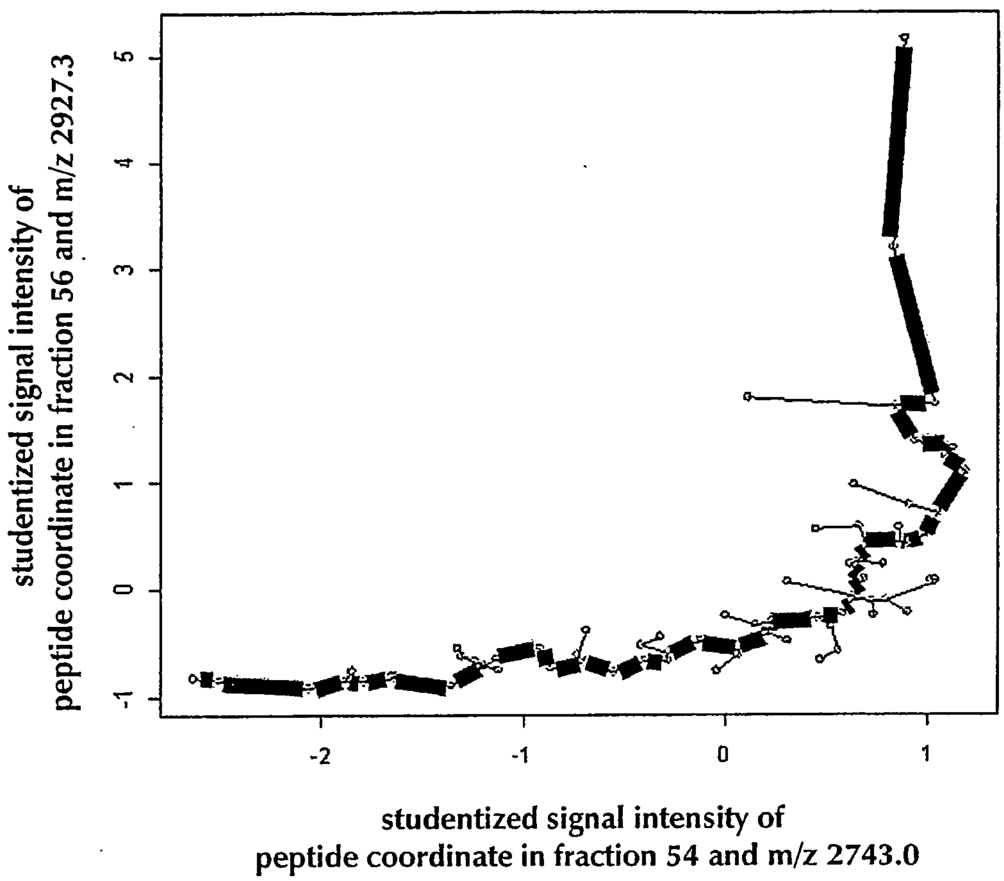
studentized signal intensity of
peptide coordinate in fraction 56 and m/z 2927.3
-1
0
1
2
3
4
5
-2
-1
0
1
studentized signal intensity of
peptide coordinate in fraction 54 and m/z 2743.0

Fig. 25c

| | n | | | |
|---|---|---|---|---|
| m/z | 1 | 2 | 3 | 4 |
| **1371.5** | -1371.5 | 0.0 | 1371.5 | 2743.0 |
| **2927.3** | 183.3 | 3109.6 | 6035.9 | 8962.2 |

**Fig. 26**

```
1         10         20         30         40
|         |          |          |          |
MKWVTFISLL FLFSSAYSRG VFRRDAHKSE VAHRTYDLGE

          50         60         70         80
          |          |          |          |
ENFKALVLIA KREAQYLQQC PFEDHVKLVN EVTEFAKTCV

          90         100        110        120
          |          |          |          |
ADESAENCDK SLHTLFGDKL CTVATLRETY GEMADCCAKQ

          130        140        150        160
          |          |          |          |
EPERNECFLQ HKDDNPNLPR LVRPEVDVMC TAFHDNEETF

          170        180        190        200
          |          |          |          |
LKKYLYEIAR RHPYFYAPEL LFFAKRYKAA FTECCQAADK

          210        220        230      238
          |          |          |        |
AACLLPKLDE LRDEGKASSA KQRLKCASLQ KFGERAFK
```

**Fig. 27**

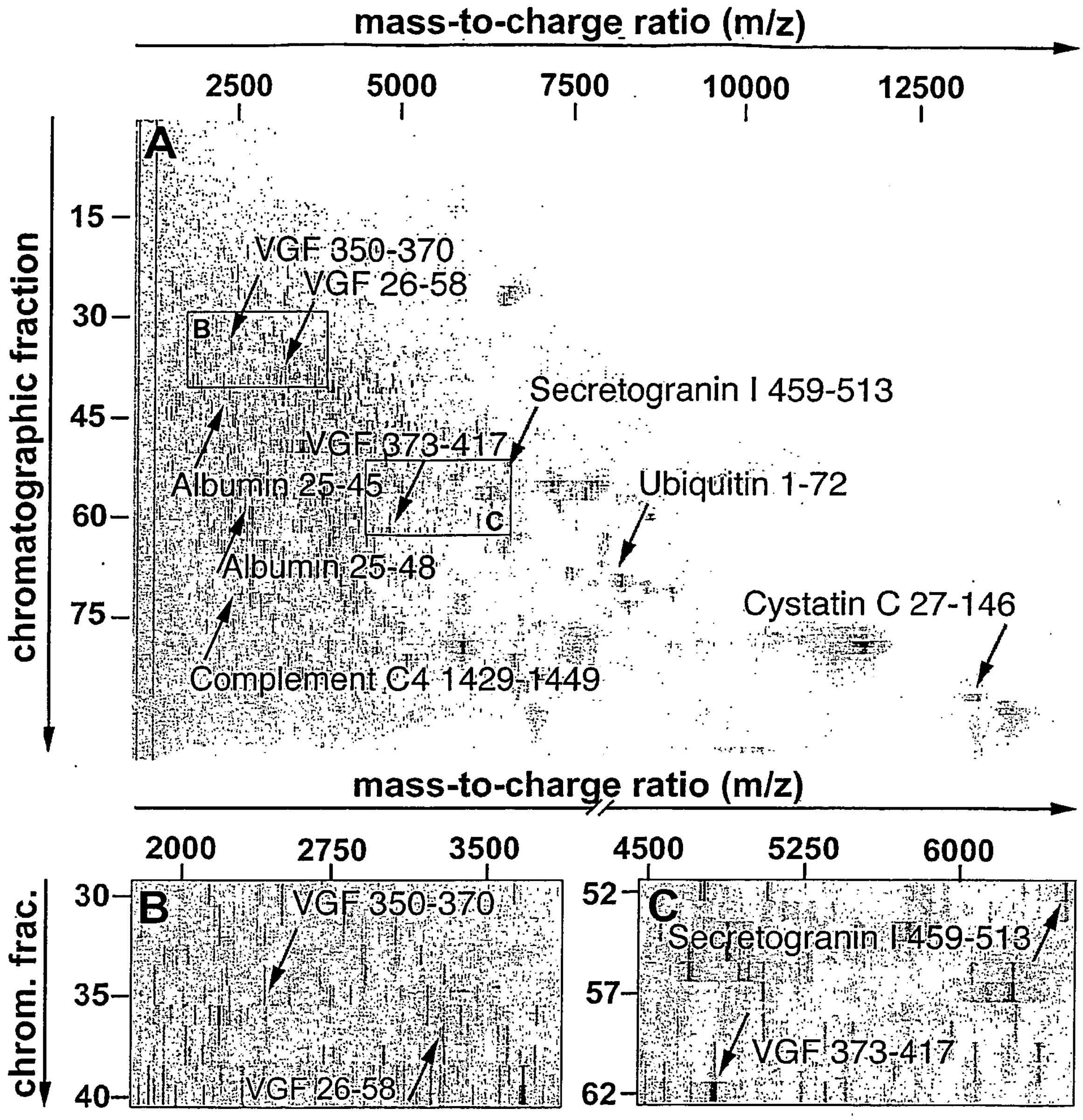
mass-to-charge ratio (m/z)
2500
5000
7500
10000
12500
A
chromatographic fraction
15
30
45
60
75
VGF 350-370
VGF 26-58
B
Secretogranin I 459-513
VGF 373-417
Albumin 25-45
C
Ubiquitin 1-72
Albumin 25-48
Cystatin C 27-146
Complement C4 1429-1449
mass-to-charge ratio (m/z)
2000
2750
3500
4500
5250
6000
chrom. frac.
30
35
40
B
VGF 350-370
VGF 26-58
52
57
62
C
Secretogranin I 459-513
VGF 373-417

Fig. 28

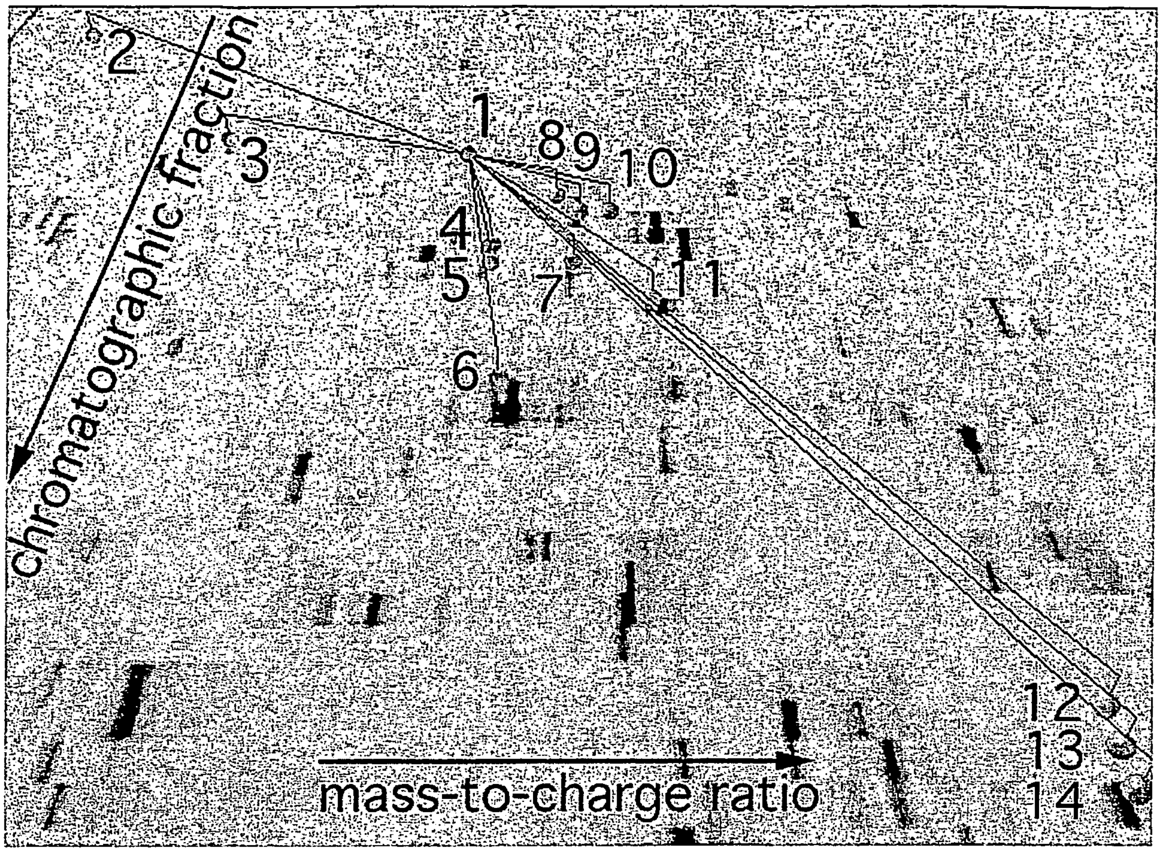
2
chromatographic fraction
3
1
8
9
10
4
5
7
11
6
12
13
14
mass-to-charge ratio

Fig. 29

```
  1 MKALRLSASA LFCLLLINGL GAAPPGRPEA QPPPLSSEHK EPVAGDAVPG PKDGSAPEVR GARNSEPQDE GELFQGVDPR
                                  # 1 VGF 26-58, Hub Peptide
                                  # 4 VGF 26-59, r=0.83
                                  # 5 VGF 26-59, r=0.69
                                  # 7 VGF 23-59, r=0.69
                                  # 8 VGF 26-61, r=0.73
                                  # 9 VGF 26-62, r=0.68
                                  #10 VGF 25-62, r=0.69

 81 ALAAVLLQAL DRPASPPAPS GSQQGPEEEA AEALLTETVR SQTHSLPAPE SPEPAAPPRP QTPENGPEAS DPSEELEALA
161 SLLQELRDFS PSSAKRQQET AAAETETRTH TLTRVNLESP GPERVWRASW GEFQARVPER APLPPPAPSQ FQARMPDSGP
                                    # 2 VGF 177-191, r=0.74

241 LPETHKFGEG VSSPKTHLGE ALAPLSKAYQ GVAAPFPKAR RPESALLGGS EAGERLLQQG LAQVEAGRRQ AEATRQAAAQ
321 EERLADLASD LLLQYLLQGG ARQRGLGGRG LQEAAEERES AREEEEAEQE RRGGEERVGE EDEEAAEAEA EAEEAERARQ
          # 3 VGF 350-370, r=0.76                            #12 VGF 373-417, r=0.73
                                                             #13 VGF 373-417, r=0.68
                                                             #14 VGF 373-417, r=0.76

401 NALLFAEEED GEAGAEDKRS QEETPGHRRK EAEGTEEGGE EEDDEEMDPQ TIDSLIELST KLHLPADDVV SIIEEVEEKR

481 KRKKNAPPEP VPPPRAAPAP THVRSPQPPP PAPAPARDEL PDWNEVLPPW DREEDEVYPP GPYHPFPNYI RPRTLQPPSA
                #12 VGF 485-522, r=0.81

561 LRRRHYHHAL PPSRHYPGRE AQARRAQEEA EAEERRLQEQ EELENYIEHV LLRRP
```

Fig. 30

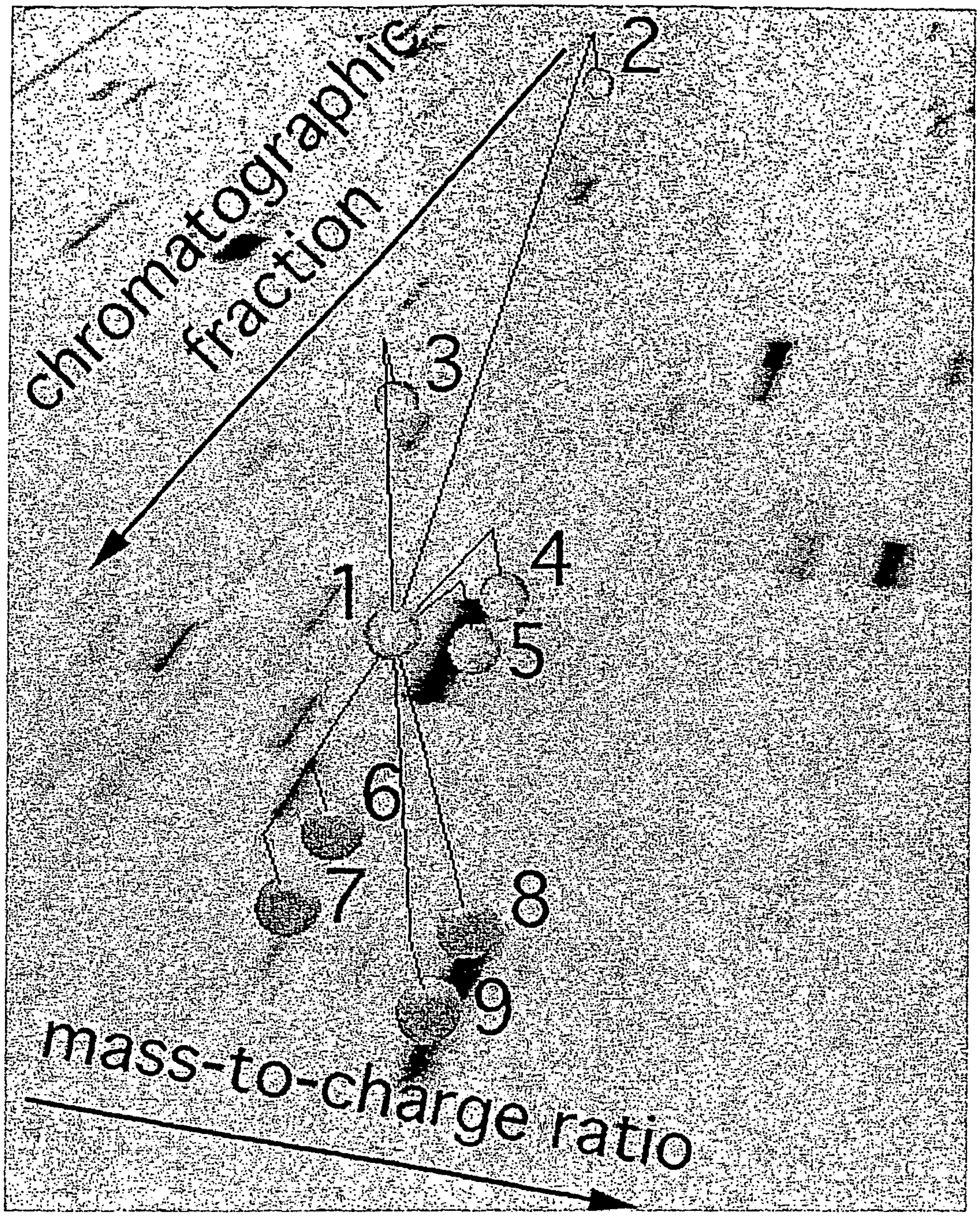


Fig. 31

| \|r\| ≥ | Number of ... Correct Precursor Predictions | False Precursor Predictions | All Predictions | Percentage of Correct Predictions |
|---|---|---|---|---|
| **0.95** | 18 | 0 | 18 | 100% |
| **0.90** | 40 | 4 | 44 | 91% |
| **0.85** | 58 | 10 | 68 | 85% |
| **0.80** | 104 | 26 | 130 | 80% |
| **0.75** | 178 | 76 | 256 | 70% |
| **0.70** | 314 | 192 | 506 | 62% |
| **0.65** | 512 | 456 | 968 | 53% |
| **0.60** | 756 | 990 | 1746 | 43% |
| **0.55** | 964 | 1872 | 2836 | 34% |
| **0.50** | 1186 | 3086 | 4272 | 28% |

Fig. 32

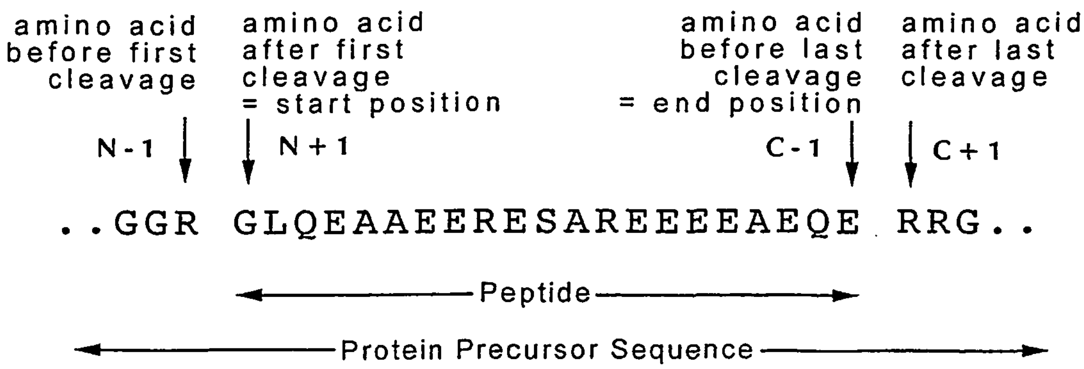
amino acid before first cleavage
amino acid after first cleavage = start position
amino acid before last cleavage = end position
amino acid after last cleavage
N-1
N+1
C-1
C+1
..GGR GLQEAAEERESAREEEEAEQE RRG..
Peptide
Protein Precursor Sequence

Fig. 33

| | Number and Percentage of Amino Acid Found at Position of Interest | | | | | | | | | | x-fold Change of Percentages of Amino Acid Compared to General Occurrence | | | |
|---|---|---|---|---|---|---|---|---|---|---|---|---|---|---|
| Amino Acid | n(N-1) | | n(N+1) | | n(C-1) | | n(C+1) | | n (Any position) | | x(N-1) | x(N+1) | x(C-1) | x(C+1) |
| A | 16 | 12% | 18 | 13% | 16 | 12% | 5 | 5% | 5612 | 8.2% | 1.4 | 1.6 | 1.4 | 0.6 |
| C | 1 | 1% | 1 | 1% | 2 | 1% | 0 | 0% | 1109 | 1.6% | 0.4 | 0.4 | 0.9 | 0.0 |
| D | 5 | 4% | 22 | 16% | 8 | 6% | 4 | 4% | 3994 | 5.8% | 0.6 | 2.8 | 1.0 | 0.6 |
| E | 4 | 3% | 5 | 4% | 30 | 22% | 3 | 3% | 8069 | 11.8% | 0.2 | 0.3 | 1.8 | 0.2 |
| F | 5 | 4% | 4 | 3% | 3 | 2% | 7 | 7% | 2195 | 3.2% | 1.1 | 0.9 | 0.7 | 2.0 |
| G | 6 | 4% | 12 | 9% | 7 | 5% | 7 | 7% | 4171 | 6.1% | 0.7 | 1.4 | 0.8 | 1.1 |
| H | 3 | 2% | 4 | 3% | 2 | 1% | 1 | 1% | 1955 | 2.9% | 0.8 | 1.0 | 0.5 | 0.3 |
| I | 0 | 0% | 1 | 1% | 3 | 2% | 2 | 2% | 1592 | 2.3% | 0.0 | 0.3 | 0.9 | 0.8 |
| K | 7 | 5% | 8 | 6% | 12 | 9% | 22 | 21% | 4235 | 6.2% | 0.8 | 0.9 | 1.4 | 3.4 |
| L | 9 | 7% | 11 | 8% | 10 | 7% | 8 | 8% | 5982 | 8.7% | 0.7 | 0.9 | 0.8 | 0.9 |
| M | 5 | 4% | 5 | 4% | 0 | 0% | 0 | 0% | 1120 | 1.6% | 2.2 | 2.2 | 0.0 | 0.0 |
| N | 4 | 3% | 4 | 3% | 8 | 6% | 0 | 0% | 2365 | 3.5% | 0.8 | 0.8 | 1.7 | 0.0 |
| P | 15 | 11% | 9 | 6% | 1 | 1% | 1 | 1% | 4310 | 6.3% | 1.7 | 1.0 | 0.1 | 0.1 |
| Q | 0 | 0% | 7 | 5% | 5 | 4% | 3 | 3% | 3334 | 4.9% | 0.0 | 1.0 | 0.7 | 0.6 |
| R | 42 | 31% | 0 | 0% | 9 | 6% | 28 | 27% | 4142 | 6.0% | 5.1 | 0.0 | 1.1 | 4.4 |
| S | 4 | 3% | 20 | 14% | 10 | 7% | 3 | 3% | 5154 | 7.5% | 0.4 | 1.9 | 1.0 | 0.4 |
| T | 4 | 3% | 3 | 2% | 4 | 3% | 3 | 3% | 3150 | 4.6% | 0.6 | 0.5 | 0.6 | 0.6 |
| V | 5 | 4% | 3 | 2% | 6 | 4% | 5 | 5% | 3581 | 5.2% | 0.7 | 0.4 | 0.8 | 0.9 |
| W | 1 | 1% | 2 | 1% | 1 | 1% | 3 | 3% | 720 | 1.1% | 0.7 | 1.4 | 0.7 | 2.7 |
| Y | 0 | 0% | 0 | 0% | 2 | 1% | 0 | 0% | 1726 | 2.5% | 0.0 | 0.0 | 0.6 | 0.0 |
| Sum | 136 | 100 % | 139 | 100 % | 139 | 100% | 105 | 100% | 68516 | 100% | | | | |

Fig. 34

| Pair of Amino Acids ... | | # of Peptides | % of Peptides | # of Amino Acid pairs in all Precursors | % of Amino Acid pairs in all Precursors | x-fold Increase |
|---|---|---|---|---|---|---|
| ... Before Amino-Terminal Cleavage | RR | 18 | 12.9% | 398 | 0.58% | 22.2 |
| | KR | 13 | 9.4% | 351 | 0.51% | 18.2 |
| ... After Amino-Terminal Cleavage | DA | 15 | 10.8% | 170 | 0.25% | 43.4 |
| | GR | 5 | 3.6% | 215 | 0.31% | 11.4 |
| ... Before Carboxy-Terminal Cleavage | QK | 7 | 5.0% | 169 | 0.25% | 20.4 |
| | VN | 6 | 4.3% | 188 | 0.27% | 15.7 |
| | GA | 6 | 4.3% | 293 | 0.43% | 10.1 |
| ... After Carboxy-Terminal Cleavage | KR | 16 | 11.5% | 351 | 0.51% | 22.4 |

Fig. 35

**Hub peptide VGF 26-58**

* predicts unknown peptide with m/z = 3688.03 as **VGF 26-62**
+ calculated m/z matches found m/z (prerequisite condition)
+ same start position as hub peptide (+ 69 bonus points)
+ R as amino acid after end position (+ 4 bonus points)

* predicts unknown peptide with m/z = 2419.41 as **VGF 350-370**
+ calculated m/z matches found m/z (prerequisite condition)
+ R as amino acid before start position (+ 5 bonus points)
+ RR as amino acids before start position (+22 bonus points)

Fig. 36

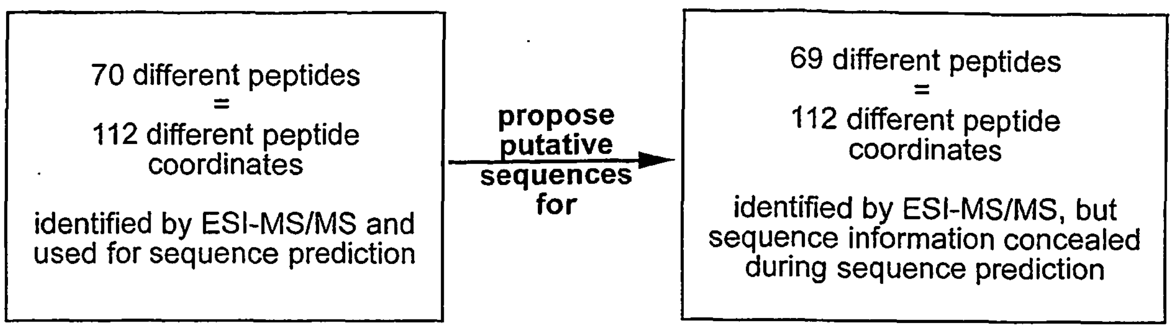
70 different peptides
=
112 different peptide coordinates
identified by ESI-MS/MS and used for sequence prediction
propose putative sequences for
69 different peptides
=
112 different peptide coordinates
identified by ESI-MS/MS, but sequence information concealed during sequence prediction

Fig. 37

| \|r\| ≥ 0.75 | Any proposal | | | Proposal With Most Bonus Points | | |
|---|---|---|---|---|---|---|
| | Precursor Correct Start- or End Position Correct | Precursor Correct Start- or End Position False | Precursor False Start- or End Position False | Precursor Correct Start- or End Position Correct | Precursor Correct Start- or End Position False | Precursor False Start- or End Position False |
| Model 1: No rules | 5 6% | 61 75% | 15 19% | 3 11% | 19 70% | 5 19% |
| Model 2: Only Rules Considering Single Amino Acids at Cleavage Sites | 13 16% | 58 72% | 10 12% | 10 37% | 14 52% | 3 11% |
| Model 3: Only Rules Considering Pairs of Amino Acid Pairs | 18 22% | 54 67% | 11 14% | 15 56% | 9 33% | 3 11% |
| Model 4: Only Rules Considering Common Start- or End Position | 17 21% | 51 63% | 13 16% | 13 48% | 11 41% | 3 11% |
| Model 5: Combination: Only Rules Considering Single Amino Acids & Pairs of Amino Acid | 19 23% | 54 67% | 8 10% | 18 67% | 6 22% | 3 11% |
| Model 6: Combination: all rules | 28 35% | 45 56% | 8 10% | 23 85% | 1 4% | 3 11% |

Fig. 38

| Correlation to: Albumin | Correlation to: Alb. 25-48 | Hub: | Monoisotop. Mass | Sequence |
|---|---|---|---|---|
| r=0.73 | | Albumin 25-48 | 2752.4 | DAHKSEVAHRFKDLGEENFKALVL |
| | | **related Peptide to Alb. 25-48:** | | |
| r=0.80 | r=0.77 | Albumin 27-50 | 2750.5 | HKSEVAHRFKDLGEENFKALVLIA |
| r=0.76 | r=0.75 | Albumin 25-50 | 2936.6 | DAHKSEVAHRFKDLGEENFKALVLIA |
| r=0.76 | r=0.75 | Albumin 25-51 | 3085.5 | DAHKSEVAHRFKDLGEENFKALVLIAF |
| r=0.83 | r=0.75 | alpha-1-Antitrypsin 397-418 | 2502.3 | LMIEQNTKSPLFMGKVVNPTQK |

Fig. 39

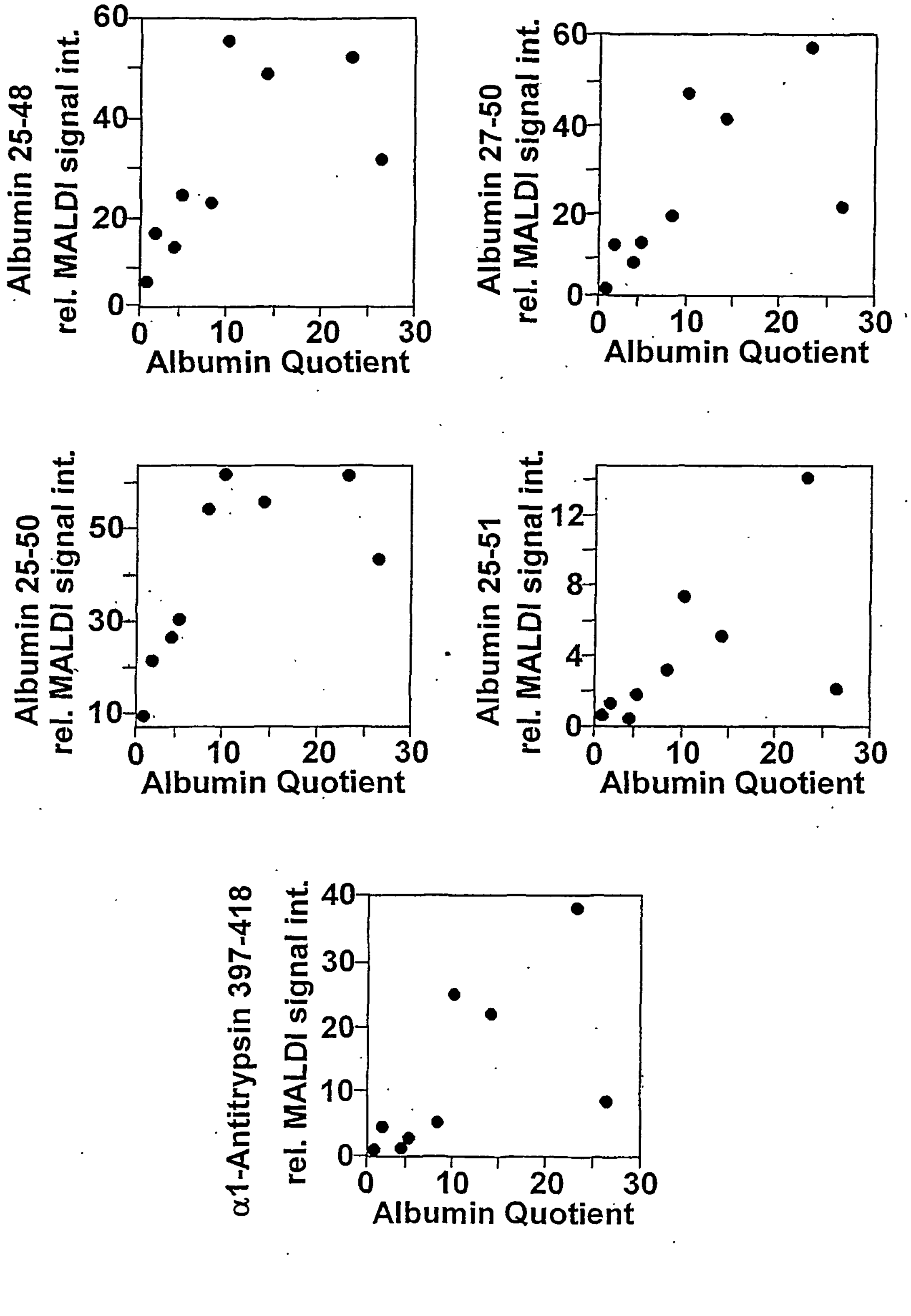
Albumin 25-48
rel. MALDI signal int.
Albumin Quotient
Albumin 27-50
rel. MALDI signal int.
Albumin Quotient
Albumin 25-50
rel. MALDI signal int.
Albumin Quotient
Albumin 25-51
rel. MALDI signal int.
Albumin Quotient
α1-Antitrypsin 397-418
rel. MALDI signal int.
Albumin Quotient

Fig. 40

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20020182649 A
- DE 10038694 A
- WO 2004008371 A
- WO 03048775 A2


### Non-patent literature cited in the description


- **Eng et al.** *Journ. Am. Soc. Mass Spectrom.,* 1994, vol. 5, 976-989
- **Havilio et al.** *Anal. Chem,* 2003, vol. 75 (3), 435-444
- **Perkins et al.** *Electrophoresis,* 1999, vol. 20 (18), 3551-3567
- **Wilkes, J. et al.** Defining and using microbial spectral databases. *JOURNAL OF THE AMERICAN SOCIETY FOR MASS SPECTROMETRY,* July 2002, vol. 13, 875-887
- **Vaidyanthan, S. et al.** Flow-injection electrospray ionization mass spectrometry of crude cell extracts for high-throughput bacterial identification. *JOURNAL OF THE AMERICAN SOCIETY FOR MASS SPECTROMETRY. UNITED STATES,* February 2002, vol. 13 (2), 118-128
- **Pevzner, P et al.** Efficiency of database search for identification of mutated and modified proteins via mass spectrometry. *GENOME RESEARCH,* February 2001, vol. 11 (2), 290-299
- **Fuller et al.** *Applied Spectroscopy,* 1988, vol. 42, 217
- **Evan.** Graph Algorithms. Computer Science Press, 1979
- **Kruskal.** *Proc. American Math. Soc.,* 1956, vol. 7, 48-50
- **Sun et al.** *Physica A,* 1993, vol. 199, 232-242
- **Falquet et al.** *Nucleic Acids Res,* 2002, vol. 30, 235-238
- **Alberts et al.** Molecular Biology of the Cell. Garland Publications, 2002
- **Coligan et al.** Short Protocols in Protein Science. John Wiley & Sons, 2003
- **Mueller ; Lemke.** Self-Organising Data Mining Extracting Knowledge From Data. Trafford Publishing, 2003
- **Engelman et al.** *Ann. Rev. Biophys. Chem.,* 1986, vol. 15, 321
- **von Heijne.** *Eur. J. Biochem.,* 1981, vol. 116, 419
- **Lamerz et al.** *Proteomics,* 2005, vol. 5, x-xx
- **Falquet et al.** *Nucleic Acids Res.,* 2002, vol. 30, 235-238
- **Reiber et al.** *J. Neurobiol.,* 1980, vol. 224, 89-99
- **Heine et al.** *J. Chromatogr. B Analyt. Technol. Biomed. Life. Sci.,* 2002, vol. 782 (1-2), 353-61
- **Heine.** *J. Chromatogr. B Analyt. Technol. Biomed. Life. Sci.,* 2002, vol. 782 (1-2), 353-61
- **Pearl et al.** *Arch. Neurolo,* 1985, vol. 42, 775-777
- **Schulz-Knappe et al.** *Comb. Chem. High Throughput. Screen.,* 2001, vol. 4, 207-217